# EUROPEAN PATENT APPLICATION

(11) **EP 3 950 693 A1**
(43) Date of publication of application: **09.02.2022**
(21) Application number: 20779336.5
(22) Date of filing: 27.03.2020
(51) Int. Cl.: C07D 495/04, C07D 498/08, A61K 31/5377, A61K 31/4439, A61P 35/00

(54) **THIENOHETEROCYCLIC DERIVATIVE, PREPARATION METHOD THEREFOR AND MEDICAL USE THEREOF**

(30) Priority: 28.03.2019 CN 201910242648; 13.03.2020 CN 202010174816
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); CHEN, Yang, Shanghai 200245 (CN); FENG, Binqiang, Shanghai 200245 (CN); HE, Feng, Shanghai 200245 (CN); TAO, Weikang, Shanghai 200245 (CN)
(74) Representative: Potter Clarkson
(86) International application number: PCT/CN2020/081582
(87) International publication number: WO 2020/192750

(57) **Abstract**

Provided are a thienoheterocyclic derivative, a preparation method therefor and the medical use thereof. In particular, provided are a thienoheterocyclic derivative represented by formula (I), a preparation method therefor, a pharmaceutical composition containing the derivative, the use of same as a therapeutic agent, particularly as an ERK inhibitor, and the use of same in the preparation of a drug for treating or preventing cancers, inflammation or other proliferative diseases, wherein each substituent of formula (I) is the same as defined in the description.

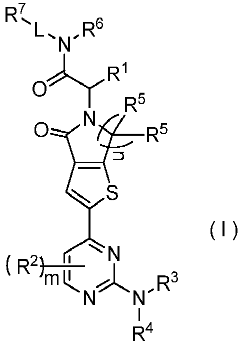

## Description

### TECHNICAL FIELD

The disclosure belongs to the field of medicine and relates to a thienyl fused heterocyclic derivative, preparation method therefor, and medical application thereof. Specifically, the present disclosure relates to a thienyl fused heterocyclic derivative represented by formula (I), a preparation method for preparing the same, a pharmaceutical composition comprising the same, and a use as an ERK inhibitor for treating ERK mediated diseases and disorders or inhibiting the MAPK-ERK signaling pathway.

### BACKGROUND

The proliferation, differentiation, metabolism and apoptosis of normal cells are strictly regulated by cell signal transduction pathways *in vivo.* Mitogen activated protein kinase (MAPK) plays a very important role in signal transduction pathway. Extracellular signal regulated kinase (ERK) is a member of MAPK family. Through the RAS-RAF-MEK-ERK pathway, the exogenous stimulation signal is transmitted to the ERK, and the activated ERK is transferred into the nucleus to regulate the activity of transcription factors, thereby regulating the biological functions of cells such as proliferation, differentiation and apoptosis, or to participate in the regulation of cell morphology and the redistribution of cytoskeleton by phosphorylating cytoskeleton components in the cytoplasm.

RAS and RAF gene mutations cause the continuous activation of the MAPK-ERK signal pathway, which promotes the malignant transformation and abnormal proliferation of cells, and finally produces tumors (Roberts PJ et al., Oncogene, 2007, 26(22), 3291-3310). The combination of a MEK inhibitor and a B-RAF inhibitor can further improve the effect of the B-RAF inhibitor in inhibiting tumor growth, and significantly improve the disease-free progression and overall survival rate of melanoma patients with BRAFV600E and v600k mutations (Frederick DT et al., Clinical Cancer Research, 2013, 19(5), 1225-1231). Although the combination of B-RAF/MEK inhibitors can inhibit tumor, their curative effect is short. Most patients will develop drug resistance within 2-18 months, and the tumor will further deteriorate. The mechanism of resistance to B-RAF/ MEK inhibitors is very complex, which is mostly related to the reactivation of the ERK signaling pathway (Smalley I et al., Cancer Discovery, 2018, 8(2), 140-142). Thus, the development of new ERK inhibitors is effective not only for patients with mutations in MAPK signaling pathway, but also for patients with resistance to B-RAF/MEK inhibitors.

B-RAF/MEK inhibitors not only inhibit tumor growth, but also regulate the immune microenvironment of tumor. B-RAF/MEK inhibitor can enhance the expression of tumor specific antigen, improve the recognition and killing of tumors by antigen-specific T cells, and promote the migration and infiltration of immune cells. In animal models, after treatment with B-RAF/MEK inhibitors, the expression of PD-L1 in tumor tissues increased. When combined with antibodies to checkpoint molecules (such as PD-1 antibody and CTLA4 antibody), it showed a better effect of inhibiting tumor growth than B-Raf / MEK inhibitors used alone (Boni A et al., Cancer Research, 2010, 70(13), 5213-5219). Studies have shown that ERK inhibitosr are similar to B-RAF/MEK inhibitors, and their combination with checkpoint antibodys can regulate the tumor microenvironment, improve the function of cytotoxic T cells, and achieve the effect of inhibiting tumor growth.

At present, many compounds have been developed. Among them, BVD-523 developed by Biomed Valley Discoveries is in the clinical phase II, MK-8353 developed by Merck and Astex-029 developed by Astex are in the clinical phase I. Relevant patents include WO1999061440A1, WO2001056557A2, WO2001056993A2, WO2001057022A2, WO2002022601A1, WO2012118850A1, WO2013018733A1, WO2014179154A2, WO2015103133A1, WO2016192063A1, WO2017180817A1, and WO2018049127A1.

### SUMMARY OF THE INVENTION

The object of the present disclosure is to provide a compound of formula (I): 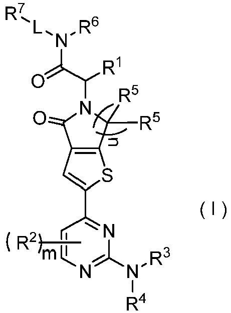 or a stereoisomer, tautomer, mesomer, racemate, enantiomer, diastereomer thereof, or mixture thereof, or a pharmaceutically acceptable salt thereof, wherein:
R¹ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R² is identical or different, and each is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R³ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁴ is selected from the group consisting of hydrogen atom, halogen, alkyl, alkoxy, haloalkyl, haloalkoxy, hydroxy, hydroxyalkyl, cyano, amino, nitro, cycloalkyl and heterocyclyl;
R⁵ is identical or different, and each is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁶ is selected from the group consisting of hydrogen atom, halogen, alkyl, alkoxy, haloalkyl, haloalkoxy, hydroxy and hydroxyalkyl;
L is a bond or alkylene, wherein the alkylene is optionally further substituted by one or more substituents selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
R⁷ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, haloalkyl, haloalkoxy and aminoalkyl;
n is 1, 2 or 3; and
m is 0, 1 or 2.

In some embodiments of the present disclosure, the compound of formula (I), or the stereoisomer, tautomer, mesomer, racemate, enantiomer, diastereomer thereof, or mixture thereof, or the pharmaceutically acceptable salt thereof, is a compound of formula (I-P1) or formula (I-P2): 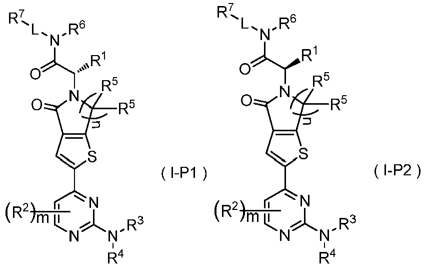 or a stereoisomer, tautomer, mesomer, racemate, enantiomer, diastereomer thereof, or mixture thereof, or a pharmaceutically acceptable salt thereof, wherein:
L, R¹ to R⁷, m and n are as defined in the compound of formula (I).

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, n is 1 or 2.

In some embodiments of the present disclosure, the compound of formula (I), or the stereoisomer, tautomer, mesomer, racemate, enantiomer, diastereomer thereof, or mixture thereof, or the pharmaceutically acceptable salt thereof, is a compound of formula (II): 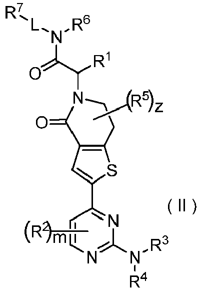 or a stereoisomer, tautomer, mesomer, racemate, enantiomer, diastereomer thereof, or mixture thereof, or a pharmaceutically acceptable salt thereof, wherein:
z is 0, 1, 2, 3 or 4; and L, R¹ to R⁷ and m are as defined in the compound of formula (I).

In some embodiments of the present disclosure, the compound of formula (I), or the stereoisomer, tautomer, mesomer, racemate, enantiomer, diastereomer thereof, or mixture thereof, or the pharmaceutically acceptable salt thereof, is a compound of formula (II-P1) or formula (II-P2): 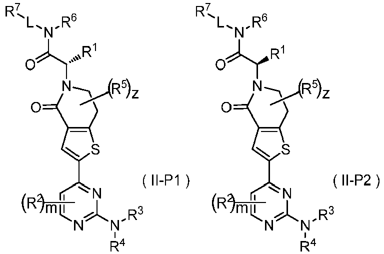 or a stereoisomer, tautomer, mesomer, racemate, enantiomer, diastereomer thereof, or mixture thereof, or a pharmaceutically acceptable salt thereof, wherein:
L, R¹ to R⁷, m or z are as defined in the compound of formula (II).

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, the L is an alkylene, wherein the alkylene is optionally further substituted by one or more substituents selected from the group consisting of alkyl, alkoxy, oxo, halogen, amino, cyano, nitro, hydroxy, hydroxyalkyl and aminoalkyl; preferably further substituted by hydroxyalkyl; and more preferably, L is - CH(R⁸)-, wherein R⁸ is C₁₋₆ hydroxyalkyl.

In some embodiments of the present disclosure, the compound of formula (I), or the stereoisomer, tautomer, mesomer, racemate, enantiomer, diastereomer thereof, or mixture thereof, or the pharmaceutically acceptable salt thereof, is a compound of formula (III): 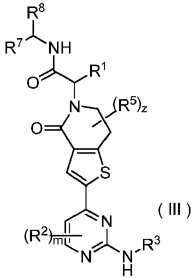 or a stereoisomer, tautomer, mesomer, racemate, enantiomer, diastereomer thereof, or mixture thereof, or a pharmaceutically acceptable salt thereof, wherein:
R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
R¹ to R³, R⁵, R⁷, m and z are as defined in the compound of formula (II).

In some embodiments of the present disclosure, the compound of formula (I), or the stereoisomer, tautomer, mesomer, racemate, enantiomer, diastereomer thereof, or mixture thereof, or the pharmaceutically acceptable salt thereof, is a compound of formula (III-P1) or formula (III-P2): 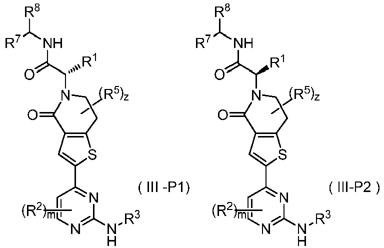 or a stereoisomer, tautomer, mesomer, racemate, enantiomer, diastereomer thereof, or mixture thereof, or a pharmaceutically acceptable salt thereof, wherein:
R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
R¹ to R³, R⁵, R⁷ and z are as defined in the compound of formula (III).

In some embodiments of the present disclosure, the compound of formula (I), or the stereoisomer, tautomer, mesomer, racemate, enantiomer, diastereomer thereof, or mixture thereof, or the pharmaceutically acceptable salt thereof, is a compound of formula (IV): 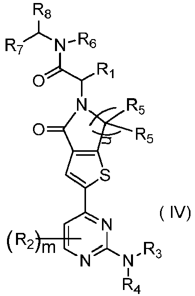 or a stereoisomer, tautomer, mesomer, racemate, enantiomer, diastereomer thereof, or mixture thereof, or a pharmaceutically acceptable salt thereof, wherein:
R¹ to R⁸, m and z are as defined in the compound of formula (III); and n is as defined in the compound of formula (I).

In some embodiments of the present disclosure, the compound of formula (I), or the stereoisomer, tautomer, mesomer, racemate, enantiomer, diastereomer thereof, or mixture thereof, or the pharmaceutically acceptable salt thereof, is a compound of formula (IV-P1) or formula (IV-P2): 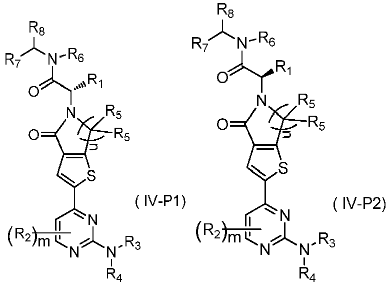 or a stereoisomer, tautomer, mesomer, racemate, enantiomer, diastereomer thereof, or mixture thereof, or a pharmaceutically acceptable salt thereof, wherein:
R¹ to R⁸, m and z are as defined in the compound of formula (III); and n is as defined in the compound of formula (I).

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R³ is selected from the group consisting of C₁₋₆ hydroxyalkyl, 3 to 6 membered heterocyclyl and 5 or 6 membered heteroaryl, wherein the C₁₋₆ hydroxyalkyl, 3 to 6 membered heterocyclyl and 5 or 6 membered heteroaryl are each optionally further substituted by one or more substituents selected from the group consisting of C₁₋₆ alkyl, C₁₋₆ alkoxy, halogen, amino, cyano, hydroxy, and C₁₋₆ hydroxyalkyl.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R³ is a heterocyclyl, wherein the heterocyclyl is optionally further substituted by one or more substituents selected from the group consisting of alkyl, alkoxy, oxo, halogen, amino, cyano, nitro, hydroxy and hydroxyalkyl.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R³ is a heteroaryl, wherein the heteroaryl is optionally further substituted by one or more substituents selected from the group consisting of alkyl, alkoxy, oxo, halogen, amino, cyano, nitro, hydroxy and hydroxyalkyl.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R⁷ is an aryl, wherein the aryl is optionally further substituted by one or more substituents selected from the group consisting of alkyl, alkoxy, oxo, halogen, amino, cyano, nitro, hydroxy and hydroxyalkyl; preferably, R⁷ is a phenyl, wherein the phenyl is optionally further substituted by one or more substituents selected from the group consisting of C₁₋₆ alkyl, C₁₋₆ alkoxy, halogen, amino, cyano, hydroxy and C₁₋₆ hydroxyalkyl.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R⁸ is selected from the group consisting of alkyl, alkoxy, oxo, halogen, amino, cyano, nitro, hydroxy, hydroxyalkyl and aminoalkyl, preferably hydroxyalkyl or aminoalkyl; and more preferably C₁₋₆ hydroxyalkyl.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R¹ is selected from the group consisting of hydrogen atom, halogen, alkyl, alkoxy, haloalkyl, haloalkoxy, hydroxy and hydroxyalkyl, preferably hydrogen atom, halogen or alkyl, more preferably hydrogen atom or alkyl; and most preferably hydrogen atom or C₁₋₆ alkyl.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R¹ is an alkyl, and R⁴ is a hydrogen atom; preferably, R¹ is a C₁₋₆ alkyl, and R⁴ is a hydrogen atom.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R² is selected from the group consisting of hydrogen atom, halogen, alkyl, alkoxy, haloalkyl, haloalkoxy, hydroxy and hydroxyalkyl, preferably hydrogen atom, halogen or alkyl; more preferably hydrogen atom, halogen or C₁₋₆ alkyl; and most preferably halogen.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R⁵ is identical or different, and each is selected from the group consisting of hydrogen atom, halogen, alkyl, alkoxy, haloalkyl, haloalkoxy, hydroxy and hydroxyalkyl; preferably hydrogen atom or alkyl; and more preferably hydrogen atom or C₁₋₆ alkyl.

In some embodiments of the present disclosure, in the compound of formula (I), or the stereoisomer, tautomer, mesomer, racemate, enantiomer, diastereomer thereof, or mixture thereof, or the pharmaceutically acceptable salt thereof, R⁶ is selected from the group consisting of hydrogen atom, halogen, alkyl, alkoxy, haloalkyl, haloalkoxy, hydroxy and hydroxyalkyl; preferably hydrogen atom or alkyl; and more preferably hydrogen atom.

Typical compounds of the present disclosure include, but are not limited to:

| Example No. | Structure and name of the compound |
|---|---|
| 1 | 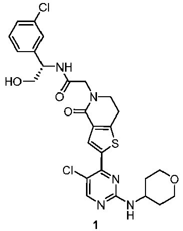 |
| | (*S*)-2-(2-(5-Chloro-2-((tetrahydro-2*H*-pyran-4-yl)amino)pyrimidin-4-yl)-4-oxo-6,7-dihydrothieno[3,2-c]pyridin-5(4*H*)-yl)-*N*-(1-(3-chlorophenyl)-2-hy droxyethyl)acetamide |
| 2 | 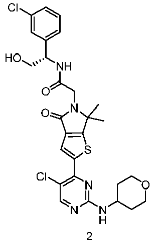 |
| | (S)-2-(2-(5-Chloro-2-((tetrahydro-2*H*-pyran-4-yl)amino)pyrimidin-4-yl)-6,6-dimethyl-4-oxo-4,6-dihydro-5*H*-thieno[2,3-c]pyrrol-5-yl)-N-(1-(3-chlo rophenyl)-2-hydroxyethyl)acetamide |
| 3-P1 | 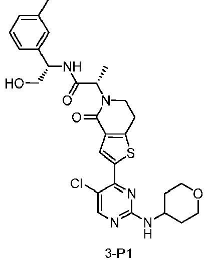 |
| | (*S*)-2-(2-(5-Chloro-2-((tetrahydro-2*H*-pyran-4-yl)amino)pyrimidin-4-yl)-4-oxo-6,7-dihydrothieno[3,2-c]pyridin-5(4*H*)-yl)-*N*-((*S*)-2-hydroxy-1-(*m*-m ethylphenyl)ethyl)propionamide |
| 3-P2 | 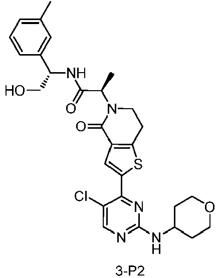 |
| | (*R*)-2-(2-(5-Chloro-2-((tetrahydro-2*H*-pyran-4-yl)amino)pyrimidin-4-yl)-4-oxo-6,7-dihydrothieno[3,2-c]pyridin-5(4*H*)-yl)-*N*-((*S*)-2-hydroxy-1-(*m*-m ethylphenyl)ethyl)propionamide |
| 4-P1 | 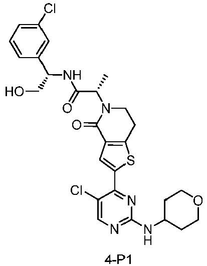 |
| | (*S*)-2-(2-(5-Chloro-2-((tetrahydro-2*H*-pyran-4-yl)amino)pyrimidin-4-yl)-4-oxo-6,7-dihydrothieno[3,2-*c*]pyridin-5(4*H*)-yl)-N-((*S*)-1-(3-chlorophenyl )-2-hydroxyethyl)propionamide |
| 4-P2 | 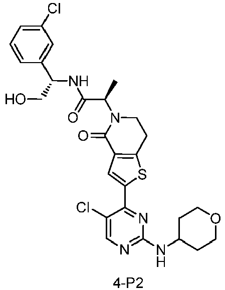 |
| | (*R*)-2-(2-(5-Chloro-2((tetrahydro-2*H*-pyran-4-yl)amino)pyrimidin-4-yl)-4 -oxo-6,7-dihydrothieno[3,2-c]pyridin-5(4*H*)-yl)-*N*-((*S*)-(3-chlorophenyl)-2-hydroxyethyl)propionamide |
| 5 | 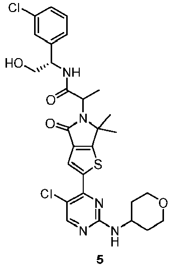 |
| | 2-(2-(5-Chloro-2-((tetrahydro-2*H*-pyran-4-yl)amino)pyrimidin-4-yl)-6,6-dimethyl-4-oxo-4,6-dihydro-5*H*-thieno[2,3-c]pyrrol-5-yl)-*N*-((*S*)-1-(3-chlor ophenyl)-2-hydroxyethyl)propionamide |
| 6 | 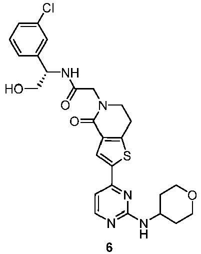 |
| | (*S*)-*N*-(1-(3-Chlorophenyl)-2-hydroxyethyl)-2-(4-oxo-2-(2-((tetrahydro-2 *H*-pyran-4-yl)amino)pyrimidin-4-yl)-6,7-dihydrothieno[3,2-c]pyridin-5(4*H* )-yl)acetamide |
| 7 | 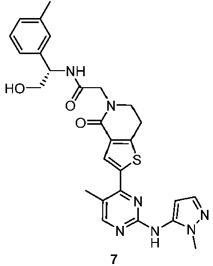 |
| | (*S*)-*N*-(2-Hydroxy-1-(*m*-methylphenyl)ethyl)-2-(2-(5-methyl-2-((1-methy 1-1*H*-pyrazol-5-yl)amino)pyrimidin-4-yl)-4-oxo-6,7-dihydrothieno[3,2-c]p yridin-5(4*H*)-yl)acetamide |
| 8-P1 | 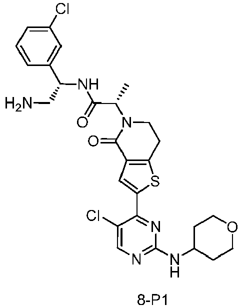 |
| | (*S*)-*N*-((*S*)-2-Amino-1-(3-chlorophenyl)ethyl)-2-(2-(5-chloro-2-((tetrahyd ro-2*H*-pyran-4-yl)amino)pyrimidin-4-yl)-4-oxo-6,7-dihydrothieno[3,2-*c*]py ridin-5(4*H*)-yl)propionamide |
| 8-P2 | 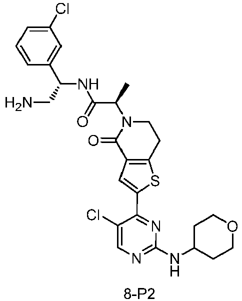 |
| | (*R*)-*N*-((*S*)-2-Amino-1-(3-chlorophenyl)ethyl)-2-(2-(5-chloro-2-((tetrahyd ro-2*H*-pyran-4-yl)amino)pyrimidin-4-yl)-4-oxo-6,7-dihydrothieno[3,2-*c*]py ridin-5(4*H*)-yl)propionamide |
| 9-P1 | 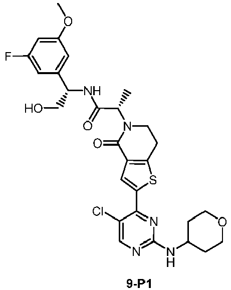 |
| | (*S*)-2-(2-(5-Chloro-2-((tetrahydro-2*H*-pyran-4-yl)amino)pyrimidin-4-yl)-4-oxo-6,7-dihydrothieno[3,2-c]pyridin-5(4*H*)-yl)-*N*-((*S*)-1-(3-fluoro-5-met hoxyphenyl)-2-hydroxyethyl)propionamide |
| 9-P2 | 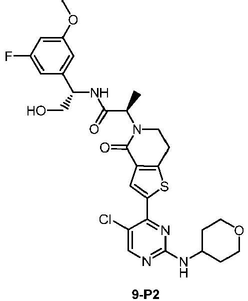 |
| | (*R*)-2-(2-(5-Chloro-2-((tetrahydro-2*H*-pyran-4-yl)amino)pyrimidin-4-yl)-4-oxo-6,7-dihydrothieno[3,2-*c*]pyridin-5(4*H*)-yl)-*N*-((*S*)-1-(3-fluoro-5-met hoxyphenyl)-2-hydroxyethyl)propionamide |
| 10-P1 | 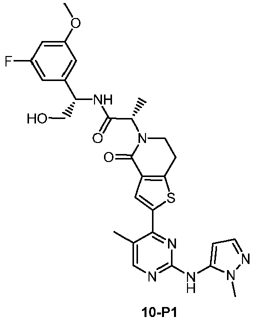 |
| | (*S*)-*N*-((*S*)-1-(3-Fluoro-5-methoxyphenyl)-2-hydroxyethyl)-2-(2-(5-meth yl-2-((1-methyl-1*H*-pyrazol-5-yl)amino)pyrimidin-4-yl)-4-oxo-6,7-dihydro thieno[3,2-c]pyridin-5(4*H*-yl)propionamide |
| 10-P2 | 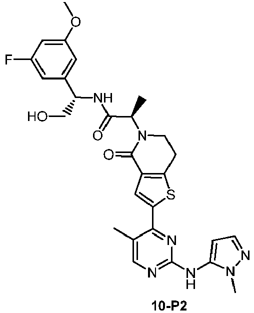 |
| | (*R*)*-N-*((*S*)-1-(3-Fluoro-5-methoxyphenyl)-2-hydroxyethyl)-2-(2-(5-meth yl-2-((1-methyl-1*H*-pyrazol-5-yl)amino)pyrimidin-4-yl)-4-oxo-6,7-dihydro thieno[3,2-*c*]pyridin-5(4*H*)-yl)propionamide |
| 11 | 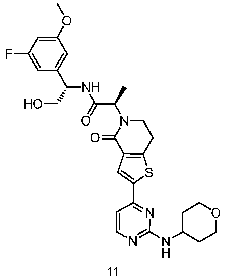 |
| | (*R*)*-N-*((*S*)-1-(3-Fluoro-5-methoxyphenyl)-2-hydroxyethyl)-2-(4-oxo-2-(2-((tetrahydro-2*H*-pyran-4-yl)amino)pyrimidin-4-yl)-6,7-dihydrothieno[3,2-*c* ]pyridin-5(4*H*)-yl)-propionamide 11 |
| 12 | 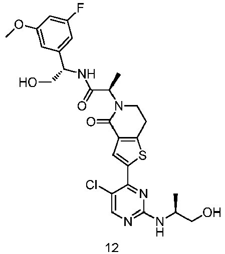 |
| | (*R*)-2-(2-(5-Chloro-2-(((S)-1-hydroxypropan-2-yl)amino)pyrimidin-4-yl)-4-oxo-6,7-dihydrothieno[3,2-c]pyridin-5(4*H*)-yl)-*N*-((*S*)-1-(3-fluoro-5-metho xyphenyl)-2-hydroxyethyl)propionamide 12 |
| 13 | 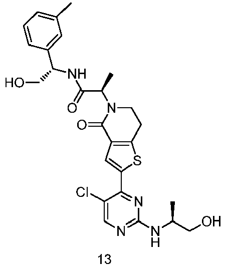 |
| | (*R*)-2-(2-(5-Chloro-2-(((*S*)-1-hydroxypropan-2-yl)amino)pyrimidin-4-yl)-4-oxo-6,7-dihydrothieno[3,2-*c*]pyridin-5(4*H*)-yl)-*N*-((*S*)-2-hydroxy-1-(*m*-met hylphenyl)ethyl)propionamide 13 |
| 14 | 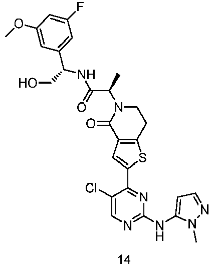 |
| | (*R*)-2-(2-(5-Chloro-2-((1-methyl-1*H*-pyrazol-5-yl)amino)pyrimidin-4-yl)-4 -oxo-6,7-dihydrothieno[3,2-*c*]pyridin-5(4*H*)-yl)-*N*-((*S*)-1-(3-fluoro-5-meth oxyphenyl)-2-hydroxyethyl)propionamide 14 |
| 15 | 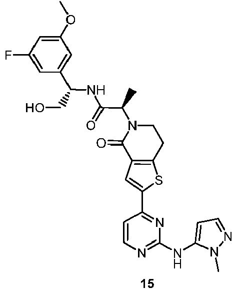 |
| | (*R*)*-N-*((*S*)-1-(3-Fluoro-5-methoxyphenyl)-2-hydroxyethyl)-2-(2-(2-((1-methyl-1*H*-pyrazol-5-yl)amino)pyrimidin-4-yl)-4-oxo-6,7-dihydrothieno[3,2-*c* ]pyridin-5(4*H*)-yl)propionamide 15 |
| 16 | 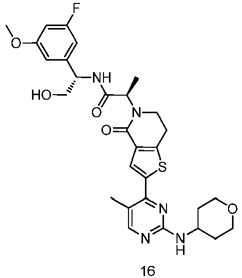 |
| | (*R*)*-N-*((*S*)-1-(3-Fluoro-5-methoxypheny*l)*-2-hydroxyethyl)-2-(2-(5-methyl-2-((tetrahydro-2*H*-pyran-4-yl)amino)pyrimidin-4-yl)-4-oxo-6,7-dihydrothie no[3,2-*c*]pyridin-5-(4*H*)-yl)propionamide 16 |
| 17 | 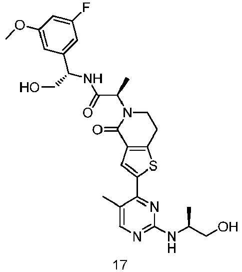 |
| | (*R*)-*N*-((*S*)-1-(3-Fluoro-5-methoxyphenyl)-2-hydroxyethyl)-2-(2-(2-(((*S*)-1-hydroxypropan-2-yl)amino)-5-methylpyrimidin-4-yl)-4-oxo-6,7-dihydrothi eno[3,2-*c*]pyridin-5(4*H*)-yl)propionamide 17 |

or a tautomer, mesomer, racemate, enantiomer, diastereomer, prodrug, mixture thereof, or a pharmaceutically acceptable salt thereof.

In another aspect, the present disclosure relates to a compound of formula (IA) or formula (IA-P1) or formula (IA-P2): 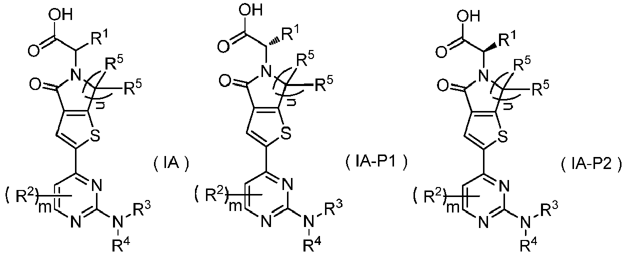 or a stereoisomer, tautomer, mesomer, racemate, enantiomer, diastereomer thereof, or mixture thereof, or a pharmaceutically acceptable salt thereof,
wherein: R¹ to R⁵, m and n are as defined in the compound of formula (I).

In some embodiments of another aspect of the present disclosure, the compound of formula (IA) is a compound of formula (IIA) or formula (IIA-P1) or formula (IIA-P2): 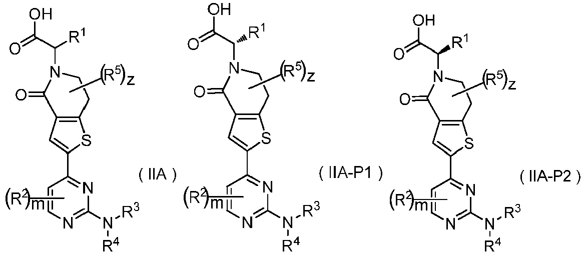 or a stereoisomer, tautomer, mesomer, racemate, enantiomer, diastereomer thereof, or mixture thereof, or a pharmaceutically acceptable salt thereof,
wherein: R¹ to R⁵, m and z are as defined in the compound of formula (II).

In some embodiments of another aspect of the present disclosure, the compound of formula (IA) is a compound of formula (IIIA) or formula (IIIA-P1) or formula (IIIA-P2): 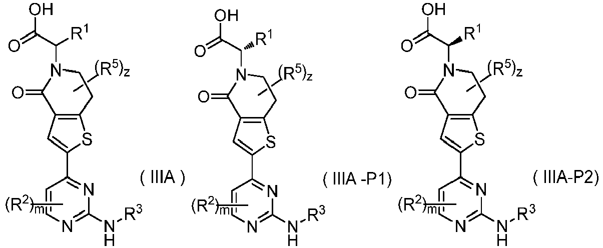 or a stereoisomer, tautomer, mesomer, racemate, enantiomer, diastereomer thereof, or mixture thereof, or a pharmaceutically acceptable salt thereof,
wherein: R¹ to R³, R⁵, m and z are as defined in the compound of formula (III).

Typical compounds of formula (IA) of the present disclosure include, but are not limited to:

| Example No. | Structure and name of the compound |
|---|---|
| 1h | 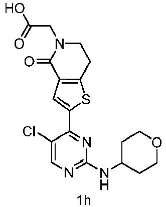 |
| | 2-(2-(5-Chloro-2-((tetrahydro-2*H*-pyran-4-yl)amino)pyrimidin-4-yl)-4-oxo-6,7-dihydrothieno[3,2-*c*]pyridin-5(4*H*)-yl)acetic acid |
| 2f | 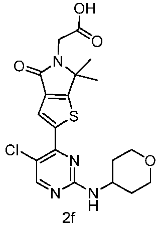 |
| | 2-(2-(5-Chloro-2-((tetrahydro-2*H*-pyran-4-yl)amino)pyrimidin-4-yl)-6,6-dimethyl-4-oxo-4,6-dihydro-5*H*-thieno[2,3-*c*]pyrrol-5-yl)acetic acid |
| 3e | 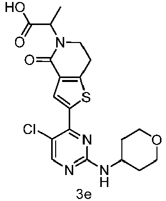 |
| | 2-(2-(5-Chloro-2-((tetrahydro-2*H*-pyran-4-yl)amino)pyrimidin-4-yl)-4-oxo-6,7-dihydrothieno[3,2-*c*]pyridin-5(4*H*)-yl)propanoic acid |
| 5f | 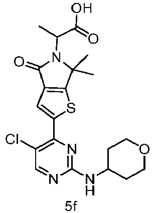 |
| | 2-(2-(5-Chloro-2-((tetrahydro-2*H*-pyran-4-yl)amino)pyrimidin-4-yl)-6,6-dimethyl-4-oxo-4,6-dihydro-5*H*-thieno[2,3-*c*]pyrrol-5-yl)propanoic acid |
| 6e | 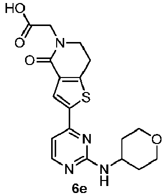 |
| | 2-(4-oxo-2-(2-((tetrahydro-2*H*-pyran-4-yl)amino)pyrimidin-4-yl)-6,7 -dihydrothieno[3,2-*c*]pyridin-5(4*H*)-yl)acetic acid |
| 7f | 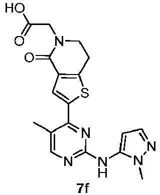 |
| | 2-(2-(5-Methyl-2-((1-methyl-1*H*-pyrazol-5-yl)amino)pyrimidin-4-yl) -4-oxo-6,7-dihydrothieno[3,2-*c*]pyridin-5(4*H*)-yl)acetic acid |
| 10b | 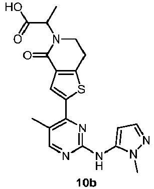 |
| | 2-(2-(5-Methyl-2-((1-methyl-1*H*-pyrazol-5-yl)amino)pyrimidin-4-yl) -4-oxo-6,7-dihydrothieno[3,2-*c*]pyridin-5(4*H*)-yl)propanoic acid |
| 11d | 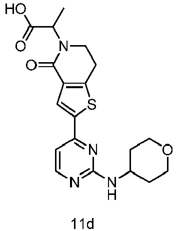 |
| | 2-(4-oxo-2-(2-((tetrahydro-2*H*-pyran-4-yl)amino)pyrimidin-4-yl)-6,7-dihydrothieno[3,2-*c*]pyridin-5(4*H*-yl)propanoic acid **11d** |
| 12c | 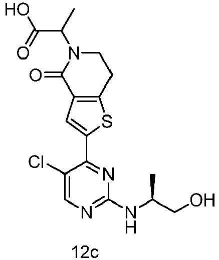 |
| | 2-(2-(5-Chloro-2-(((*S*)-1-hydroxypropan-2-yl)amino)pyrimidin-4-yl)-4-oxo-6,7-dihydrothieno[3,2-*c*]pyridin-5(4*H*)-yl)propanoic acid **12c** |
| 14c | 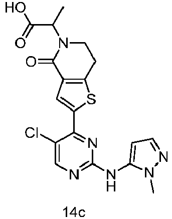 |
| | 2-(2-(5-Chloro-2-((1-methyl-1*H*-pyrazol-5-yl)amino)pyrimidin-4-yl)-4 -oxo-6,7-dihydrothieno[3,2-*c*]pyridin-5(4*H*)-yl)propanoic acid **14c** |
| 15e | 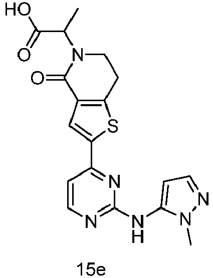 |
| | 2-2-(2-((1-Methyl-1*H*-pyrazol-5-yl)amino)pyrimidin-4-yl)-4-oxo-6,7 -dihydrothieno[3,2-*c*]pyridin-5(4*H*)-yl)propanoic acid 15e |
| 16e | 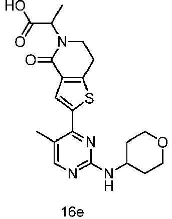 |
| | 2-(2-(5-Methyl-2-((tetrahydro-2*H*-pyran-4-yl)amino)pyrimidin-4-yl)-4-oxo-6,7-dihydrothieno[3,2-*c*]pyridin-5(4*H*)-yl)propanoic acid 16e |
| | 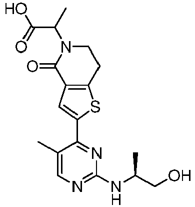 |
| | 2-(2-(5-Methyl-2-(((*S*)-1-hydroxypropan2-yl)amino)pyrimidin-4-yl)-4-oxo-6,7-dihydrothieno[3,2-*c*]pyridin-5(4*H*)-yl)propanoic acid |

or a tautomer, mesomer, racemate, enantiomer, diastereomer, prodrug, mixture thereof, or a pharmaceutically acceptable salt thereof.

In another aspect, the present disclosure relates to a method for preparing the compound of formula (I), comprising a step of: 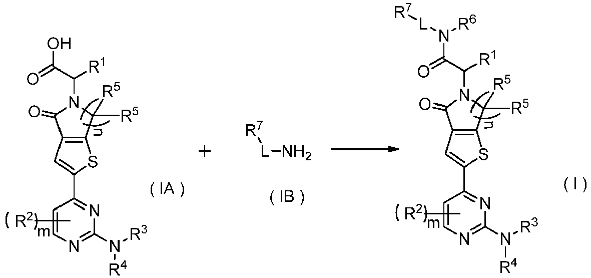 subjecting a compound of formula (IA) and a compound of (IB) to a condensation reaction in the presence of an alkaline reagent to obtain the compound of formula (I),
wherein: the alkaline reagent is preferably *N*,*N*-diisopropyl ethylamine; and
R¹ to R⁷, L, m and n are as defined in the compound of formula (I).

In another aspect, the present disclosure relates to a method for preparing the compound of formula (I-P1), comprising a step of: 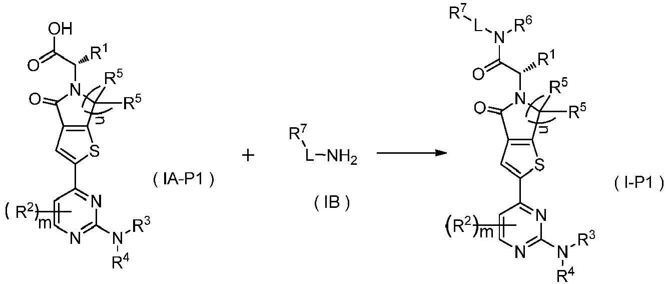 subjecting a compound of formula (IA-P1) and a compound of (IB) to a condensation reaction in the presence of an alkaline reagent to obtain the compound of formula (I-P1), wherein: R¹ to R⁷, L, m and n are as defined in the compound of formula (I).

In another aspect, the present disclosure relates to a method for preparing the compound of formula (I-P2), comprising a step of: 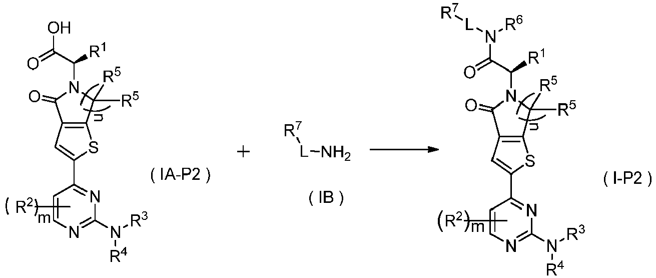 subjecting a compound of formula (IA-P2) and a compound of (IB) to a condensation reaction in the presence of an alkaline reagent to obtain the compound of formula (I-P2), wherein: R¹ to R⁷, L, m and n are as defined in the compound of formula (I).

Another aspect of the present disclosure relates to a method for preparing the compound of formula (II), comprising a step of: 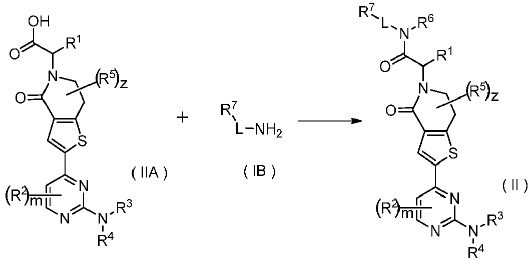 subjecting a compound of formula (IIA) and a compound of (IB) to a condensation reaction in the presence of an alkaline reagent to obtain the compound of formula (II), wherein: the alkaline reagent is preferably *N*,*N*-diisopropyl ethylamine; z is 0, 1, 2, 3 or 4; and R¹ to R⁷, L and m are as defined in the compound of formula (I).

In another aspect, the present disclosure relates to a method for preparing the compound of formula (II-P1), comprising a step of: 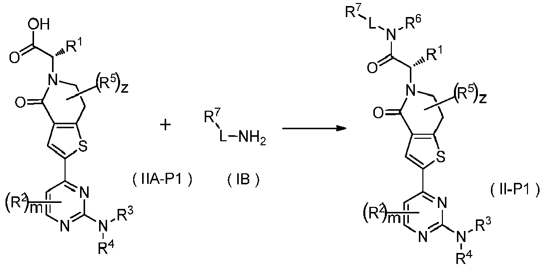 subjecting a compound of formula (IIA-P1) and a compound of (IB) to a condensation reaction in the presence of an alkaline reagent to obtain the compound of formula (II-P1), wherein: the alkaline reagent is preferably *N*,*N*-diisopropyl ethylamine; and R¹ to R⁷, L, m and z are as defined in the compound of formula (II).

In another aspect, the present disclosure relates to a method for preparing the compound of formula (II-P2), comprising a step of: 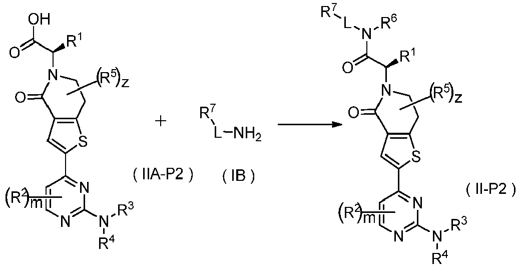 subjecting a compound of formula (IIA-P2) and a compound of (IB) to a condensation reaction in the presence of an alkaline reagent to obtain the compound of formula (II-P2), wherein: the alkaline reagent is preferably *N*,*N*-diisopropyl ethylamine; and R¹ to R⁷, L, m and z are as defined in the compound of formula (II).

In another aspect, the present disclosure relates to a method for preparing the compound of formula (III), comprising a step of: 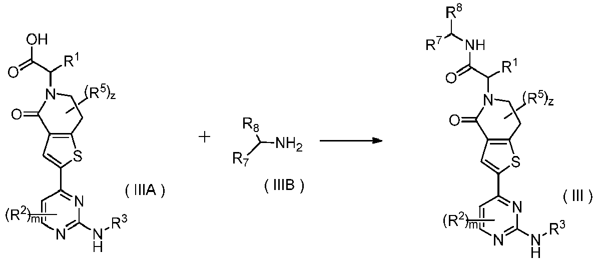 subjecting a compound of formula (IIIA) and a compound of (IIIB) to a condensation reaction in the presence of an alkaline reagent to obtain the compound of formula (III), wherein: the alkaline reagent is preferably *N*,*N*-diisopropyl ethylamine; and R¹ to R³, R⁵, R⁷, R⁸, m and z are as defined in the compound of formula (III).

In another aspect, the present disclosure relates to a method for preparing the compound of formula (III-P1), comprising a step of: 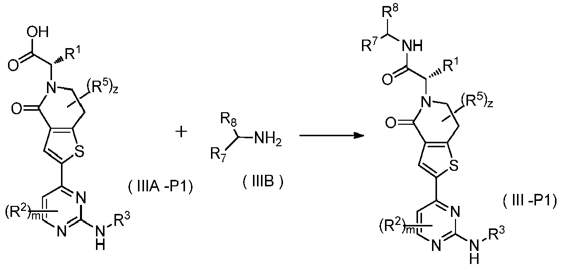 subjecting a compound of formula (IIIA-P1) and a compound of (IIIB) to a condensation reaction in the presence of an alkaline reagent to obtain the compound of formula (III-P1),
wherein: the alkaline reagent is preferably *N*,*N*-diisopropyl ethylamine; and R¹ to R³, R⁵, R⁷, R⁸, m and z are as defined in the compound of formula (III).

In another aspect, the present disclosure relates to a method for preparing the compound of formula (III-P2), comprising a step of: 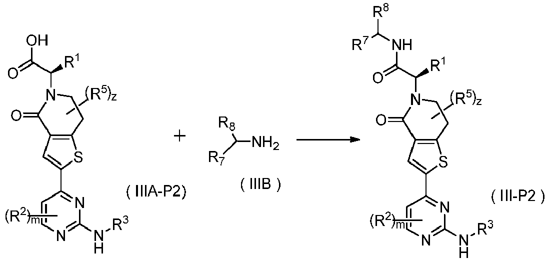 subjecting a compound of formula (IIIA-P2) and a compound of (IIIB) to a condensation reaction in the presence of an alkaline reagent to obtain the compound of formula (III-P2),
wherein: the alkaline reagent is preferably *N*,*N*-diisopropyl ethylamine; and R¹ to R³, R⁵, R⁷, R⁸, m and z are as defined in the compound of formula (III).

In another aspect of the present disclosure relates to a method for preparing the compound of formula (IV), comprising a step of: 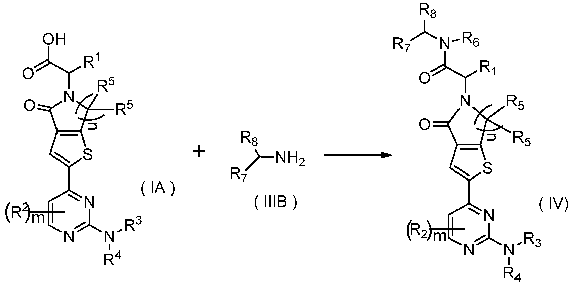 subjecting a compound of formula (IA) and a compound of (IIIB) to a condensation reaction in the presence of an alkaline reagent to obtain the compound of formula (IV),
wherein: the alkaline reagent is preferably *N*,*N*-diisopropyl ethylamine; and R¹ to R⁸, m and n are as defined in the compound of formula (IV).

In another aspect, the present disclosure relates to a method for preparing the compound of formula (IV-P1), comprising a step of: 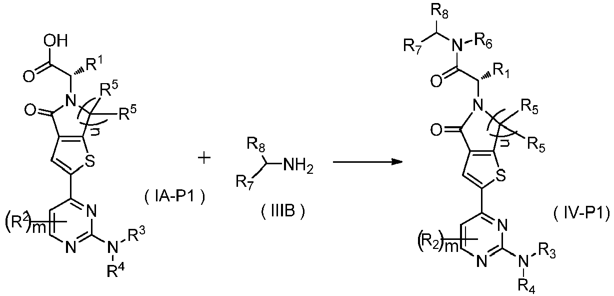 subjecting a compound of formula (IA-P1) and a compound of (IIIB) to a condensation reaction in the presence of an alkaline reagent to obtain the compound of formula (IV-P1),
wherein: the alkaline reagent is preferably *N*,*N*-diisopropyl ethylamine; and R¹ to R⁸, m and n are as defined in the compound of formula (IV).

In another aspect, the present disclosure relates to a method for preparing the compound of formula (IV-P2), comprising a step of: 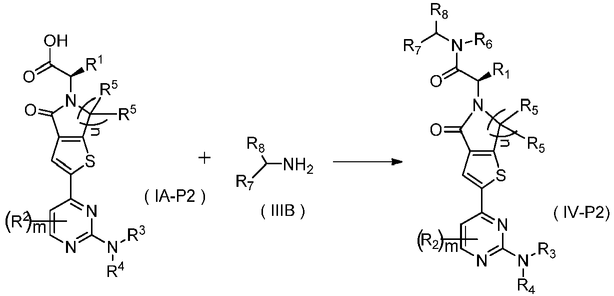 subjecting a compound of formula (IA-P2) and a compound of (IIIB) to a condensation reaction in the presence of an alkaline reagent to obtain the compound of formula (IV-P2),
wherein: the alkaline reagent is preferably *N*,*N*-diisopropyl ethylamine;
R¹ to R⁸, m and n are as defined in the compound of formula (IV).

In another aspect, the present disclosure relates to a pharmaceutical composition, comprising a therapeutically effective amount of the compound of formula (I) or formula (II) or formula (III) or formula (IV) or the tautomer, mesomer, racemate, enantiomer, diastereomer, or mixture thereof, or the pharmaceutically acceptable salt thereof of the present disclosure, and one or more pharmaceutically acceptable carriers, diluents or excipients.

The present disclosure further relates to a use of the compound of formula (I) or formula (II) or formula (III) or formula (IV) or the tautomer, mesomer, racemate, enantiomer, diastereomer, or mixture thereof, or the pharmaceutically acceptable salt thereof, or the pharmaceutical composition comprising the same in the preparation of a medicament for inhibiting ERK.

The present disclosure further relates to a use of the compound of formula (I) or formula (II) or formula (III) or formula (IV) or the tautomer, mesomer, racemate, enantiomer, diastereomer, or mixture thereof, or the pharmaceutically acceptable salt thereof, or the pharmaceutical composition comprising the same in the preparation of a medicaments for treating or preventing cancer, inflammation, or other proliferative diseases, preferably in the preparation of a medicament for treating or preventing cancer; wherein the cancer is selected from the group consisting of melanoma, liver cancer, kidney cancer, lung cancer (such as non-small cell lung cancer or small cell lung cancer), nasopharyngeal carcinoma, colorectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma, and glioma.

The present disclosure also relates to a method for inhibiting ERK, comprising a step of administering to a patient in need thereof a therapeutically effective amount of the compound of formula (I) or formula (II) or formula (III) or formula (IV) or the tautomer, mesomer, racemate, enantiomer, diastereomer, or mixture thereof, or the pharmaceutically acceptable salt thereof, or the pharmaceutical composition comprising the same.

The present disclosure also relates to a method for treating or preventing ERK-mediated diseases, comprising a step of administering to a patient in need thereof a therapeutically effective amount of the compound of formula (I) or formula (II) or formula (III) or formula (IV) or the tautomer, mesomer, racemate, enantiomer, diastereomer, or mixture thereof, or the pharmaceutically acceptable salt thereof, or the pharmaceutical composition comprising the same.

The present disclosure also relates to a method for treating or preventing cancer, inflammation, or other proliferative diseases, preferably cancer, comprising a step of administering to a patient in need thereof a therapeutically effective amount of the compound of formula (I) or formula (II) or formula (III) or formula (IV) or the tautomer, mesomer, racemate, enantiomer, diastereomer, or mixture thereof, or the pharmaceutically acceptable salt thereof, or the pharmaceutical composition comprising the same; wherein the cancer is selected from the group consisting of melanoma, liver cancer, kidney cancer, lung cancer (such as non-small cell lung cancer or small cell lung cancer), nasopharyngeal carcinoma, colorectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma, and glioma.

The present disclosure further relates to the compound of formula (I) or formula (II) or formula (III) or formula (IV) or the tautomer, mesomer, racemate, enantiomer, diastereomer, or mixture thereof, or the pharmaceutically acceptable salt thereof, or the pharmaceutical composition comprising the same, for use as a medicament.

The present disclosure further relates to the compound of formula (I) or formula (II) or formula (III) or formula (IV) or the tautomer, mesomer, racemate, enantiomer, diastereomer, or mixture thereof, or a pharmaceutically acceptable salt thereof, or the pharmaceutical composition comprising the same, for use as an ERK inhibitor.

The present disclosure further relates to the compound of formula (I) or formula (II) or formula (III) or formula (IV) or the tautomer, mesomer, racemate, enantiomer, diastereomer, or mixture thereof, or a pharmaceutically acceptable salt thereof, or the pharmaceutical composition comprising the same, for use in treating or preventing ERK-mediated diseases.

The present disclosure further relates to the compound of formula (I) or formula (II) or formula (III) or formula (IV) or the tautomer, mesomer, racemate, enantiomer, diastereomer, or mixture thereof, or the pharmaceutically acceptable salt thereof, or the pharmaceutical composition comprising the same, for use in treating or preventing cancer, inflammation, or other proliferative diseases, preferably in treating or preventing cancer; wherein the cancer is selected from the group consisting of melanoma, liver cancer, kidney cancer, lung cancer (such as non-small cell lung cancer or small cell lung cancer), nasopharyngeal carcinoma, colorectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma, and glioma.

The active compound can be prepared in a form suitable for administration by any appropriate route, and the active compound is preferably in a unit dose form, or in a form in which the patient can self-administer in a single dose. The unit dose of the compound or composition of the present disclosure can be expressed in the form of tablets, capsules, cachets, bottled syrups, powders, granules, lozenges, suppositories, regenerated powders or liquid formulations.

The dosage of the compound or composition used in the treatment method of the present disclosure will generally vary according to the severity of the disease, the weight of the patient, and the relative efficacy of the compound. However, as a general guide, a suitable unit dose can be 0.1 to 1000 mg.

In addition to the active compound, the pharmaceutical composition of the present disclosure can also comprise one or more auxiliaries including a filler (diluent), binder, wetting agent, disintegrant, excipient and the like. Depending on the administration mode, the composition can comprise 0.1 to 99% by weight of the active compound.

The pharmaceutical composition comprising the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such a composition can comprise one or more ingredient(s) selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets.

An aqueous suspension comprises an active ingredient in admixture with excipients suitable for the manufacture of an aqueous suspension. The aqueous suspension can also comprise one or more preservatives such as ethyl paraben or *n*-propyl paraben, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation.

The active ingredient in admixture with the dispersants or wetting agents, suspending agents or one or more preservatives can be prepared as dispersible powders or granules suitable for the preparation of an aqueous suspension by adding water. Suitable dispersants or wetting agents and suspending agents are exemplified by those already mentioned above. Additional excipients, such as sweeteners, flavoring agents and colorants, can also be added. These compositions can be preserved by adding an antioxidant such as ascorbic acid.

The pharmaceutical composition of the present disclosure can also be in the form of an oil-in-water emulsion.

The pharmaceutical composition can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. For example, the active ingredient is dissolved in a mixture of soybean oil and lecithin. The oil solution is then added into a mixture of water and glycerol, and processed to form a micro-emulsion. The injectable solution or micro-emulsion can be introduced into a patient's bloodstream by local bolus injection. Alternatively, the solution and micro-emulsion are preferably administrated in a manner that maintains a constant circulating concentration of the compound of the present invention. In order to maintain this constant concentration, a continuous intravenous delivery device can be used. An example of such a device is Deltec CADD-PLUS. TM. 5400 intravenous injection pump.

The pharmaceutical composition can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium.

The compound of the present disclosure can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug. Such materials include cocoa butter, glycerin gelatin, hydrogenated vegetable oils, mixtures of polyethylene glycols with various molecular weights and fatty acid esters of polyethylene glycols.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### TERM DEDINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl comprising 1 to 12 carbon atoms, and more preferably an alkyl comprising 1 to 6 carbon atoms. Non-limiting examples include methyl, ethyl, n-propyl, isopropyl, *n*-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl comprising 1 to 6 carbon atoms, and non-limiting examples include methyl, ethyl, *n*-propyl, isopropyl, n-butyl, isobutyl, *tert-butyl, sec-*butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "alkylene" refers to a saturated linear or branched aliphatic hydrocarbon group having two residues derived from the removal of two hydrogen atoms from the same carbon atom or two different carbon atoms of the parent alkane. It is a linear or branched alkylene comprising 1 to 20 carbon atoms, preferably 1 to 12 carbon atoms, and more preferably 1 to 6 carbon atoms. Non limiting examples of alkylene include, but are not limited to, methylene (-CH₂-), 1,1-ethylidene (-CH(CH₃)-), 1,2-ethylidene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-) and the like. The alkylene can be substituted or unsubstituted. When substituted, the substituent group(s) can be substituted at any available connection point. The substituent group(s) is preferably one or more groups independently optionally selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkoxy" refers to an -O-(alkyl) and -O-(unsubstituted cycloalkyl) group, wherein the alkyl is defined as above. Non limiting examples of alkoxy include methoxy, ethoxy, propoxy, butoxy, cyclopropoxy, cyclobutoxy, cyclopentoxy, and cyclohexoxy. The alkoxy can be optionally substituted or unsubstituted. When substituted, the substituent group(s) is preferably one or more groups independently selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent group comprising 3 to 20 carbon atoms, preferably 3 to 12 carbon atoms, more preferably 3 to 8 (for example 3, 4, 5, 6, 7 or 8) carbon atoms, and most preferably 3 to 6 (for example 3, 4, 5 or 6) carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like, preferably cycloalkyl. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

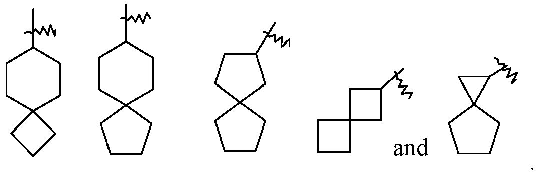

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

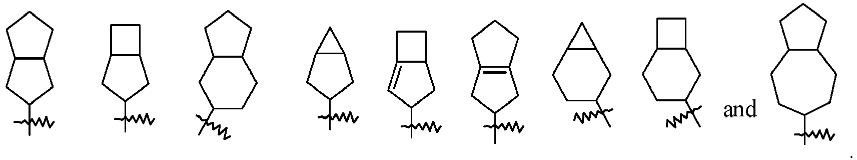

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

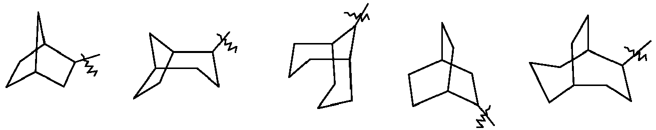

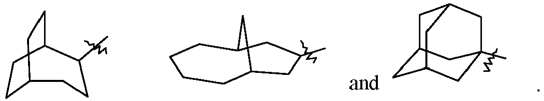

The cycloalkyl ring includes the above cycloalkyl (including monocycloalkyl, spiro cycloalkyl, fused cycloalkyl and bridged cycloalkyl) fused to the ring of aryl, heteroaryl or heterocyclyl, wherein the ring bound to the parent structure is cycloalkyl. Non-limiting examples include indanyl, tetrahydronaphthyl, benzocycloheptyl and the like, and preferably benzocyclopentyl, tetrahydronaphthyl.

The cycloalkyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "heterocyclyl" refers to a 3 to 20 membered saturated or partially unsaturated monocyclic or polycyclic hydrocarbon substituent group, wherein one or more ring atoms are heteroatoms selected from the group consisting of N, O, S, S(O) and S(O)₂, but excluding -O-O-, -O-S- or -S-S- in the ring, with the remaining ring atoms being carbon atoms. Preferably, the heterocyclyl has 3 to 12 ring atoms wherein 1 to 4 (for example 1, 2, 3, or 4) atoms are heteroatoms; preferably, 3 to 8 (for example 3, 4, 5, 6, 7 or 8) ring atoms wherein 1 to 3 atoms are heteroatoms; and preferably 3 to 6 ring atoms wherein 1 to 3 atoms are heteroatoms. Non-limiting examples of monocyclic heterocyclyl include azetidinyl, pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and the like, and preferably tetrahydropyranyl, piperidinyl, pyrrolidinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O, S, S(O) and S(O)₂, with the remaining ring atoms being carbon atoms, where the rings can contain one or more double bonds, but none of the rings has a completely conjugated π-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl is divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

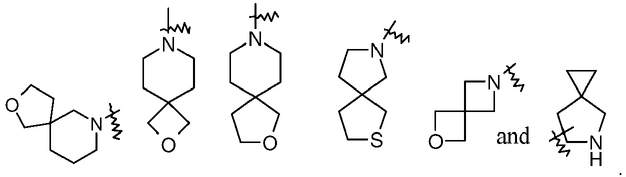

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O, S, S(O) and S(O)₂, with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and the fused heterocyclyl is preferably a bicyclic or tricyclic fused heterocyclyl, and more preferably a 5-membered/5-membered or 5-membered/6-membered bicyclic fused heterocyclyl. Non-limiting examples of fused heterocyclyl include:

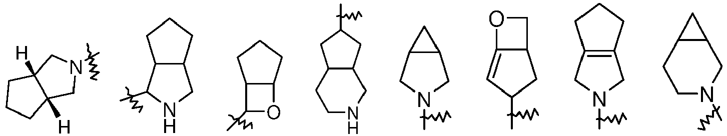

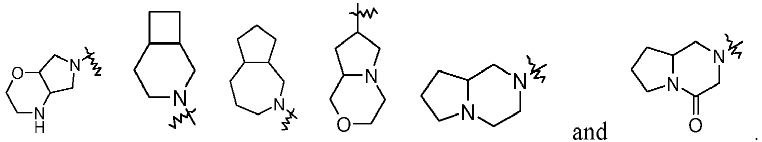

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O, S, S(O) and S(O)₂, with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

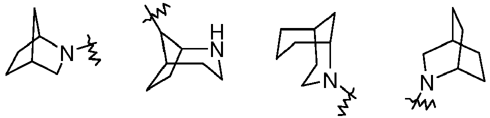

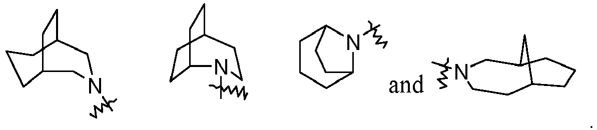

The heterocyclyl ring includes the above heterocyclyl (including mono-heterocyclyl, fused heterocyclyl, spiro heterocyclyl and bridged heterocyclyl) fused to the ring of aryl, heteroaryl or cycloalkyl, wherein the ring bound to the parent structure is heterocyclyl. Non-limiting examples thereof include: 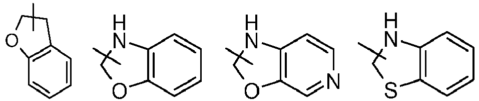 and the like.

The heterocyclyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "aryl" refers to a 6 to 20 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, and more preferably a 6 membered aryl, for example, phenyl and naphthyl. The aryl ring can be fused to the ring of heteroaryl, heterocyclyl or cycloalkyl, wherein the ring bound to the parent structure is aryl ring. Non-limiting examples thereof include:

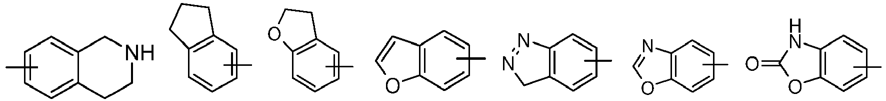

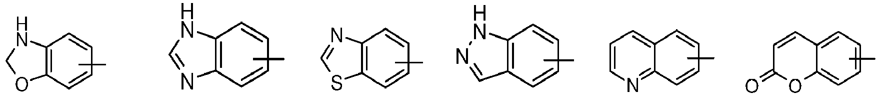

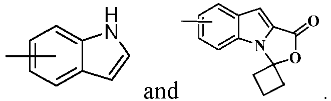

The aryl can be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "heteroaryl" refers to a 5 to 20 membered heteroaromatic system comprising 1 to 4 (for example 1, 2, 3, or 4) heteroatoms selected from the group consisting of O, S and N. The heteroaryl is preferably a 5 to 10 (for example 5, 6, 7, 8, 9 or 10) membered heteroaryl comprising 1 to 3 heteroatoms, and more preferably a 5 or 6 membered heteroaryl comprising 1 to 3 heteroatoms. Non-limiting examples include: for example, pyrazolyl, imidazolyl, furyl, thienyl, thiazolyl, oxazolyl, pyrrolyl, triazolyl, tetrazolyl, pyridyl, pyrimidinyl, thiadiazolyl, pyrazinyl and the like. The heteroaryl ring can be fused to the ring of aryl, heterocyclyl or cycloalkyl, wherein the ring bound to the parent structure is heteroaryl ring. Non-limiting examples thereof include: 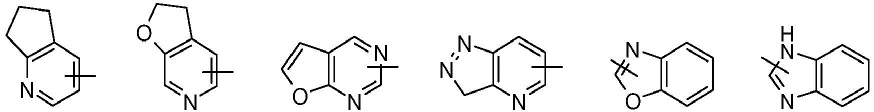 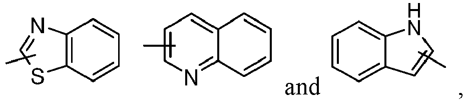 preferably

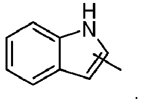

The heteroaryl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkylthio" refers to a -S-(alkyl) and -S-(unsubstituted cycloalkyl) group, wherein the alkyl is defined as above. Non limiting examples of alkylthio include methylthio, ethylthio, propylthio, butylthio, cyclopropylthio, cyclobutylthio, cyclopentylthio, and cyclohexylthio. The alkylthio can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyloxy" refers to a -O-cycloalkyl group, wherein the cycloalkyl is as defined above.

The term "haloalkyl" refers to an alkyl group substituted by halogen(s), wherein the alkyl is as defined above.

The term "haloalkoxy" refers to an alkoxy group substituted by halogen(s), wherein the alkoxy is as defined above.

The term "hydroxyalkyl" refers to an alkyl group substituted by hydroxy(s), wherein the alkyl is as defined above.

The term "aminoalkyl" refers to an alkyl group substituted by amino(s), wherein the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "aldehyde" refers to a -C(O)H group.

The term "carboxy" refers to a -C(O)OH group.

The term "alkoxycarbonyl" refers to a -C(O)O(alkyl) or -C(O)O(cycloalkyl) group, wherein the alkyl and cycloalkyl are as defined above.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5 (for example 1, 2, 3, 4 or 5), and more preferably 1 to 3 hydrogen atoms, independently substituted by a corresponding number of substituents, wherein each substituent has independent options (that is, the substituents can be identical or different). It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

The term "pharmaceutical composition" refers to a mixture of one or more of the compounds described herein or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present disclosure, which is safe and effective in mammals and has the desired biological activity.

The compound of the present disclosure can also comprise isotopic derivatives thereof. The term "isotopic derivatives" refers to compounds that differ in structure only in the presence of one or more isotopically enriched atoms. For example, a compound having the structure of the present disclosure except replacing hydrogen with "deuterium" or "tritium", or replacing fluorine with an ¹⁸F-fluorine labeling (¹⁸F isotope), or replacing carbon with ¹¹C-, ¹³C-, or ¹⁴C-enriched carbon (¹¹C-, ¹³C-, or ¹⁴C-carbon labeling; ¹¹C-, ¹³C-, or ¹⁴C-isotope) is within the scope of the present disclosure. Such compounds can be used, for example, as analytical tools or probes in biological assays, or as tracers for *in vivo* diagnostic imaging of disease, or as tracers for pharmacodynamics, pharmacokinetics or receptor studies. Deuterated compounds can generally retain activity comparable to non-deuterated compounds, and when deuterated at certain specific sites, the resulting compounds can achieve better metabolic stability, thereby obtaining certain therapeutic advantages (such as increased *in vivo* half-life or reduced dosage requirements).

For drugs or pharmacologically active agents, the term "therapeutically effective amount" refers to a sufficient amount of a drug or agent that is non-toxic but can achieve the desired effect. The determination of the effective amount varies from person to person, depending on the age and general condition of the recipient, and also on the specific active substance. The appropriate effective amount in a case can be determined by the person skilled in the art according to routine experiments.

### Synthesis Method of the Compound of the Present Disclosure

In order to achieve the object of the present disclosure, the present disclosure applies the following technical solutions:

### Scheme I

A method for preparing the compound of formula (I) or the tautomer, mesomer, racemate, enantiomer, diastereomer thereof, or mixture thereof, or the pharmaceutically acceptable salt thereof according to the present disclosure, comprises the following step of: 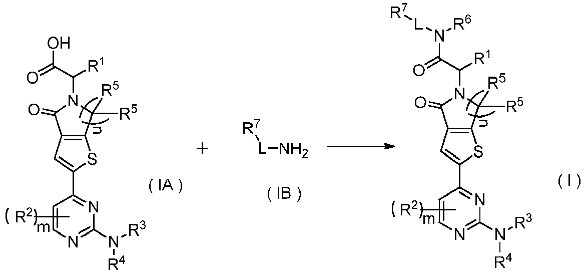 subjecting a compound of formula (IA) and a compound of (IB) to a condensation reaction in the presence of an alkaline reagent to obtain the compound of formula (I), wherein: R¹ to R⁷, L, m and n are as defined in the compound of formula (I).

### Scheme II

A method for preparing the compound of formula (I-P1) or the tautomer, mesomer, racemate, enantiomer, diastereomer thereof, or mixture thereof, or the pharmaceutically acceptable salt thereof according to the present disclosure, comprises the following step of: 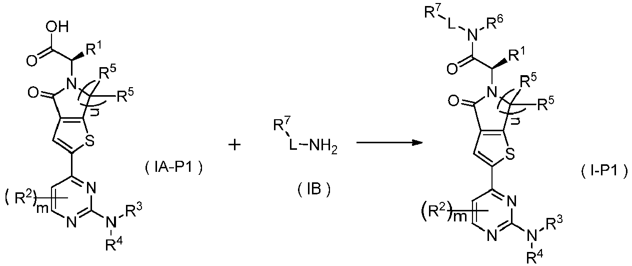 subjecting a compound of formula (IA-P1) and a compound of (IB) to a condensation reaction in the presence of an alkaline reagent to obtain the compound of formula (I-P1), wherein: R¹ to R⁷, L, m and n are as defined in the compound of formula (I).

### Scheme III

A method for preparing the compound of formula (I-P2) or the tautomer, mesomer, racemate, enantiomer, diastereomer thereof, or mixture thereof, or the pharmaceutically acceptable salt thereof according to the present disclosure, comprises the following step of: 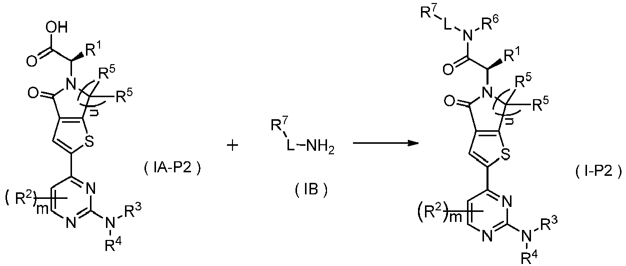 subjecting a compound of formula (IA-P2) and a compound of (IB) to a condensation reaction in the presence of an alkaline reagent to obtain the compound of formula (I-P2), wherein: R¹ to R⁷, L, m and n are as defined in the compound of formula (I).

### Scheme IV

A method for preparing the compound of formula (II) or the tautomer, mesomer, racemate, enantiomer, diastereomer thereof, or mixture thereof, or the pharmaceutically acceptable salt thereof according to the present disclosure, comprises the following step of: 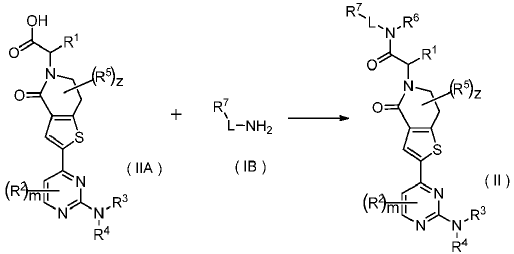 subjecting a compound of formula (IIA) and a compound of (IB) to a condensation reaction in the presence of an alkaline reagent to obtain the compound of formula (II), wherein: the alkaline reagent is preferably *N*,*N*-diisopropyl ethylamine; z is 0, 1, 2, 3 or 4; and R¹ to R⁷, L and m are as defined in the compound of formula (I).

### Scheme V

A method for preparing the compound of formula (II-P1) or the tautomer, mesomer, racemate, enantiomer, diastereomer thereof, or mixture thereof, or the pharmaceutically acceptable salt thereof according to the present disclosure, comprises the following step of: 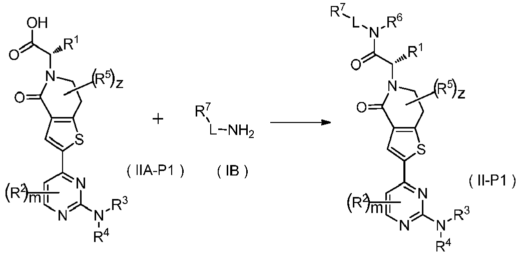 subjecting a compound of formula (IIA-P1) and a compound of (IB) to a condensation reaction in the presence of an alkaline reagent to obtain the compound of formula (II-P1), wherein: the alkaline reagent is preferably *N*,*N*-diisopropyl ethylamine; and R¹ to R⁷, L, m and z are as defined in the compound of formula (II).

### Scheme VI

A method for preparing the compound of formula (II-P2) or the tautomer, mesomer, racemate, enantiomer, diastereomer thereof, or mixture thereof, or the pharmaceutically acceptable salt thereof according to the present disclosure, comprises the following step of: 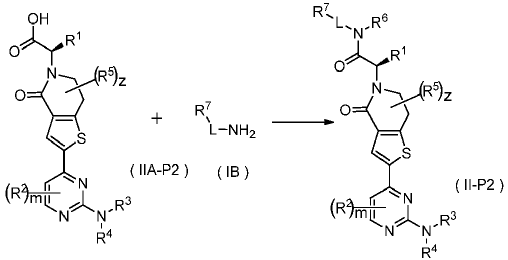 subjecting a compound of formula (IIA-P2) and a compound of (IB) to a condensation reaction in the presence of an alkaline reagent to obtain the compound of formula (II-P2), wherein: the alkaline reagent is preferably *N*,*N*-diisopropyl ethylamine; and R¹ to R⁷, L, m and z are as defined in the compound of formula (II).

### Scheme VII

A method for preparing the compound of formula (III) or the tautomer, mesomer, racemate, enantiomer, diastereomer thereof, or mixture thereof, or the pharmaceutically acceptable salt thereof according to the present disclosure, comprises the following step of: 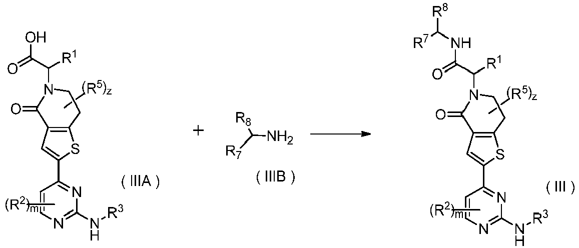 subjecting a compound of formula (IIIA) and a compound of (IIIB) to a condensation reaction in the presence of an alkaline reagent to obtain the compound of formula (III), wherein: the alkaline reagent is preferably *N*,*N*-diisopropyl ethylamine; and R¹ to R³, R⁵, R⁷, R⁸, m and z are as defined in the compound of formula (III).

### Scheme VIII

A method for preparing the compound of formula (III-P1) or the tautomer, mesomer, racemate, enantiomer, diastereomer thereof, or mixture thereof, or the pharmaceutically acceptable salt thereof according to the present disclosure, comprises the following step of: 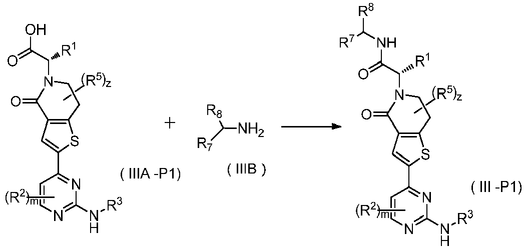 subjecting a compound of formula (IIIA-P1) and a compound of (IIIB) to a condensation reaction in the presence of an alkaline reagent to obtain the compound of formula (III-P1), wherein: the alkaline reagent is preferably *N*,*N*-diisopropyl ethylamine; and R¹ to R³, R⁵, R⁷, R⁸, m and z are as defined in the compound of formula (III).

### Scheme IX

A method for preparing the compound of formula (III-P2) or the tautomer, mesomer, racemate, enantiomer, diastereomer thereof, or mixture thereof, or the pharmaceutically acceptable salt thereof according to the present disclosure, comprises the following step of: 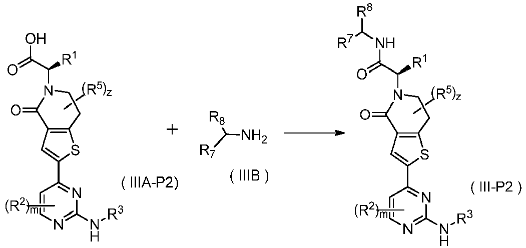 subjecting a compound of formula (IIIA-P2) and a compound of (IIIB) to a condensation reaction in the presence of an alkaline reagent to obtain the compound of formula (III-P2), wherein: the alkaline reagent is preferably *N*,*N*-diisopropyl ethylamine; and R¹ to R³, R⁵, R⁷, R⁸, m and z are as defined in the compound of formula (III).

### Scheme X

A method for preparing the compound of formula (IV) or the tautomer, mesomer, racemate, enantiomer, diastereomer thereof, or mixture thereof, or the pharmaceutically acceptable salt thereof according to the present disclosure, comprises the following step of: 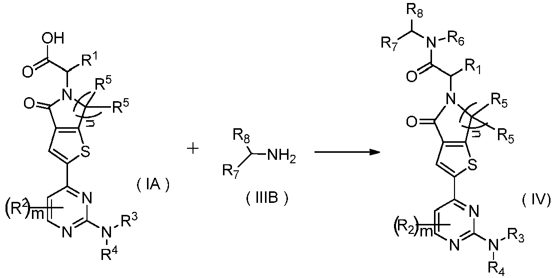 subjecting a compound of formula (IA) and a compound of (IIIB) to a condensation reaction in the presence of an alkaline reagent to obtain the compound of formula (IV), wherein: the alkaline reagent is preferably *N*,*N*-diisopropyl ethylamine; and R¹ to R⁸, m and n are as defined in the compound of formula (IV).

### Scheme XI

A method for preparing the compound of formula (IV-P1) or the tautomer, mesomer, racemate, enantiomer, diastereomer thereof, or mixture thereof, or the pharmaceutically acceptable salt thereof according to the present disclosure, comprises the following step of: 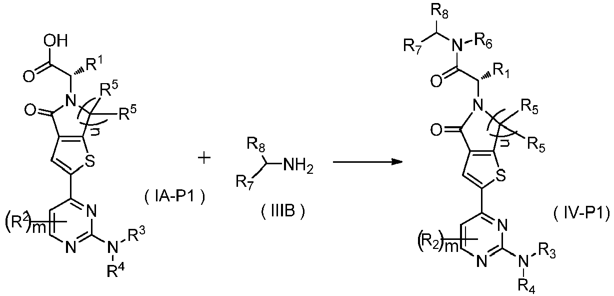 subjecting a compound of formula (IA-P1) and a compound of (IIIB) to a condensation reaction in the presence of an alkaline reagent to obtain the compound of formula (IV-P1), wherein: the alkaline reagent is preferably *N*,*N*-diisopropyl ethylamine; and R¹ to R⁸, m and n are as defined in the compound of formula (IV).

### Scheme XII

A method for preparing the compound of formula (IV-P2) or the tautomer, mesomer, racemate, enantiomer, diastereomer thereof, or mixture thereof, or the pharmaceutically acceptable salt thereof according to the present disclosure, comprises the following step of: 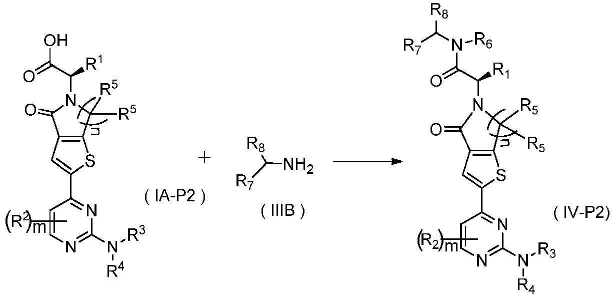 subjecting a compound of formula (IA-P2) and a compound of (IIIB) to a condensation reaction in the presence of an alkaline reagent to obtain the compound of formula (IV-P2), wherein: the alkaline reagent is preferably *N*,*N*-diisopropyl ethylamine; R¹ to R⁸, m and n are as defined in the compound of formula (IV); and the condensation agent used in the condensation reaction is preferably 2-(7-azabenzotriazol-1-yl)-*N*,*N*,*N*',*N*-tetramethyluronium hexafluorophosphate.

The alkaline reagent in the above reactions includes organic bases and inorganic bases. The organic bases include, but are not limited to, triethylamine, N,N-diisopropylethylamine, *n*-butyllithium, lithium diisopropylamide, potassium acetate, sodium *tert*-butoxide and potassium *tert*-butoxide. The inorganic bases include, but are not limited to, sodium hydride, potassium phosphate, sodium carbonate, sodium acetate, potassium acetate, potassium carbonate, cesium carbonate, sodium hydroxide, lithium hydroxide and potassium hydroxide; preferably, N,N-diisopropylethylamine.

The above reactions are carried out in a solvent. The solvent used includes, but is not limited to: acetic acid, methanol, ethanol, *n*-butanol, toluene, tetrahydrofuran, dichloromethane, petroleum ether, ethyl acetate, *n*-hexane, dimethyl sulfoxide, 1,4-dioxane, ethylene glycol dimethyl ether, water or N,N-dimethylformamide and the mixture thereof, preferably N,N-dimethylformamide.

The condensation agent in the above condensation reactions includes, but is not limited to 1-(3-dimethylaminopropyl)-3-ethylcarbodiimide hydrochloride, *N,N'-*dicyclohexyl carbodiimide, *N*,*N*'-diisopropyl carbodiimide, O-(benzotriazol-1-yl)-*N*,*N*,*N*',*N*-tetramethyluronium tetrafluoroborate, 1-hydroxybenzotriazole, 1-hydroxy-7-azobenzotriazole, O-(benzotriazol-1-yl)-*N*,*N*,*N*',*N*'-tetramethyluronium hexafluorophosphate, 2-(7-azobenzotriazole)-*N*,*N*,*N*',N'-tetramethyluronium hexafluorophosphate, 2-(7-azabenzotriazol-1-yl)-*N*,*N*,*N*',*N*-tetramethyluronium hexafluorophosphate, benzotriazol-1-yloxytris(dimethylamino)phosphonium hexafluorophosphate and benzotriazol-1-yl-oxytripyrrolyl phosphate hexafluorophosphate, preferably 2-(7-azabenzotriazol-1-yl)-*N*,*N*,*N*',*N*-tetramethyluronium hexafluorophosphate.

The details of one or more embodiments of the present disclosure are set forth in the above specification. Although any methods and materials similar or identical to those described herein can be used to perform or test the present disclosure, the preferred methods and materials are described below. Through the specification and claims, other features, purposes and advantages of the present disclosure will be apparent. In the specification and claims, unless the context clearly indicates otherwise, the singular form includes the plural referent. Unless otherwise defined, all technical and scientific terms used herein have the general meanings as understood by the person of ordinary skill in the art to which the present disclosure belongs. All patents and publications cited in the specification are incorporated by reference. The following examples are provided to more fully illustrate the preferred embodiments of the present disclosure. These examples should not be construed as limiting the scope of the present disclosure in any way, and the scope of the present disclosure is defined by the claims.

### DETAILED DESCRIPTION

### EXAMPLES

The structures of the compounds were identified by nuclear magnetic resonance (NMR) and/or mass spectrometry (MS). NMR shifts (δ) are given in 10⁻⁶ (ppm). NMR is determined by a Bruker AVANCE-400 machine. The solvents for determination are deuterated-dimethyl sulfoxide (DMSO-*d*₆), deuterated-chloroform (CDCl₃) and deuterated-methanol (CD₃OD), and the internal standard is tetramethylsilane (TMS).

MS was determined by an Agilent 1200 /1290 DAD- 6110/6120 Quadrupole MS liquid chromatograph/mass spectrometer (manufacturer: Agilent, MS model: 6110/6120 Quadrupole MS), waters ACQuity UPLC-QD/SQD (manufacturer: waters, MS model: waters ACQuity Qda Detector/waters SQ Detector), THERMO Ultimate 3000-Q Exactive (manufacturer: THERMO, MS model: THERMO Q Exactive).

High performance liquid chromatography (HPLC) was determined on an Agilent HPLC 1200DAD, Agilent HPLC 1200VWD and Waters HPLC e2695-2489 high pressure liquid chromatograph.

Chiral HPLC was determined on an Agilent 1260 DAD high performance liquid chromatograph.

Preparative HPLC was carried out on Waters 2545-2767, Waters 2767-SQ Detecor2, Shimadzu LC-20AP and Gilson GX-281 preparative chromatographs.

Chiral preparation was carried out on a Shimadzu LC-20AP preparative chromatograph.

CombiFlash rapid preparation instrument used was Combiflash Rf200 (TELEDYNE ISCO).

Yantai Huanghai HSGF254 or Qingdao GF254 silica gel plate was used as the thin layer silica gel chromatography (TLC) plate. The dimension of the silica gel plate used in TLC was 0.15 mm to 0.2 mm, and the dimension of the silica gel plate used in product purification was 0.4 mm to 0.5 mm.

Yantai Huanghai 200 to 300 mesh silica gel was generally used as a carrier for silica gel column chromatography.

The average kinase inhibition rates and IC₅₀ values were determined by a NovoStar microplate reader (BMG Co., Germany).

The known starting materials of the present disclosure can be prepared by the known methods in the art, or can be purchased from ABCR GmbH & Co. KG, Acros Organics, Aldrich Chemical Company, Accela ChemBio Inc., Dari Chemical Company etc.

Unless otherwise stated, the reactions can be carried out under argon atmosphere or nitrogen atmosphere.

Argon atmosphere or nitrogen atmosphere means that a reaction flask is equipped with an argon or nitrogen balloon (about1 L).

Hydrogen atmosphere means that a reaction flask is equipped with a hydrogen balloon (about1 L).

Pressurized hydrogenation reaction was performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, and the above operation was repeated three times.

CEM Discover-S 908860 type microwave reactor was used in microwave reactions.

Unless otherwise stated in the examples, the solution refers to an aqueous solution.

Unless otherwise stated in the examples, the reaction temperature is room temperature from 20°C to 30°C.

The single configuration in the examples can be confirmed by the synthesis from chiral raw materials or single crystal structure.

The reaction process in the examples was monitored by thin layer chromatography (TLC). The developing solvent used in the reactions, the eluent system in column chromatography and the developing solvent system in thin layer chromatography for purification of the compounds included: A: dichloromethane/methanol system, B: *n*-hexane/ethyl acetate system, C: petroleum ether/ethyl acetate system, D: acetone, E: dichloromethane/acetone system, F: ethyl acetate/dichloromethane system, G: ethyl acetate/dichloromethane/*n*-hexane, H: ethyl acetate/dichloromethane/acetone. The ratio of the volume of the solvent was adjusted according to the polarity of the compounds, and a small quantity of alkaline reagent such as triethylamine or acidic reagent such as acetic acid could also be added for adjustment.

### Example 1

(*S*)-2-(2-(5-Chloro-2-((tetrahydro-2*H*-pyran-4-yl)amino)pyrimidin-4-yl)-4-oxo-6,7-dihy drothieno[3,2-*c*]pyridin-5(4*H*)-yl)-*N*-(1-(3-chlorophenyl)-2-hydroxyethyl)acetamide 1

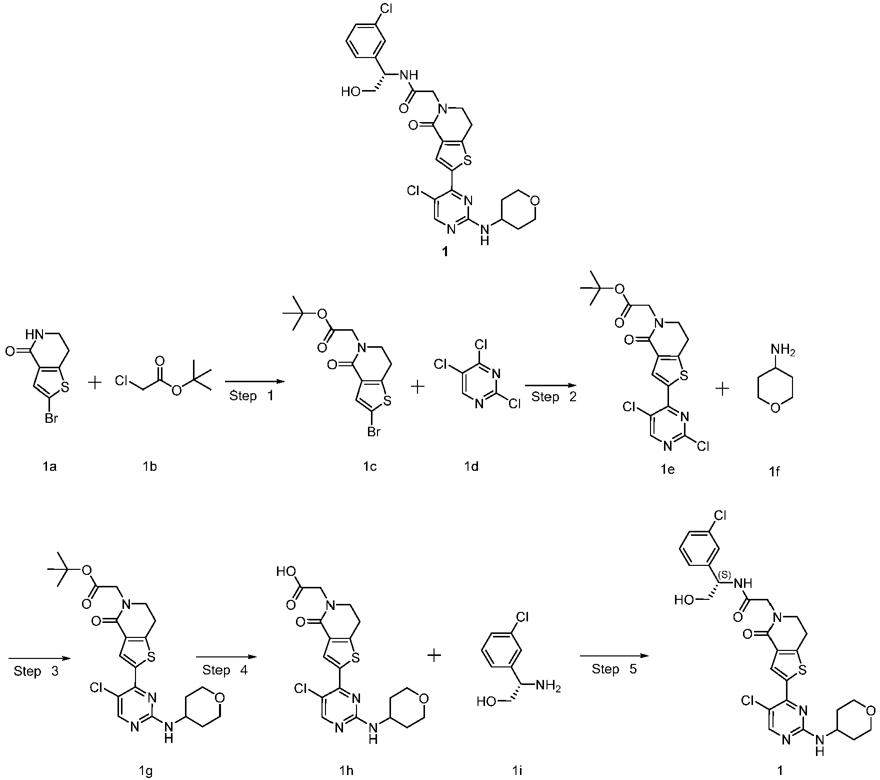

### Step 1

*tert-Butyl* 2-(2-bromo-4-oxo-6,7-dihydrothieno[3,2-*c*]pyridin-5(4*H*)-yl)acetate **1c** Compound 2-bromo-6,7-dihydrothieno[3,2-*c*]pyridin-4(5*H*)-one **1a** (600 mg, 2.58 mmol) was dissolved in 10 mL of tetrahydrofuran, followed by the addition of 1 M a solution of lithium hexamethyldisilazide in tetrahydrofuran (3.8 mL, 3.87 mmol). The reaction solution was stirred at -78°C for 1 hour, followed by the addition of *tert-butyl* 2-chloroacetate **1b** (622 mg, 4.13mmol). The reaction solution was slowly warmed up to room temperature and reacted for 15 hours. 30 mL of water was added, and the reaction solution was extracted with ethyl acetate (20 mL×3). The organic phase was washed with saturated sodium chlorate solution, dried over anhydrous sodium sulfate, and then concentrated under reduced pressure. The residue was purified by silica gel column chromatography with developing solvent system C to obtain the title compound **1c** (900 mg), yield: 99%.

MS m/z (ESI): 346.5 [M+1].

### Step 2

### tert-Butyl

### 2-(2-(2,5-dichloropyrimidin-4-yl)-4-oxo-6,7-dihydrothieno[3,2-c]pyridin-5(4H)-yl)acet ate 1e

2,4,5-Trichloropyrimidine **1d** (200 mg, 1.09 mmol), hexamethyldistannane (375 mg, 1.09 mmol), lithium chloride (65 mg, 1.09 mmol), and tetrakis(triphenylphosphine)palladium (163 mg, 0.15 mmol) were mixed and suspended in 6 mL of dioxane, and then the reaction solution was heated to 100°C and stirred for 8 hours. The reaction solution was cooled to room temperature, followed by the addition of compound **1c** (85 mg, 0.25 mmol), cuprous iodide (30 mg, 0.16 mmol) and bis(triphenylphosphine)palladium (II) dichloride (43 mg, 0.07 mmol). The reaction solution was heated to 105°C and stirred for 3 hours. 5 mL of saturated potassium fluoride solution was added, and the reaction solution was stirred for 2 hours, and then extracted with ethyl acetate. The organic phase was concentrated under reduced pressure. The residue was purified by thin layer chromatography with developing solvent system C to obtain the title compound **1e** (38 mg), yield: 25.3%.

MS m/z (ESI): 414.3 [M+1].

### Step 3

### tert-Butyl

### 2-(2-(5-chloro-2((tetrahydro-2H-pyran-4-yl)amino)pyrimidin-4-yl)-4-oxo-6,7-dihydroth ieno[3,2-c]pyridin-5(4H)-yl)acetate 1g

Compound **1e** (38 mg, 0.09 mmol) was dissolved in 2 mL tetrahydrofuran solvent, followed by the addition of tetrahydro-2*H*-pyran-4-amine **1f** (18 mg, 0.18 mmol, Shanghai Bide Pharmatech Ltd.) and *N*,*N*-diisopropyl ethylamine (60 mg, 0.45 mmol). The reaction solution was reacted at 120°C in microwave reactor for 1 hour, and then concentrated under reduced pressure. The residue was purified by thin layer chromatography with developing solvent system C to obtain title compound **1g** (30 mg), yield: 68.2%.

MS m/z (ESI): 479.3 [M+1].

### Step 4

### 2-(2-(5-Chloro-2-((tetrahydro-2H-pyran-4-yl)amino)pyrimidin-4-yl)-4-oxo-6,7-dihydrot hieno[3,2-c]pyridin-5(4H)-yl)acetic acid1h

Compound **1g** (18 mg, 37 µmol) was dissolved in 2 mL of dichloromethane, followed by the addition of trifluoroacetic acid (0.3 mL). The reaction solution was stirred for 2 hours, and then concentrated under reduced pressure to obtain the crude title compound **1h**, which was directly used in the next step.

MS m/z (ESI): 423.3 [M+1].

### Step 5

### (S)-2-(2-(5-Chloro-2((tetrahydro-2H-pyran-4-yl)amino)pyrimidin-4-yl)-4-oxo-6,7-di hydrothieno[3,2-c]pyridin-5(4H)-yl)-N-(1-(3-chlorophenyl)-2-hydroxyethyl)acetamide1

The crude compound **1h** (18 mg, 42 µmol), (S)-2-amino-2-(3-chlorophenyl) ethan-1-ol **1i** (14 mg, 81 µmol, Shanghai Bide Pharmatech Ltd.), 2-(7-azabenzotriazol-1-yl)-*N*,*N*,*N*',*N*'-tetramethyluronium hexafluorophosphate (15 mg, 1.09 mmol), and *N*,*N*-diisopropyl ethylamine (27 mg, 200 µmol) were dissolved in 2 mL of *N*,*N*-dimethyl formamide, and the reaction solution was stirred for 2 hours. The title compound 1 (5 mg) was obtainded by preparation and purification, yield: 20.4%.

MS m/z (ESI): 576.3 [M+1].

¹H NMR (400 MHz, CD₃OD): δ 8.45 (s, 1H), 8.22 (s, 1H), 7.49-7.52 (m, 1H), 7.20-7.22 (m, 3H), 4.98-4.99 (m, 1H), 3.97-4.01 (m, 3H), 3.88-3.91 (m, 4H), 3.72-3.75 (m, 4H), 3.57-3.61 (s, 2H), 1.96-1.98 (m, 2H), 1.58-1.61 (m, 2H).

### Example 2

### (S)-2-(2-(5-Chloro-2-((tetrahydro-2H-pyran-4-yl)amino)pyrimidin-4-yl)-6,6-dimethyl-4 -oxo-4,6-dihydro-5H-thieno[2,3-c]pyrrol-5-yl)-N-(1-(3-chlorophenyl)-2-hydroxyethyl)a cetamide 2

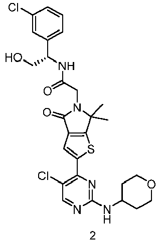

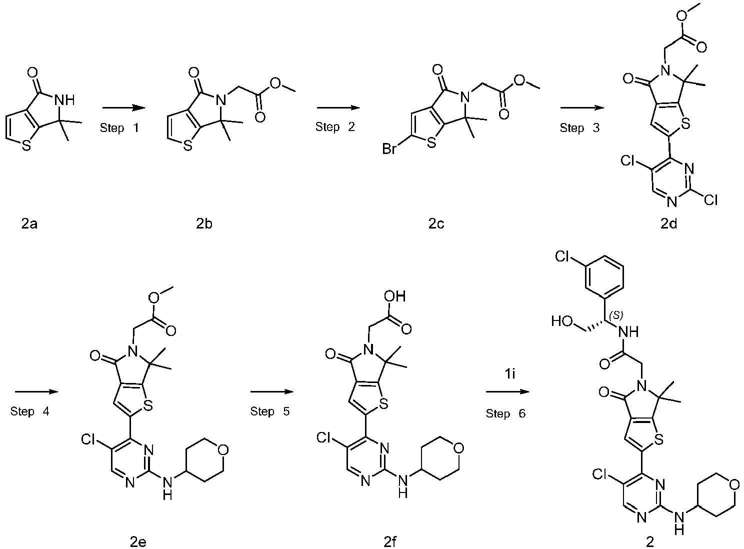

### Step 1

6,6-Dimethyl-5,6-dihydro-4*H*-thieno[2,3-*c*]pyrrol-4-one **2b** 6,6-Dimethyl-5,6-dihydro-4*H*-thieno[2,3-*c*]pyrrol-4-one **2a** (180 mg, 1 mmol, prepared according to the method disclosed in patent application "WO2016106029") was dissolved in 10 mL of *N*,*N*-dimethyl formamide, followed by the addition of sodium hydrogen (82 mg, 2.1 mmol) at 0°C, and the reaction solution was stirred for 1 hour. Methyl chloroacetate (233 mg, 2.1 mmol) was added, and the reaction solution was stirred overnight. Ethyl acetate (150 mL) was added, and the reaction solution was washed with water. The organic phase was dried over anhydrous sodium sulfate, and concentrated under reduced pressure. The residue was purified by silica gel column chromatography with developing solvent system C to obtain the title compound **2b** (130 mg), yield: 51%.

MS m/z (ESI): 240.1 [M+1].

### Step 2

### Methyl

2-(2-bromo-6,6-dimethyl-4-oxo-4,6-dihydro-5*H*-thieno[2,3-c]pyrrol-5-yl)acetate **2c** Compound **2b** (130 mg, 0.54 mmol) was dissolved in 10 mL of acetonitrile, followed by the addition of *N*-bromosuccinimide (97 mg, 0.54 mmol) at 0°C. The reaction solution was stirred for 16 hours, and then concentrated to dryness by rotary evaporation. The residue was purified by silica gel column chromatography with developing solvent system C to obtain the title compound **2c** (120 mg), yield: 69%.

MS m/z (ESI): 318.2 [M+1].

### Step 3

### Methyl

### 2-(2-(2,5-dichloropyrimidin-4-yl)-6,6-dimethyl-4-oxo-4,6-dihydro-5H-thieno[2,3-c]pyrr ol-5-yl)acetate 2d

Compound **2c** (110 mg, 0.35 mmol) was dissloved in 20 mL of dioxane, followed by the addition of 4,4,4',4',5,5,5',5'-octamethyl-2,2'-bis(1,3,2-dioxaborolane) (210 mg, 0.83 mmol), potassium acetate (100 mg, 1.0 mmol), and [1,1'-bis(diphenylphosphino)ferrocene]palladium (II) dichloride (25 mg, 0.03 mmol). After the addition was completed, the reaction system was purged with argon three times, and stirred at 90°C for 2 hours. The reaction solution was cooled to room temperature, followed by the addition of 2,4,5-trichloropyrimidine (95 mg, 0.52 mmol), anhydrous potassium carbonate (72 mg, 0.52 mmol), and tetrakis(triphenylphosphine)palladium (40 mg, 0.03 mmol). 0.3 mL of water was added dropwise. After the addition was completed, the reaction system was purged with argon three times, and stirred at 90°C for 2 hours. The reaction solution was cooled to room temperature and filtered. The filtrate was concentrated to obtain compound **2d** (60 mg). The product was directly used in the next step without purification.

MS m/z (ESI): 386.1 [M+1].

### Step 4

### Methyl

### 2-(2-(5-chloro-2-((tetrahydro-2H-pyran-4-yl)amino)pyrimidin-4-yl)-6,6-dimethyl-4-oxo -4,6-dihydro-5H-thieno[2,3-c]pyrrol-5-yl)acetate 2e

The crude compound **2d** (60 mg, 0.16 mmol) was dissolved in 5 mL tetrahydrofuran, followed by the addition of tetrahydropyran-4-amine (24 mg, 0.24 mmol) and *N*,*N*-diisopropyl ethylamine (100 mg, 1.0 mmol). The reaction solution was reacted at 100°C in microwave reactor for 1 hour, and then concentrated under reduced pressure. The residue was purified by silica gel column chromatography with developing solvent system C to obtain the title compound **2e** (30 mg), yield: 43%.

MS m/z (ESI): 451.1 [M+1].

### Step 5

### 2-(2-(5-Chloro-2-((tetrahydro-2H-pyran-4-yl)amino)pyrimidin-4-yl)-6,6-dimethyl-4-ox o-4,6-dihydro-5H-thieno[2,3-c]pyrrol-5-yl)acetic acid 2f

Compound **2e** (30 mg, 0.06 mmol) was dissolved in 3 mL of methanol and 0.5 mL of water, followed by the addition of lithium hydroxide (16 mg, 0.7 mmol), and the reaction solution was reacted for 1 hour. 1 M diluted hydrochloric acid was added dropwise, and the reaction solution was adjusted to pH 3. The title crude compound **2f** (29 mg) was obtained after concentration. The product was directly used in the next step without purification.

MS m/z (ESI): 437.2 [M+1].

### Step 6

### (S)-2-(2-(5-Chloro-2-((tetrahydro-2H-pyran-4-yl)amino)pyrimidin-4-yl)-6,6-dimethyl-4 -oxo-4,6-dihydro-5H-thieno[2,3-c]pyrrol-5-yl)-N-(1-(3-chlorophenyl)-2-hydroxyethyl)a cetamide 2

The crude compound **2f** (29 mg, 0.06 mmol) wasdisslolved in 5 mL of *N*,*N*-dimethyl formamide, followed by the addition of 2-(7-azabenzotriazol-1-yl)-*N*,*N*,*N*',*N*'-tetramethyluronium hexafluorophosphate (15 mg, 0.06 mmol), compound **1i** (11 mg, 0.06 mmol, Shanghai Bide Pharmatech Ltd.), and *N*,*N*-diisopropyl ethylamine (100 mg, 1.0 mmol). The reaction solution was reacted overnight, and then filtered. The residue was purified by silica gel column chromatography with developing solvent system A to obtain **2** (5 mg), yield: 13%.

MS m/z (ESI): 590.1 [M+1].

¹H NMR (400 MHz, CD₃OD): δ 8.30 (s, 2H), 7.41-7.28 (m, 4H), 4.99-4.97 (m, 1H), 4.25 (d, 2H), 4.02-4.00 (m, 3H), 3.80-3..78 (m, 2H), 3.76-3.58 (m, 2H), 1.66-1.58 (m, 2H), 1.66-1.58 (m, 8H).

### Examples 3-P1 and 3-P2

### (S)-2-(2-(5-Chloro-2-((tetrahydro-2H-pyran-4-yl)amino)pyrimidin-4-yl)-4-oxo-6,7-dihy drothieno[3,2-c]pyridin-5(4H)-yl)-N-((S)-2-hydroxy-1-(m-methylphenyl)ethyl)propiona mide 3-P1

### (R)-2-(2-(5-Chloro-2-((tetrahydro-2H-pyran-4-yl)amino)pyrimidin-4-yl)-4-oxo-6,7-dihy drothieno[3,2-c]pyridin-5(4H)-yl)-N-((S)-2-hydroxy-1-(m-methylphenyl)ethyl)propiona mide 3-P2

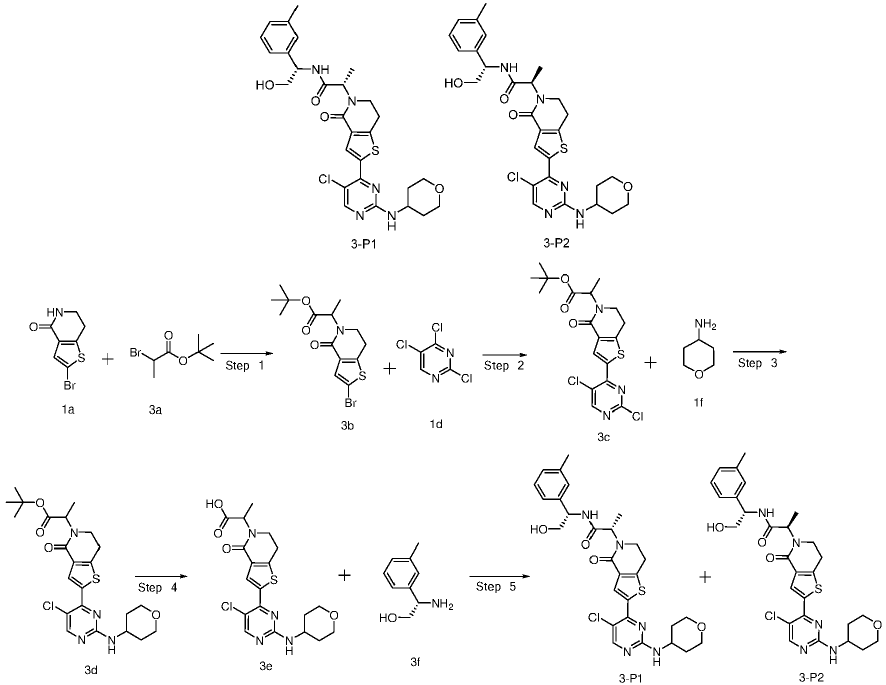

### Step 1

*tert*-Butyl 2-(2-bromo-4-oxo-6,7-dihydrothieno[3,2-c]pyridin-5(4*H*)-yl)propionate **3b** Compound **1a** (800 mg, 3.44 mmol) was dissolved in 10 mL of tetrahydrofuran, followed by the addition of 1 M a solution of lithium hexamethyldisilazide in tetrahydrofuran (5.2 mL, 5.20 mmol). The reaction solution was stirred at -78°C for 1 hour, followed by the addition of *tert-Butyl* 2-bromopropionate **3a** (622 mg, 4.13 mmol, Tokyo Chemical Industry (Shanghai) Co., Ltd.). The reaction solution was slowly warmed up to room temperature, and then reacted for 15 hours. 30 mL of water was added, and the reaction solution was extracted with ethyl acetate (20 mL × 3). The organic phase was washed with saturated sodium chloride solution, dried over anhydrous sodium sulfate, and then concentrated under reduced pressure. The residue was purified by silica gel column chromatography with developing solvent system C to obtain the title compound **3b** (1.10 g), yield: 88.6%.

MS m/z (ESI): 360.2 [M+1].

### Step 2

### tert-Butyl

### 2-(2-(2,5-dichloropyrimidin-4-yl)-4-oxo-6,7-dihydrothieno[3,2-c]pyridin-5(4H)-yl)prop ionate 3c

Compound **1d** (200 mg, 1.09 mmol), hexarnethyldiscannane (375 mg, 1.09 mmol), lithium chloride (65 mg, 1.09 mmol), tetrakis(triphenylphosphine)palladium (163 mg, 0.15 mmol) were mixed and suspended in 6 mL of dioxane, and then the reaction solution was heated to 100°C and stirred for 8 hours. The reaction solution was cooled to room temperature, followed by the addition of compound **3b** (115 mg, 0.32 mmol), cuprous iodide (30 mg, 0.16 mmol), and bis(triphenylphosphine) palladium (II) dichloride (43 mg, 0.07 mmol). The reaction solution was heated to 105°C and stirred for 3 hours. Saturated potassium fluoride solution was added, and the reaction solution was stirred for 2 hours, and then extracted with ethyl acetate. The organic phases were combined and concentrated under reduced pressure. The residue was purified by thin layer chromatography with developing solvent system C to obtain the title compound **3c** (50 mg), yield: 31.3%.

MS m/z (ESI): 428.3 [M+1].

### Step 3

### tert-Butyl

### 2-(2-(5-chloro-2-((tetrahydro-2H-pyran-4-yl)amino)pyrimidin-4-yl)-4-oxo-6,7-dihydro thieno[3,2-c]pyridin-5(4H)-yl)propionate 3d

Compound **3c** (50 mg, 0.12 mmol) was dissolved in 2 mL tetrahydrofuran, followed by the addition of 4-aminotetrahydropyran **1f** (23 mg, 0.24 mmol) and *N*,*N*-diisopropyl ethylamine (75 mg, 0.58 mmol). The reaction solution was reacted at 120°C in microwave reactor for 1 hour, and then concentrated under reduced pressure. The residue was purified by thin layer chromatography with developing solvent system C to obtain the title compound **3d** (50 mg), yield: 86.8%.

MS m/z (ESI): 493.3 [M+1].

### Step 4

### 2-(2-(5-Chloro-2-((tetrahydro-2H-pyran-4-yl)amino)pyrimidin-4-yl)-4-oxo-6,7-dihydro thieno[3,2-c]pyridin-5(4H)-yl)propanoic acid 3e

Compound **3d** (20 mg, 40 µmol) was dissolved in 2 mL of dichloromethane, followed by the addition of trifluoroacetic acid (0.7 mL), and the reaction solution was stirred for 2 hours. The reaction solution was concentrated under reduced pressure to obtain the crude title compound **3e** (40 mg). The product was directly used in the next step without purification.

MS m/z (ESI): 437.3 [M+1].

### Step 5

### (S)-2-(2-(5-Chloro-2-((tetrahydro-2H-pyran-4-yl)amino)pyrimidin-4-yl)-4-oxo-6,7-dihy drothieno[3,2-c]pyridin-5(4H)-yl)-N-((S)-2-hydroxy-1-(m-methylphenyl)ethyl)propiona

### mide 3-P1

### (R)-2-(2-(5-Chloro-2-((tetrahydro-2H-pyran-4-yl)amino)pyrimidin-4-yl)-4-oxo-6,7-dihy drothieno[3,2-c]pyridin-5(4H)-yl)-N-((S)-2-hydroxy-1-(m-methylphenyl)ethyl)propiona mide 3-P2

The crude compound **3e** (40 mg, 91 µmol), (S)-2-amino-2-(m-methylphenyl) ethan-1-ol **3f** (27 mg, 180 µmol, Shanghai Bide Pharmatech Ltd.), 2-(7-azabenzotriazol-1-yl)-*N*,*N*,*N*',*N*'-tetramethyluronium hexafluorophosphate (40 mg, 106 µmol), and *N*,*N*-diisopropyl ethylamine (59 mg, 456 µmol) were dissolved in 2mL *N*,*N*-dimethyl formamide. The reaction solution was stirred for 2 hours, and then concentrated under reduced pressure. The residue was purified by preparative HPLC (instrument model: Gilson 281, chromatographic column: Sharpsil-T, Prep 21.2^{∗}150 mm, 5 µm, C18; mobile phase: A-water (0.1% trifluoroacetic acid), B-acetonitrile; flow rate: 30 mL/min; column temperature: room temperature) to obtain the title compounds **3-P1** and **3-P2** (11 mg, 9 mg).

### 3-P1 Single configuration compound (shorter retention time)

MS m/z (ESI): 570.1 [M+1].

HPLC analysis: Retention time 11.21 min, purity: 98.2% (chromatographic column: Sharpsil-T, Prep 21.2^{∗}150 mm; 5 µm; mobile phase: A-water (0.1% trifluoroacetic acid), B-acetonitrile, gradient ratio: A 5%-95%).

¹H NMR (400 MHz, CD₃OD): δ 8.45 (s, 1H), 8.31 (s, 1H), 7.03-7.19 (m, 4H), 5.36-5.39 (m, 1H), 4.96-4.98 (m, 1H), 3.97-4.01 (m, 3H), 3.74-3.77 (m, 2H), 3.52-3.56 (m, 1H), 3.51-3.53 (m, 3H), 3.01-3.03 (m, 2H), 2.25 (s, 3H), 2.02 (d, 2H), 1.59-1.63 (m, 2H), 1.44 (d, 3H).

### 3-P2 Single configuration compound (longer retention time)

MS m/z (ESI): 570.3 [M+1].

HPLC analysis: Retention time 12.14 min, purity: 96.2% (chromatographic column: Sharpsil-T, Prep 21.2^{∗}150 mm; 5 µm; mobile phase: A-water (0.1% trifluoroacetic acid), B-acetonitrile, gradient ratio: A 5%-95%).

¹H NMR (400 MHz, CD₃OD): δ 8.46 (s, 1H), 8.27 (s, 1H), 7.07-7.23 (m, 4H), 5.32-5.35 (m, 1H), 4.96-4.98 (m, 1H), 3.97-4.01 (m, 3H), 3.73-3.76 (m, 4H), 3.52-3.56 (m, 2H), 3.22-3.25 (m, 1H), 3.15-3.17 (m, 1H), 2.33 (s, 3H), 2.01-2.03 (m, 2H), 1.59-1.63 (m, 2H), 1.46 (d, 3H).

### Examples 4-P1 and 4-P2

### (S)-2-(2-(5-Chloro-2-((tetrahydro-2H-pyran-4-yl)amino)pyrimidin-4-yl)-4-oxo-6,7-di hydrothieno[3,2-c]pyridin-5(4H)-yl)-N-((S)-1-(3-chlorophenyl)-2-hydroxyethyl)propion amide 4-P1

### (R)-2-(2-(5-Chloro-2((tetrahydro-2H-pyran-4-yl)amino)pyrimidin-4-yl)-4-oxo-6,7-di hydrothieno[3,2-c]pyridin-5(4H)-yl)-N-((S)-(3-chlorophenyl)-2-hydroxyethyl)propiona mide 4-P2

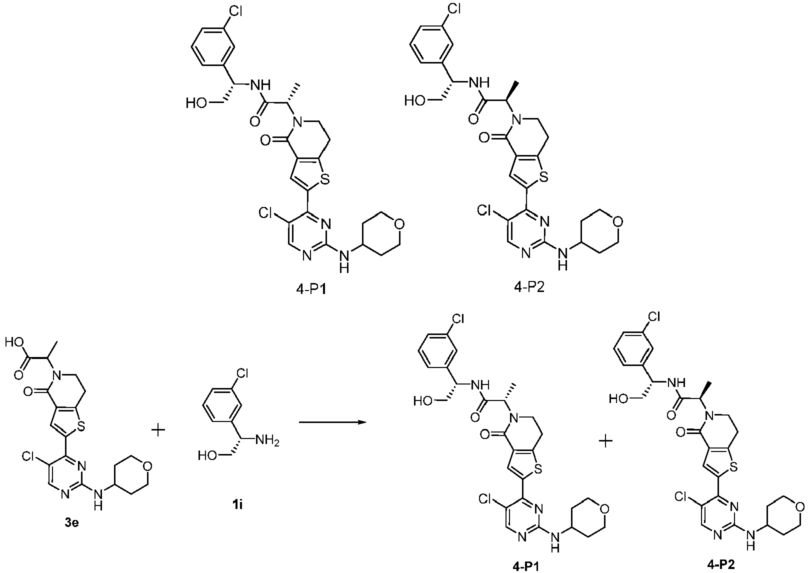

The crude compound **3e** (53 mg, 121 µmol), compound **1i** (50 mg, 240 µmol), 2-(7-azabenzotriazol-1-yl)-*N*,*N*,*N*',*N*'-tetramethyluronium hexafluorophosphate (75 mg, 180 µmol), and *N*,*N*-diisopropyl ethylamine (78 mg, 600 µmol) were dissolved in 2 mL of *N*,*N*-dimethyl formamide. The reaction solution was stirred for 2 hours, and then concentrated under reduced pressure. The residue was purified by preparative HPLC (instrument model: Gilson 281; chromatographic column: Sharpsil-T, Prep 21.2^{∗}150 mm, 5 µm, C18; mobile phase: A-water (10 mM ammonium bicarbonate), B-acetonide; flow rate: 30 mL/min; column temperature: room temperature) to obtain the title compounds **4-P1** and **4-P2** (15 mg, 15 mg).

### 4-P1 Single configuration compound (shorter retention time)

MS m/z (ESI): 590.3 [M+1].

HPLC analysis: Retention time 10.75 min, purity: 98.6% (chromatographic column: Sharpsil-T, Prep 21.2^{∗}150 mm, 5 µm; mobile phase: A-water (10 mM ammonium bicarbonate), B-acetonitrile, gradient ratio: A 5%-95%).

¹H NMR (400 MHz, CD₃OD): δ 8.43 (s, 1H), 8.26 (s, 1H), 7.35 (s, 1H), 7.22-7.24 (m, 3H), 5.30-5.35 (m, 1H), 4.98-4.99 (m, 1H), 3.95-3.97 (m, 3H), 3.76-3.78 (m, 2H), 3.70-3.72 (m, 1H), 3.55-3.57 (m, 3H), 3.04-3.06 (m, 2H), 2.00-2.02 (m, 2H), 1.58-1.62

(m, 2H), 1.44 (d, 3H).

### 4-P2 Single configuration compound (longer retention time)

MS m/z (ESI): 590.3 [M+1].

HPLC analysis: Retention time 11.75 min, purity: 99.1% (chromatographic column: Sharpsil-T, Prep 21.2^{∗}150 mm, 5 µm; mobile phase: A-water (10 mM ammonium bicarbonate), B-acetonitrile, gradient ratio: A 5%-95%).

¹H NMR (400 MHz, CD₃OD): δ 8.40 (s, 1H), 8.23 (s, 1H), 7.39 (s, 1H), 7.24-7.27 (m, 3H), 5.30-5.34 (m, 1H), 4.98-4.99 (m, 1H), 4.00-4.02 (m, 3H), 3.76-3.78 (m, 4H), 3.50-3.53 (m, 2H), 3.22-3.26 (m, 1H), 3.13-3.15 (m, 1H), 2.00-2.02 (m, 2H), 1.58-1.62 (m, 2H), 1.43 (d, 3H).

### Example 5

### 2-(2-(5-Chloro-2-((tetrahydro-2H-pyran-4-yl)amino)pyrimidin-4-yl)-6,6-dimethyl-4-oxo-4,6-dihydro-5H-thieno[2,3-c]pyrrol-5-yl)-N-((S)-1-(3-chlorophenyl)-2-hydroxy ethyl)propionamide 5

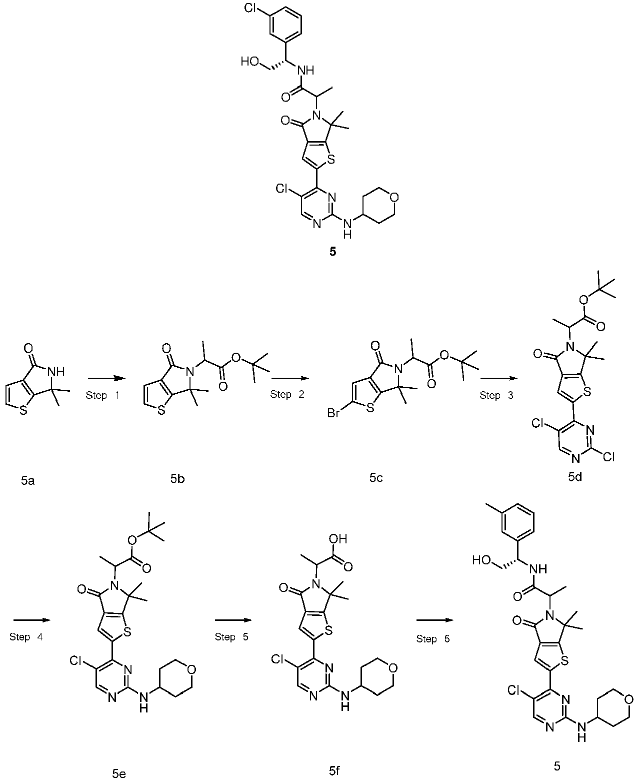

### Step 1

### tert-Butyl 2-(6,6-dimethyl-4-oxo-4,6-dihydro-5H-thieno[2,3-c]pyrrol-5-yl)propionate 5b

6,6-Dimethyl-5,6-dihydro-4*H*-thieno[2,3-*c*]pyrrol-4-one **5a** (1 g, 6 mmol, prepared according to the method disclosed in the patent application "WO2016106029") was dissolved in 10 mL of *N*,*N*-dimethyl formamide, followed by the addition of sodium hydrogen (458 mg, 12 mmol) at 0°C, and the reaction solution was stirred at 0°C for 1 hour. *tert-Butyl* 2-bromopropionate (1.9 g, 9 mmol) was added, and the reaction solution was stirred overnight. Ethyl acetate (200 mL) was added, and the reaction solution was washed with water. The organic phase was dried over anhydrous sodium sulfate, and concentrated under reduced pressure. The residue was purified by thin layer chromatography with developing solvent system A to obtain the title compound **5b** (1.2 g), yield: 67.9%.

MS m/z (ESI): 296.1 [M+1].

### Step 2

### tert-Butyl

### 2-(2-bromo-6,6-dimethyl-4-oxo-4,6-dihydro-5H-thieno[2,3-c]pyrrol-5-yl)propionate 5c

Compound **5b** (1.2 g, 4.0 mmol) was dissolved in 20 mL of acetonitrile, followed by the addition of *N*-bromosuccinimide (730 mg, 4.0 mmol) at 0°C. The reaction solution was stirred for 16 hours, and then concentrated. The residue was purified by thin layer chromatography with developing solvent system A to obtain the title compound **5c** (1.2 g), yield: 78.9%.

MS m/z (ESI): 374.2 [M+1].

### Step 3

### tert-Butyl

### 2-(2-(2,5-dichloropyrimidin-4-yl)-6,6-dimethyl-4-oxo-4,6-dihydro-5H-thieno[2,3]pyrrol -5-yl)propionate 5d

Compound **5c** (374 mg, 1 mmol) was dissolved in 20 mL dioxane, followed by the addition of 4,4,4',4',5,5,5',5'-octamethyl-2,2'-bis(1,3,2-dioxaborolane) (609 mg, 2.4 mmol), potassium acetate (294 mg, 3.0 mmol) and [1,1'-bis(diphenylphosphino)ferrocene] platinum (II) dichloride (73 mg, 0.1 mmol). After the addition was completed, the reaction system was purged with argon three times, and stirred at 90°C for 2 hours. The reaction solution was cooled to room temperature, followed by the addition of 2,4,5-trichloropyrimidine (275 mg, 1.5 mmol), anhydrous potassium carbon (72 mg, 0.52 mmol), and tetrakis(triphenylphosphine)palladium (207 mg, 1.5 mmol). 1 mL of water was added dropwise. After the addition was completed, the reaction system was purged with argon three times and stirred at 90°C for 2 hours. The reaction solution was cooled to room temperature and filtered. The filtrate was concentrated, and the residue was purified by thin layer chromatography with developing solvent system C to obtain compound **5d** (150 mg), yield: 34%.

MS m/z (ESI): 442.1 [M+1].

### Step 4

### tert-Butyl

### 2-(2-(5-chloro-2-((tetrahydro-2H-pyran-4-yl)amino)pyrimidin-4-yl)-6,6-dimethyl-4-oxo -4,6-dihydro-5H-thieno[2,3-c]pyrrol-5-yl)propionate 5e

The crude compound **5d** (150 mg, 0.34 mmol) was dissolved in 5 mL of tetrahydrofuran, followed by the addition of compound **1f** (52 mg, 0.51 mmol) and *N*,*N*-diisopropyl ethylamine (100 mg, 1.0 mmol). The reaction solution was reacted at 100°C in microwave reactor for 1 hour, and then concentrated under reduced pressure. The residue was purified by thin layer chromatography with developing solvent system A to obtain compound **5e** (80 mg), yield: 46.5%.

MS m/z (ESI): 407.1 [M+1].

### Step 5

### 2-(2-(5-Chloro-2-((tetrahydro-2H-pyran-4-yl)amino)pyrimidin-4-yl)-6,6-dimethyl-4-oxo-4,6-dihydro-5H-thieno[2,3-c]pyrrol-5-yl)propanoic acid 5f

Compound **5e** (80 mg, 0.16 mmol) was dissolved in 5 mL of dichloromethane, followed by the dropwise addition of 1 mL of trifluoroacetic acid. The reaction solution was reacted for 1 hour, and then concentrated to obtain the title compound **5f** (71 mg), yield: 100%.

MS m/z (ESI): 450.8 [M+1].

### Step 6

### 2-(2-(5-Chloro-2-((tetrahydro-2H-pyran-4-yl)amino)pyrimidin-4-yl)-6,6-dimethyl-4-oxo-4,6-dihydro-5H-thieno[2,3-c]pyrrol-5-yl)-N-((S)-2-hydroxy-1-(m-methylphenyl)eth yl)propionamide 5

Compound **5f** (71 mg, 0.16 mmol) was dissolved in 5 mL of *N,N*-dimethyl formamide, followed by the addition of 2-(7-azabenzotriazol-1-yl)-N,N,N',N'-tetramethyluronium hexafluorophosphate (44 mg, 0.19 mmol), compound **3f** (24 mg, 0.16 mmol) and *N,N*-diisopropyl ethylamine (41 mg, 0.3 mmol). The reaction solution was reacted overnight, and then concentrated. The residue was purified by thin layer chromatography with solvent system A to obtain compound **5** (15 mg), yield: 16%.

MS m/z (ESI): 583.8 [M+1].

¹H NMR (400 MHz, CD₃OD): δ 8.32 (s, 2H), 7.25-7.02 (m, 4H), 4.99-4.97 (m, 1H), 4.36-4.27 (m, 1H), 4.05-3.95 (m, 3H), 3.81-3.70 (m, 2H), 3.63-3.50 (m, 2H), 2.30 (d, 3H), 2.05-1.95 (m, 2H), 1.78-1.74 (m, 3H), 1.68-1.57 (m, 8H).

### Example 6

### (S)-N-(1-(3-Chlorophenyl)-2-hydroxyethyl)-2-(4-oxo-2-(2-((tetrahydro-2H-pyran-4-yl) amino)pyrimidin-4-yl)-6,7-dihydrothieno[3,2-c]pyridin-5(4H)-yl)acetamide 6

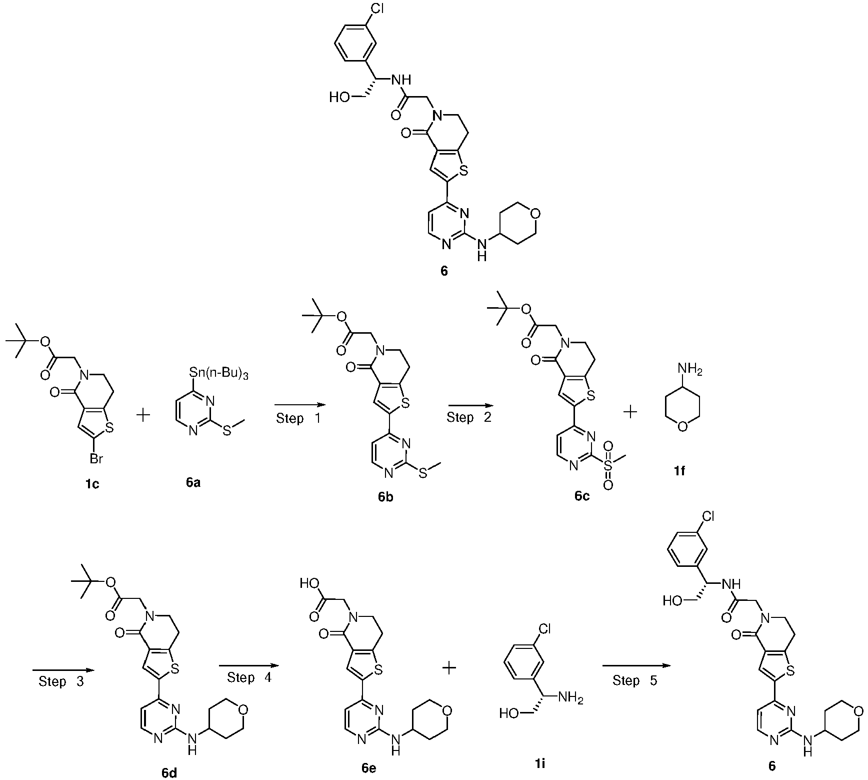

### Step 1

### tert-Butyl

### 2-(2-(2-(methylthio)pyrimidin-4-yl)-4-oxo-6,7-dihydrothieno[3,2-c]pyridin-5(4H)-yl)ac etate 6b

2-(Methylthio)-4-(*n*-butyltin)pyrimidine **6a** (60 mg, 0.14 mmol, Tokyo Chemical Industry (Shanghai) Co., Ltd.), compound **1c** (50 mg, 0.14 mmol), cuprous iodide (10 mg, 0.05 mmol), and bis(triphenylphosphine)palladium (II) dichloride (20 mg, 0.03 mmol) were heated to 105°C and stirred for 3 hours. 5 mL of saturated potassium fluoride solution was added, and the reaction solution was stirred for 2 hours, and then extracted with ethyl acetate. The organic phases were combined and concentrated under reduced pressure. The residue was purified by thin layer chromatography with developing solvent system C to obtain the title compound **6b** (15 mg), yield: 26.5%.

MS m/z (ESI): 392.3 [M+1].

### Step 2

### tert-Butyl

### 2-(2-(2-(methylsulfonyl)pyrimidin-4-yl)-4-oxo-6,7-dihydrothieno[3,2-c]pyridin-5(4H)-yl)acetate 6c

Compound **6b** (15 mg, 0.04 mmol) was dissolved in 2 mL of dichloromethane, followed by the addition of m-chloroperoxobenzoic acid (19 mg, 0.09 mmol). The reaction solution was reacted for 3 hours, and then washed with saturated sodium thiosulfate solution and saturated sodium chloride solution. The organic phases were combined, dried and concentrated under reduced pressure to obtain the title compound **6c** (20 mg). The product was directly used in the next step without purification.

MS m/z (ESI): 424.3 [M+1].

### Step 3

### tert-Butyl

### 2-(4-oxo-2-(2-((tetrahydro-2H-pyran-4-yl)amino)pyrimidin-4-yl)-6,7-dihydrothieno[3,2 -c]pyridin-5(4H)-yl)acetate 6d

The crude compound **6c** (10 mg, 23 µmol) was dissolved in 2 mL of tetrahydrofuran, followed by the addition of compound **1f** (11 mg, 0.108 mmol) and *N*,*N*-diisopropyl ethylamine (15 mg, 0.108 mmol). The reaction was carried out in microwave reactor at 120°C for 1 hour, and then concentrated under reduced pressure. The residue was purified by thin layer chromatography with developing solvent system C to obtain the title compound for **6d** (5 mg), yield: 47.6%.

MS m/z (ESI): 445.3 [M+1].

### Step 4

### 2-(4-Oxo-2-(2-((tetrahydro-2H-pyran-4-yl)amino)pyrimidin-4-yl)-6,7-dihydrothieno[3, 2-c]pyridin-5(4H)-yl)acetic acid 6e

Compound **6d** (5 mg, 11 µmol) was dissolved in 1 mL of dichloromethane, followed by the addition of trifluoroacetic acid (0.3 mL). The reaction was stirred for 2 hours, and then concentrated under reduced pressure to obtain the crude title compound **6e** (10 mg). The product was directly used in the next step without purification.

MS m/z (ESI): 389.3 [M+1].

### Step 5

### (S)-N-(1-(3-Chlorophenyl)-2-hydroxyethyl)-2-(4-oxo-2-(2-((tetrahydro-2H-pyran-4-yl) amino)pyrimidin-4-yl)-6,7-dihydrothieno[3,2-c]pyridin-5(4H)-yl)acetamide 6

The crude compound **6e** (5 mg, 13 µmol), compound **1i** (5 mg, 25 µmol), 2-(7-azabenzotriazol-1-yl)-N,N,N',N'-tetramethyluronium hexafluorophosphate (10 mg, 13 µmol), and *N,N*-diisopropyl ethylamine (8 mg, 60 µmol) were dissolved in 1 mL of *N,N*-dimethyl formamide, and the reaction solution was stirred for 2 hours. The title compound **6** (4 mg) was obtained by preparation and purification, yield: 50.1%.

MS m/z (ESI): 542.3 [M+1].

¹H NMR (400 MHz, CD₃OD): δ 7.50 (s, 2H), 7.37-7..42 (m, 5H), 5.10-5.13 (m, 1H), 4.59 (s, 1H), 4.48 (t, 2H), 3.92-3.95 (m, 2H), 3.78-3.80 (m, 2H), 3.49-3.51 (m, 2H), 3.01 (s, 4H), 1..35-1.37 (m, 4H).

### Example 7

### (S)-N-(2-Hydroxy-1-(m-methylphenyl)ethyl)-2-(2-(5-methyl-2-((1-methyl-1H-pyrazol-5 -yl)amino)pyrimidin-4-yl)-4-oxo-6,7-dihydrothieno[3,2-c]pyridin-5(4H)-yl)acetamide 7

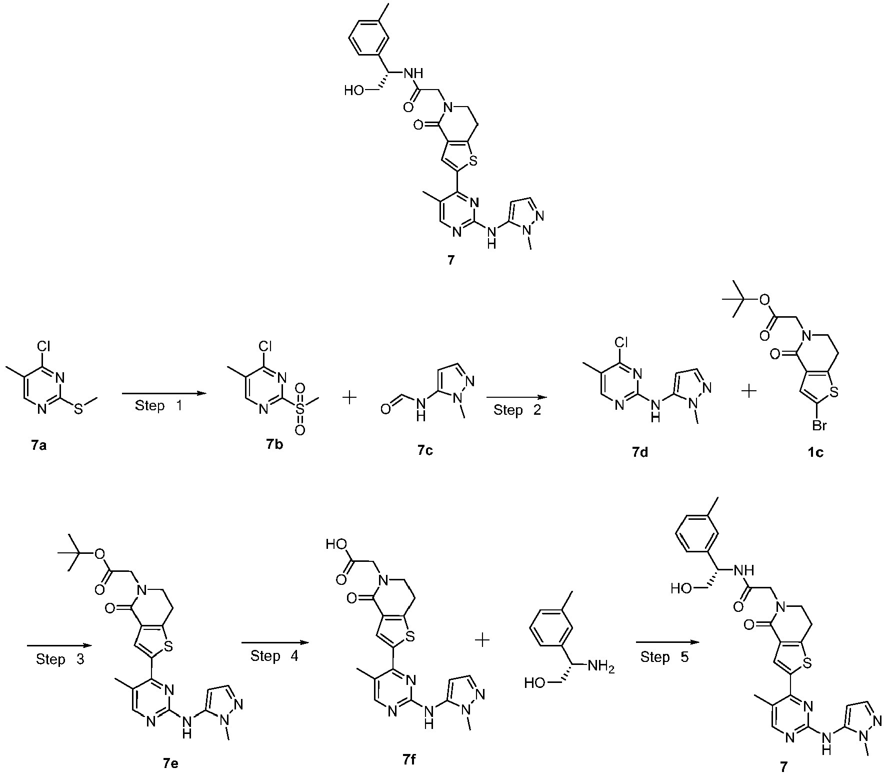

### Step 1

### 4-Chloro-5-methyl-2-(methylsulfonyl)pyrimidine 7b

Compound 4-chloro-5-methyl-2-(methylthio)pyrimidine **7a** (500 mg, 2.86 mmol, Tokyo Chemical Industry (Shanghai) Co., Ltd.) was dissolved in 10 mL of dichloromethane, followed by the addition of m-Chloroperoxobenzoic acid (1.270 g, 6.3 mmol). The reaction solution was stirred for 2 hours, and then washed with saturated sodium thiosulfate solution and saturated brine, dried over anhydrous sodium sulfate and concentrated under reduced pressure to obtain the crude title compound **7b** (510 mg). The product was directly used in the next step without purification.

MS m/z (ESI): 207.2 [M+1].

### Step 2

### 4-Chloro-5-methyl-N-(1-methyl-1H-pyrazol-5-yl)pyrimidin-2-amine 7d

*N*-(1-methyl-1*H*-pyrazol-5-yl)formamide **7c** (270 mg, 2.15 mmol, prepared according to the method disclosed in patent application "WO2017/80979") was dissolved in *N*,*N*-dimethyl formamide, followed by the addition of sodium hydride (60%, 250 mg, 6.5 mmol) at 0°C. The reaction solution was stirred for 0.5 hours, followed by the addition of compound **7b** (445 mg, 2.15 mmol). The reaction solution was further reacted for 2 hours. 20 mL of water was added, and the reaction solution was extracted with ethyl acetate. The organic phases were combined and concentrated under reduced pressure. The residue was purified by thin layer chromatography with developing solvent system C to obtain the title compound **7d** (240 mg), yield: 49.7%.

MS m/z (ESI): 224.3 [M+1].

### Step 3

### tert-Butyl

### 2-(2-(5-methyl-2-((1-methyl-1H-pyrazol-5-yl)amino)pyrimidin-4-yl)-4-oxo-6,7-dihydro thieno[3,2-c]pyridin-5(4H)-yl)acetate 7e

Compound **7d** (80 mg, 0.36 mmol), hexamethyldistannane (140 mg, 0.43 mmol), lithium chloride (21 mg, 0.35 mmol) and tetrakis(triphenylphosphine)palladium (61 mg, 0.05 mmol) were mixed and suspended in 6 mL of dioxane, and then the reaction solution was heated to 100°C and stirred for 8 hours. The reaction solution was cooled to room temperature, followed by the addition of compound **1c** (80 mg, 0.24 mmol), cuprous iodide (22 mg, 0.12 mmol), bis(triphenylphosphine)palladium (II) dichloride (32 mg, 0.06 mmol). The reaction solution was heated to 105°C and stirred for 3 hours. Saturated potassium fluoride solution was added, and the reaction solution was stirred for 2 hours, and then extracted with ethyl acetate. The organic phases were combined and concentrated under reduced pressure. The residue was purified by thin layer chromatography with developing solvent system C to obtain the title compound 7e (50 mg), yield: 45.5%.

MS m/z (ESI): 455.3 [M+1].

### Step 4

### 2-(2-(5-Methyl-2-((1-methyl-1H-pyrazol-5-yl)amino)pyrimidin-4-yl)-4-oxo-6,7-dihydro thieno[3,2-c]pyridin-5(4H)-yl)acetic acid 7f

Compound **7e** (25 mg, 55 µmol) was dissolved in 2 mL of dichloromethane, followed by the addition of trifluoroacetic acid (0.7 mL). The reaction solution was stirred for 2 hours, and then concentrated under reduced pressure to obtain the crude title compound **7f**, which was directly used in the next step.

MS m/z (ESI): 399.3 [M+1].

### Step 5

### (S)-N-(2-Hydroxy-1-(m-methylphenyl)ethyl-2-(2-(5-methyl-2-((1-methyl-1H-pyrazol-5-yl)amino)pyrimidin-4-yl)-4-oxo-6,7-dihydrothieno[3,2-c]pyridin-5(4H)-yl)acetamide 7

The crude compound **7f** (22 mg, 55 µmol), compound **3f** (16 mg, 105 µmol), 2-(7-azabenzotriazol-1-yl)-N,N,N',N'-tetramethyluronium hexafluorophosphate (38 mg, 106 µmol) and *N,N*-diisopropyl ethylamine (57 mg, 450 µmol) were dissolved in 2 mL of *N,N*-dimethyl formamide, and the reaction solution was stirred for 2 hours. The title compound 7 (15 mg) was obtained by preparation and purification, yield: 51.0%.

MS m/z (ESI): 532.5 [M+1].

¹H NMR (400 MHz, CD₃OD): δ 8.25 (s, 1H), 7.90 (s, 1H), 7.78 (s, 1H), 7.05-7.19 (m, 4H), 6.36 (s, 1H), 4.04-4.07 (m, 1H), 4.53 (s, 3H), 4.29 (s, 2H), 3.76-3.79 (m, 4H), 3.15-3.19 (m, 2H), 2.44 (s, 3H), 2.32 (s, 3H).

### Examples 8-P1 and 8-P2

### (S)-N-((S)-2-Amino-1-(3-chlorophenyl)ethyl)-2-(2-(5-chloro-2-((tetrahydro-2H-pyran-4 -yl)amino)pyrimidin-4-yl)-4-oxo-6,7-dihydrothieno[3,2-c]pyridin-5(4H)-yl)propionami de 8-P1

### (R)-N-((S)-2-Amino-1-(3-chlorophenyl)ethyl)-2-(2-(5-chloro-2-((tetrahydro-2H-pyran-4 -yl)amino)pyrimidin-4-yl)-4-oxo-6,7-dihydrothieno[3,2-c]pyridin-5(4H)-yl)propionami de 8-P2

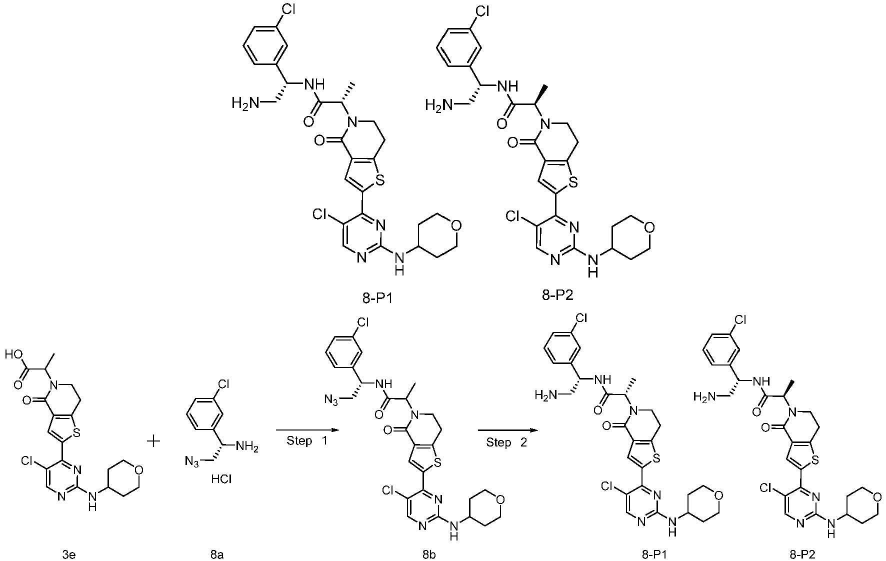

### Step 1

### N-((S)-2-Azido-1-(3-chlorophenyl)ethyl)-2-(2-(5-chloro-2-((tetrahydro-2H-pyran-4-yl) amino)pyrimidin-4-yl)-4-oxo-6,7-dihydrothieno[3,2-c]pyridin-5(4H)-yl)propionamide

### 8b

The crude compound **3e** (43 mg, 98 µmol), (*S*)-2-azido-1-(3-chlorophenyl)ethan-1-amine hydrochloride **8a** (40 mg, 200 µmol, prepared according to the method disclosed in patent application US2016362407A1), 2-(7-azabenzotriazol-1-yl)-N,N,N',N'-tetramethyluronium hexafluorophosphate (75 mg, 200 µmol), and *N,N*-diisopropyl ethylamine (63 mg, 490 µmol) were dissolved in 2 mL of *N,N*-dimethyl formamide. The reaction solution was stirred for 2 hours, and then concentrated under reduced pressure. The residue was purified by thin layer chromatography with developing solvent system C to obtain the title compound **8b** (40 mg), yield: 66.0%.

MS m/z (ESI): 615.3 [M+1].

### Step 2

### (S)-N-((S)-2-Amino-1-(3-chlorophenyl)ethyl)-2-(2-(5-chloro-2-((tetrahydro-2H-pyran-4-yl)amino)pyrimidin-4-yl)-4-oxo-6,7-dihydrothieno[3,2-c]pyridin-5(4H)-yl)propionam ide 8-P1

### (R)-N-((S)-2-Amino-1-(3-chlorophenyl)ethyl)-2-(2-(5-chloro-2-((tetrahydro-2H-pyran-4 -yl)amino)pyrimidin-4-yl)-4-oxo-6,7-dihydrothieno[3,2-c]pyridin-5(4H)-yl)propionami de 8-P2

Compound **8b** (40 mg, 95 µmol) was dissolved in 2 mL methanol solution, followed by the addition of palladium carbon hydrogenation catalyst (wet) (10%, 27 mg, 120 µmol). The reaction solution was stirred for 1 hour under hydrogen atmosphere. The reaction solution was filtered and concentrated under reduced pressure. The residue was purified by preparative HPLC (instrument model: Gilson 281; chromographic column: Sharpsil-T, Prep 30^{∗}150 mm, 5 µm, C18; mobile phase: A-water (0.1% trifluoroacetic acid), B-acetonide; flow rate: 30 mL/min; column temperature: room temperature) to obtain the title compounds **8-P1** and **8-P2** (18 mg, 18 mg).

### 8-P1 Single configuration compound (shorter retention time):

MS m/z (ESI): 589.3 [M+1].

HPLC analysis: Retention time 15.06 min, purity: 96.8% (chromatographic column: Sharpsil-T, Prep 30^{∗}150 mm, 5 µm; mobile phase: A-water (0.1% trifluoroacetic acid), B-acetonitrile, gradient ratio: A 5%-95%).

¹H NMR (400 MHz, CD₃OD): δ 8.45 (s, 1H), 8.28 (s, 1H), 7.30-7.41 (m, 4H), 5.30-5.32 (m, 1H), 5.16-5.19 (m, 1H), 3.97-4.01 (m, 3H), 3.70-3.72 (m, 1H), 3.52-3.56 (m, 3H), 3.35-3.38 (m, 2H), 3.08-3.10 (m, 1H), 3.00-3.02 (m, 1H), 2.02-2.04 (m, 2H), 1.63-1.66 (m, 2H), 1.46 (d, 3H).

### 8-P2 Single configuration compound (longer retention time):

MS m/z (ESI): 589.3 [M+1].

HPLC analysis: Retention time 15.91 min, purity: 98.2% (chromatographic column: Sharpsil-T, Prep 30^{∗}150 mm, 5 µm; mobile phase: A-water (0.1% trifluoroacetic acid), B-acetonitrile, gradient ratio: A 5%-95%).

¹H NMR (400 MHz, CD₃OD): δ 8.41 (s, 1H), 8.27 (s, 1H), 7.32-7.45 (m, 4H), 5.25-5.29 (m, 1H), 4.99-5.01 (m, 1H), 3.97-4.01 (m, 3H), 3.76-3.87 (m, 2H), 3.52-3.55 (m, 2H), 3.34-3.36 (m, 2H), 3.18-3.20 (m, 2H), 2.01-2.03 (m, 2H), 1.63-1.66 (m, 2H), 1.51 (d, 3H).

### Examples 9-P1 and 9-P2

### (S)-2-(2-(5-Chloro-2-((tetrahydro-2H-pyran-4-yl)amino)pyrimidin-4-yl)-4-oxo-6,7-dih ydrothieno[3,2-c]pyridin-5(4H)-yl)-N-((S)-1-(3-fluoro-5-methoxyphenyl)-2-hydroxyeth yl)propionamide 9-P1

### (R)-2-(2-(5-Chloro-2-((tetrahydro-2H-pyran-4-yl)amino)pyrimidin-4-yl)-4-oxo-6,7-dih ydrothieno[3,2-c]pyridin-5(4H)-yl)-N-((S)-1-(3-fluoro-5-methoxyphenyl)-2-hydroxyeth yl)propionamide 9-P2

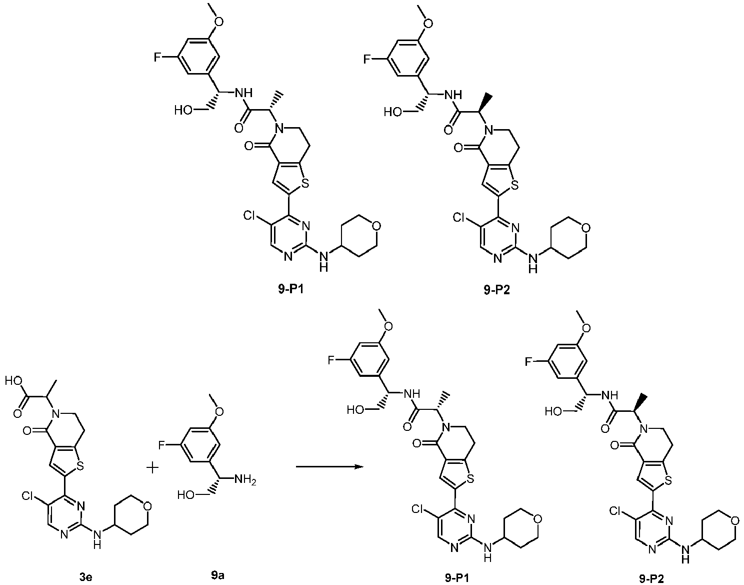

The crude compound **3e** (53 mg, 121 µmol), compound **9a** (33 mg, 180 µmol), 2-(7-azabenzotriazol-1-yl)-N,N,N',N'-tetramethyluronium hexafluorophosphate (75 mg, 180 µmol), and *N,N*-diisopropyl ethylamine (78 mg, 600 µmol) were dissolved in 2 mL of *N,N*-dimethyl formamide. The reaction solution was stirred for 2 hours, and then concentrated under reduced pressure. The residue was purified by preparative HPLC (separation conditions: chromographic column: Sharpsil-T prep 30^{∗}150 mm, 5 µm, C18; mobile phase: A-water (0.1% TFA), B-acetonide; column temperature: room temperature, flow rate: 30 mL/min). The corresponding components were collected and concentrated under reduced pressure to obtain the title compounds **9-P1** and **9-P2** (20 mg, 20 mg).

### 9-P1 Single configuration compound (shorter retention time):

MS m/z (ESI): 604.1 [M+1].

Preparative HPLC: Retention time 19.20 min, purity: 98.8% (chromatographic column: Sharpsil-T Prep 30^{∗}150 mm; 5 µm; C18; mobile phase: A-water (0.1%TFA), B-acetonitrile; column temperature: room temperature, flow rate: 30 mL/min);

¹H NMR (400 MHz, CD₃OD): δ 8.40 (s, 1H), 8.26 (d, 1H), 6.73 (s, 1H), 6.68 (d, 1H), 6.57 (d, 1H), 5.35-5.32 (m, 1H), 4.96-4.93 (m, 1H), 3.99-3.96 (m, 3H), 3.76-3.74 (m, 6H), 3.58-3.56 (m, 3H), 3.08-3.05 (m, 2H), 2.01-1.99 (m, 2H), 1.63-1.60 (m, 2H), 1.45 (d, 3H).

### 9-P2 Single configuration compound (longer retention time):

MS m/z (ESI):604.1 [M+1];

Preparative HPLC: Retention time 20.800 min, purity: 99.2% (chromatographic column: Sharpsil-T Prep 30^{∗}150 mm; 5 µm; C18; mobile phase: A-water (0.1%TFA), B-acetonitrile; column temperature: room temperature, flow rate: 30 mL/min);

¹H NMR (400 MHz, CD₃OD): δ 8.42 (s, 1H), 8.25 (d, 1H), 6.75 (s, 1H), 6.68 (d, 1H), 6.59 (d, 1H), 5.36-5.32 (m, 1H), 4.94-4.92 (m, 1H), 4.01-3.98 (m, 3H), 3.77-3.74 (m, 7H), 3.58-3.56 (m, 2H), 3.25-3.23 (m, 1H), 3.14-3.12 (m, 1H), 2.02-1.99 (m, 2H), 1.66-1.63 (m, 2H), 1.45 (d, 3H).

### Examples 10-P1 and 10-P2

### (S)-N-((S)-1-(3-Fluoro-5-methoxyphenyl)-2-hydroxyethyl)-2-(2-(5-methyl-2-((1-methyl -1H-pyrazol-5-yl)amino)pyrimidin-4-yl)-4-oxo-6,7-dihydrothieno[3,2-c]pyridin-5(4H)-yl)propionamide 10-P1

### (R)-N-((S)-1-(3-Fluoro-5-methoxyphenyl)-2-hydroxyethyl)-2-(2-(5-methyl-2-((1-methyl -1H-pyrazol-5-yl)amino)pyrimidin-4-yl)-4-oxo-6,7-dihydrothieno[3,2-c]pyridin-5(4H)-yl)propionamide 10-P2

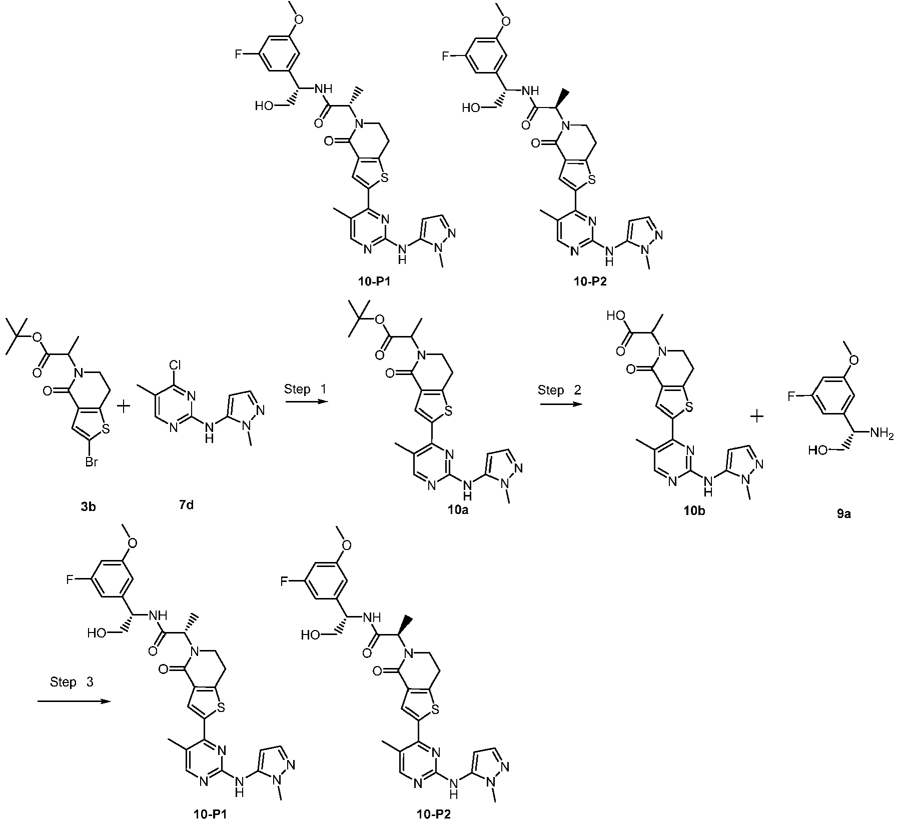

### Step 1

### tert-Butyl

### 2-(2-(5-methyl-2-((1-methyl-1H-pyrazol-5-yl)amino)pyrimidin-4-yl)-4-oxo-6,7-dihydro thieno[3,2-c]pyridin-5(4H)-yl)propionate 10a

Compound **7d** (80mg, 0.36 mmol), hexamethyldistannane (140 mg, 0.43 mmol), lithium chloride (21 mg, 0.35 mmol), tetrakis(triphenylphosphine)palladium (61 mg, 0.05 mmol) were mixed and suspended in 5 mL of dioxane, and then the reaction solution was heated to 105°C and stirred for 8 hours. The reaction solution was cooled to room temperature, followed by the addition of compound **3b** (130 mg, 0.36 mmol), cuprous iodide (35 mg, 0.18 mmol), and bis(triphenylphosphine)palladium (II) dichloride (49 mg, 0.07 mmol). The reaction solution was heated to 105°C and stirred for 3 hours. Saturated potassium fluoride solution was added, and the reaction solution was stirred for 2 hours, and then extracted with ethyl acetate. The organic phases were combined and concentrated under reduced pressure. The residue was purified by thin layer chromatography with developing solvent system C to obtain the title compound **10a** (40 mg), yield: 23.1%.

MS m/z (ESI): 469.1 [M+1].

### Step 2

### 2-(2-(5-Methyl-2-((1-methyl-1H-pyrazol-5-yl)amino)pyrimidin-4-yl)-4-oxo-6,7-dihydro thieno[3,2-c]pyridin-5(4H)-yl)propanoic acid 10b

Compound **10a** (40 mg, 85µmol) was dissolved in 1 mL of dichloromethane, followed by the addition of trifluoroacetic acid (0.5 mL). The reaction solution was stirred for 2 hours, and then concentrated under reduced pressure to obtain the crude title compound **10b** (35 mg), which was directly used in the next step.

MS m/z (ESI): 413.2 [M+1].

### Step 3

### (S)-N-((S)-1-(3-Fluoro-5-methoxyphenyl)-2-hydroxyethyl)-2-(2-(5-methyl-2-((1-methyl -1H-pyrazol-5-yl)amino)pyrimidin-4-yl)-4-oxo-6,7-dihydrothieno[3,2-c]pyridin-5(4H)-yl)propionamide 10-P1

### (R)-N-((S)-1-(3-Fluoro-5-methoxyphenyl)-2-hydroxyethyl)-2-(2-(5-methyl-2-((1-methyl -1H-pyrazol-5-yl)amino)pyrimidin-4-yl)-4-oxo-6,7-dihydrothieno[3,2-c]pyridin-5(4H)-yl)propionamide 10-P2

The crude compound **10b** (35 mg, 85 µmol), compound **9a** (18.9 mg, 102 µmol), 2-(7-azabenzotriazol-1-yl)-*N,N,N',N'*-tetramethyluronium hexafluorophosphate (38 mg, 106 µmol), and *N,N*-diisopropyl ethylamine (57 mg, 450 µmol) were dissolved in 2 mL of *N,N*-dimethyl formamide. The reaction solution was stirred for 2 hours, and then concentrated under reduced pressure. The residue was purified by preparative HPLC with the separation conditions (chromographic column: Gemini 20^{∗}250 mm, 5 µm, C18; mobile phase: A-water (10 mmol ammonium acetate), B-acetonide; column temperature: room temperature; flow rate: 18 mL/min). The corresponding components were collected and concentrated under reduced pressure to obtain the title compounds **10-P1** and **10-P2** (10 mg, 10 mg).

### 10-P1 Single configuration compound (shorter retention time):

MS m/z (ESI): 580.2[M+1];

HPLC analysis: Retention time 10.83min, purity: 98.3% (chromatographic column: Sharpsil-T, Prep 21.2^{∗}150 mm, 5 µm; mobile phase: A-water (10 mM ammonium bicarbonate), B-acetonitrile);

¹H NMR (400 MHz, CDCl₃): δ 8.25 (s, 1H), 7.89 (s, 1H), 7.53 (d, 1H), 7.21 (d, 1H), 6.76 (s, 1H), 6.58-6.54 (m, 2H), 6.49 (d, 1H), 6.38 (d, 1H), 5.41-5.31 (m, 2H), 5.03-5.00 (m, 1H), 3.89-3.85 (m, 2H), 3.82 (s, 3H), 3.69 (s, 3H), 3.62-3.60 (m, 2H), 3.49-3.47 (m, 2H), 2.47 (s, 3H), 1.32 (d, 3H).

### 10-P2 Single configuration compound (longer retention time):

MS m/z (ESI):580.2 [M+1];

HPLC analysis: Retention time 14.93min, purity: 98.7% (chromatographic column: Sharpsil-T, Prep 21.2^{∗}150 mm, 5 µm; mobile phase: A-water(10 mM ammonium bicarbonate), B-acetonitrile);

¹H NMR (400 MHz, CDCl₃): δ 8.25 (s, 1H), 7.87 (s, 1H), 7.50 (d, 1H), 7.15 (d, 1H), 6.84 (s, 1H), 6.66-6.62 (m, 2H), 6.55 (d, 1H), 6.37 (d, 1H), 5.37-5.31 (m, 2H), 5.03-5.01 (m, 1H), 3.89-3.86 (m, 2H), 3.82 (s, 3H), 3.79 (s, 3H), 3.73-3.69 (m, 2H), 3.15-3.08 (m, 2H), 2.46 (s, 3H), 1.32 (d, 3H).

### Example 11

(*R*)-*N*-((*S*)-1-(3-Fluoro-5-methoxyphenyl)-2-hydroxyethyl)-2-(4-oxo-2-(2-((tetrahydro-2*H*-pyran-4-yl)amino)pyrimidin-4-yl)-6,7-dihydrothieno[3,2-*c*]pyridin-5(4*H*)-yl)-propio namide **11**

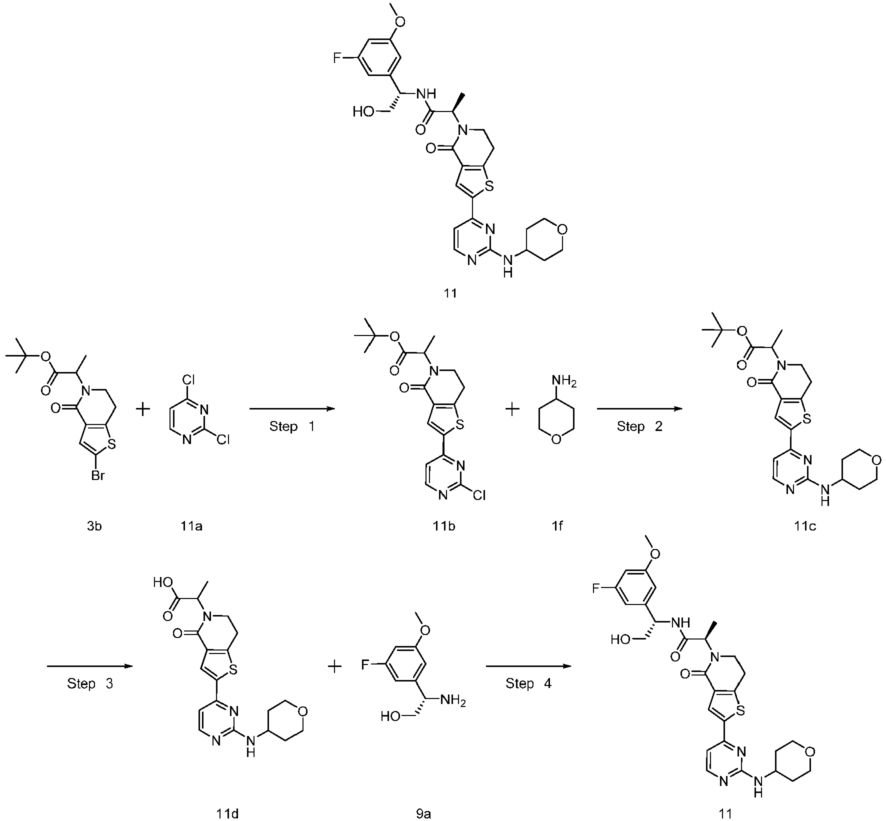

### Step 1

### tert-Butyl

### 2-(2-(2-chloropyrimidin-4-yl)-4-oxo-6,7-dihydrothieno[3,2-c]pyridin-5(4H)-yl)propion ate 11b

Compound **3b** (2.0 g, 5.55 mmol), bis(pinacolato)diboron (4.2 g, 16.5 mmol), cuprous iodide (1.0 g, 5.55 mmol), tributylphosphine (1.9 g, 9.4 mmol) and potassium *tert*-butanol (934 mg, 8.3 mmol) were mixed and suspended in 30 mL tetrahydrofuran. Under argon atmosphere, the reaction solution was heated to 60°C and stirred for 40 minutes. The reaction solution was cooled to room temperature and filtered through diatomaceous earth, followed by the addition of compound 2,4-dichloropyrimidine **11a** (804 mg, 5.4 mmol), [1,1'-bis(diphenylphosphino)ferrocene]palladium (II) dichloride dichloromethane complex (457 mg, 0.54 mmol), sodium carbonate (1.1 g, 10.7 mmol), and 5 mL of water. Under argon atmosphere, the reaction solution was heated to 60°C and stirred for 2 hours. Saturated sodium chloride solution was added, and the reaction solution was stirred for 2 hours, and then extracted with ethyl acetate. The organic phases were combined and concentrated under reduced pressure. The residue was purified by thin layer chromatography with developing solvent system C to obtain the title compound **11b** (430 mg), yield: 20.2%.

MS m/z (ESI): 392.1 [M+1].

### Step 2

### tert-Butyl

### 2-(4-oxo-2-(2-((tetrahydro-2H-pyran-4-yl)amino)pyrimidin-4-yl)-6,7-dihydrothieno[3,2 -c]pyridin-5(4H)-yl)propionate 11c

Compound **11b** (430 mg, 1.1 mmol) was dissolved in 10 mL of tetrahydrofuran, followed by the addition of 4-aminotetrahydropyran **1f** (331 mg, 3.1 mmol) and *N,N*-diisopropyl ethylamine (423 mg, 3.3 mmol). The reaction solution was reacted at 110°C in microwave reactor for 2 hours, and then concentrated under reduced pressure. The residue was purified by thin layer chromatography with the developing solvent system C to obtain the title compound **11c** (150 mg), yield: 30.0%.

MS m/z (ESI): 459.2 [M+1].

### Step 3

### 2-(4-Oxo-2-(2-((tetrahydro-2H-pyran-4-yl)amino)pyrimidin-4-yl)-6,7-dihydrothieno[3, 2-c]pyridin-5(4H)-yl)propanoic acid 11d

Compound **11c** (115 mg, 0.25 mmol) was dissolved in 20 mL of dichloromethane, followed by the addition of trifluoroacetic acid (0.7 mL). The reaction solution was stirred for 2 hours, and then concentrated under reduced pressure to obtain the crude title compound **11d** (100 mg). The product was directly used in the next step without purification.

MS m/z (ESI): 403.2 [M+1].

### Step 4

### (R)-N-((S)-1-(3-Fluoro-5-methoxyphenyl)-2-hydroxyethyl)-2-(4-oxo-2-(2-((tetrahydro-2

*H*-pyran-4-yl)amino)pyrimidin-4-yl)-6,7-dihydrothieno[3,2-*c*]pyridin-5(4*H*)-yl)-propion amide **11**

The crude compound **11d** (230 mg, 0.57 mmol), compound **9a** (211 mg, 1.1 mmol), 2-(7-azabenzotriazol-1-yl)-N,N,N',N'-tetramethyluronium hexafluorophosphate (270 mg, 1.1 mmol), and *N,N*-diisopropyl ethylamine (150 mg, 1.1 mmol) were dissolved in 10 mL of *N,N*-dimethyl formamide. The reaction solution was stirred for 2 hours, and then concentrated under reduced pressure. The residue was purified with preparative HPLC (instrument model: waters, chromatographic column: X-bridge Prep 30^{∗}150 mm, 5 µm, C18; mobile phase: A-water (10 mM ammonium acetate), B-acetonide; flow rate: 30 mL/min, column temperature: room temperature). At retention time 17.32 min, compound **11** (110 mg, compound in a single configuration with longer retention time) was obtained, yield: 30.7%.

MS m/z (ESI): 570.2 [M+1].

¹H NMR (400 MHz, CDCl₃): δ 8.26 (s, 1H), 7.87 (s, 1H), 7.72 (d, 1H), 6.83 (d, 1H), 6.69 (d, 2H), 6.53 (d, 1H), 5.41 (dd, 2H), 5.07 (d, 1H), 4.03 (d, 3H), 3.88 (dd, 3.7 Hz, 1H), 3.81 (d, 5H), 3.70 (dt, 1H), 3.58 (t, 2H), 3.24 - 3.11 (m, 1H), 3.04 (dt, 1H), 2.16- 1.97 (m, 3H), 1.61 (dt, 2H), 1.45 (d, 3H).

### Example 12

### (R)-2-(2-(5-Chloro-2-(((S)-1-hydroxypropan-2-yl)amino)pyrimidin-4-yl)-4-oxo-6,7-dih ydrothieno[3,2-c]pyridin-5(4H)-yl)-N-((S)-1-(3-fluoro-5-methoxyphenyl)-2-hydroxyeth yl)propionamide 12

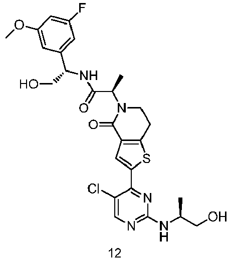

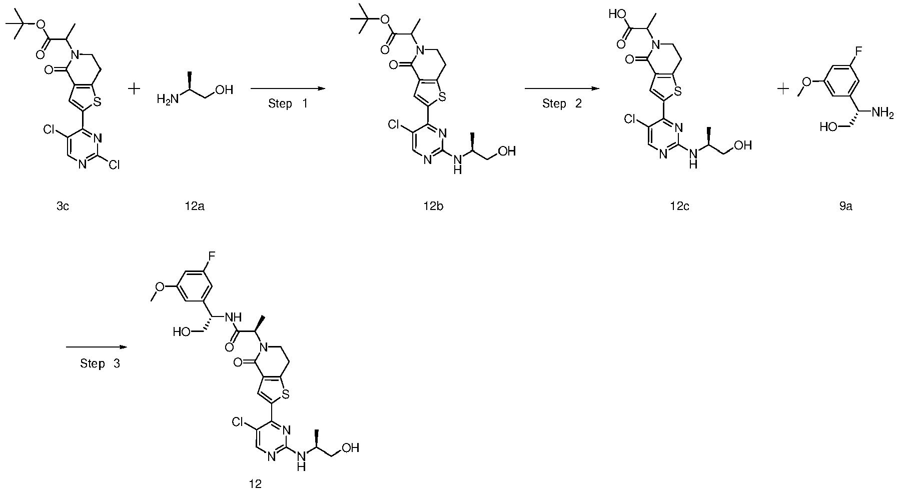

### Step 1

### tert-Butyl

### 2-(2-(5-chloro-2-(((S)-1-hydroxypropan-2-yl)amino)pyrimidin-4-yl)-4-oxo-6,7-dihydrot hieno[3,2-c]pyridin-5(4H)-yl)propionate 12b

Compound **3c** (60 mg, 0.14 mmol) was dissolved in 2 mL of tetrahydrofuran, followed by the addition of (*S*)-2-aminopropan-1-ol **12a** (21 mg, 0.28 mmol) and *N,N*-diisopropyl ethylamine (75 mg, 0.58 mmol). The reaction solution was reacted at 120°C in microwave reactor for 1 hour, and then concentrated under reduced pressure. The residue was purified by thin layer chromatography with developing solvent system C to obtain the title compound **12b** (35 mg), yield: 53.5%.

MS m/z (ESI): 467.3 [M+1].

### Step 2

### 2-(2-(5-Chloro-2-(((S)-1-hydroxypropan2-yl)amino)pyrimidin-4-yl)-4-oxo-6,7-dihydrot hieno[3,2-c]pyridin-5(4H)-yl)propanoic acid 12c

Compound **12b** (30 mg, 65µmol) was dissolved in 2 mL of dichloromethane, followed by the addition of trifluoroacetic acid (0.7 mL). The reaction solution was stirred for 2 hours, and then concentrated under reduced pressure to obtain the crude title compound **12c** (27 mg). The product was directly used in the next step without purification.

MS m/z (ESI): 411.3 [M+1].

### Step 3

### (R)-2-(2-(5-Chloro-2-(((S)-1-hydroxypropan-2-yl)amino)pyrimidin-4-yl)-4-oxo-6,7-dih ydrothieno[3,2-c]pyridin-5(4H)-yl)-N-((S)-1-(3-fluoro-5-methoxyphenyl)-2-hydroxyeth yl)propionamide 12

The crude compound **12c** (27 mg, 65 µmol), compound **9a** (24 mg, 130 µmol, Shanghai Bide Pharmatech Ltd.), 2-(7-azabenzotriazol-1-yl)-N,N,N',N'-tetramethyluronium hexafluorophosphate (22 mg, 93 µmol), and *N,N*-diisopropyl ethylamine (42 mg, 324 µmol) were dissolved in 2 mL of *N,N*-dimethyl formamide. The reaction solution was stirred for 2 hours, and then concentrated under reduced pressure. The residue was purified with preparative HPLC (instrument model: Gilson, chromatographic column: Sharpsil-T Prep 30^{∗}150 mm, 5 µm, C18; mobile phase: A-water (10 mM ammonium acetate), B-acetonide; flow rate: 30 mL/min, column temperature: room temperature). At retention time 14.75 min, compound **12** (15 mg, 39.5%, compound in a single configuration with longer retention time) was obtained.

MS m/z (ESI): 578.2[M+1].

¹H NMR (400 MHz, CD₃OD): δ 8.44 (s, 1H), 8.25 (s, 1H), 6.74 (s, 1H), 6.70 (d, 1H), 6.59 (d, 1H), 5.34-5.32 (m, 1H), 4.96-4.93(m, 1H), 4.08-4.05 (m, 1H), 3.78-3.75(m, 7H), 3.63-3.60 (m, 2H), 3.26-3.23 (m, 1H), 3.14-3.11 (m, 1H), 1.45 (d, 3H), 1.26 (d, 3H).

### Example 13

### (R)-2-(2-(5-Chloro-2-(((S)-1-hydroxypropan-2-yl)amino)pyrimidin-4-yl)-4-oxo-6,7-dih ydrothieno[3,2-c]pyridin-5(4H)-yl)-N-((S)-2-hydroxy-1-(m-methylphenyl)ethyl)propion amide 13

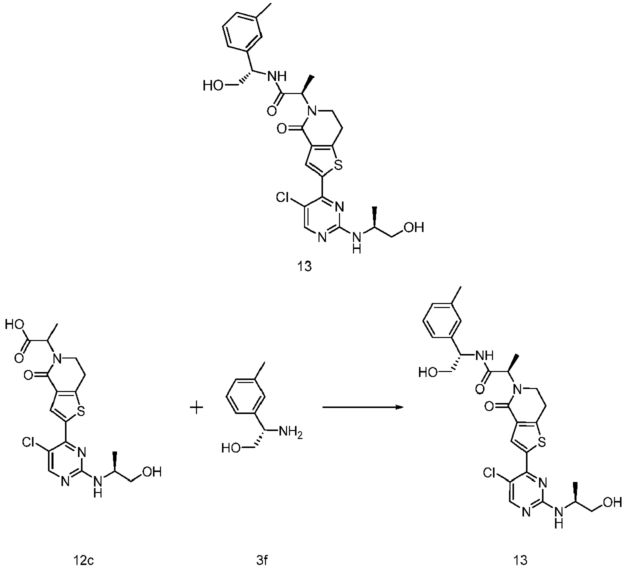

The crude compound **12c** (145 mg, 352 µmol), compound **3f** (80 mg, 529 µmol, Shanghai Bide Pharmatech Ltd.), 2-(7-azabenzotriazol-1-yl)-N,N,N',N'-tetramethyluronium hexafluorophosphate (200 mg, 528 µmol), and *N,N*-diisopropyl ethylamine (273 mg, 2.1 mmol) were dissolved in 2 mL of *N,N*-dimethyl formamide. The reaction solution was stirred for 2 hours, and concentrated under reduced pressure. The residue was purified with preparative HPLC (instrument model: waters, chromatographic column: X-bridge Prep 30^{∗}150 mm, 5 µm, C18; mobile phase: A-water (10 mM ammonium acetate), B-acetonide; flow rate: 30 mL/min, column temperature: room temperature). At retention time 13.97 min, compound **13** (30 mg, 15.6%, compound in a single configuration with longer retention time) was obtained.

MS m/z (ESI): 544.2[M+1].

¹H NMR (400 MHz, CD₃OD): δ 8.44 (s, 1H), 8.24 (s, 1H), 7.22 (t, 1H), 7.18 (d, 2H), 7.08 (d, 1H), 5.34-5.31 (m, 1H), 4.97-4.94 (m, 1H), 4.07-4.03 (m 1H), 3.76-3.74 (m, 4H), 3.63-3.60 (m, 2H), 3.26-3.23 (m, 1H), 3.13-3.10 (m, 1H), 2.33(s, 3H), 1.43 (d, 3H), 1.28 (d, 3H).

### Example 14

### (R)-2-(2-(5-Chloro-2-((1-methyl-1H-pyrazol-5-yl)amino)pyrimidin-4-yl)-4-oxo-6,7-dih ydrothieno[3,2-c]pyridin-5(4H)-yl)-N-((S)-1-(3-fluoro-5-methoxyphenyl)-2-hydroxyeth yl)propionamide14

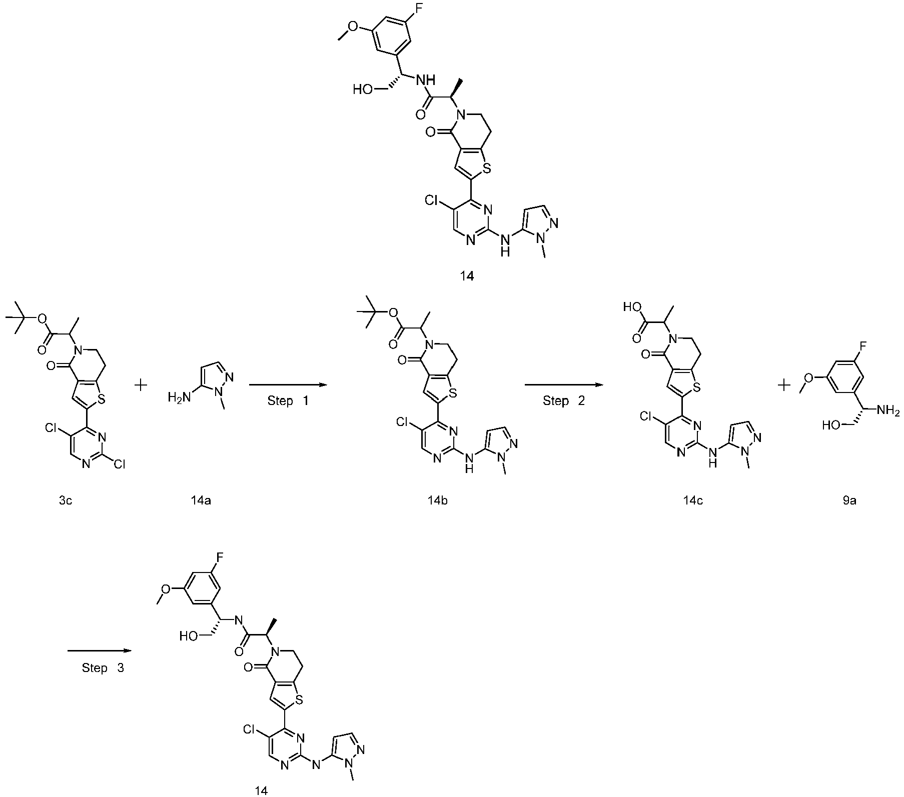

### Step 1

### tert-Butyl

### 2-(2-(5-chloro-2-((1-methyl-1H-pyrazol-5-yl)amino)pyrimidin-4-yl)-4-oxo-6,7-dihydrot hieno[3,2-c]pyridin-5(4H)-yl)propionate 14b

Compound **3c** (240 mg, 0.56 mmol) was dissolved in 5 mL of dioxane, followed by the addition of 1-methyl-1*H*-pyrazol-5-amine **14a** (108 mg, 1.11 mmol), tris(dibenzylideneacetone)dipaladium (76 mg, 0.08 mmol), 4,5-bis(diphenylphosphino)-9,9-dimethylxanthene (64 mg, 0.11 mmol), and cesium carbonate (365 mg, 1.12 mmol). The reaction solution was reacted at 100°C in microwave reactor for 1 hour, and then concentrated under reduced pressure. The residue was purified by thin layer chromatography with developing solvent system C to obtain the title compound **14b** (40 mg), yield: 14.9%.

MS m/z (ESI): 489.3 [M+1].

### Step 2

### 2-(2-(5-Chloro-2-((1-methyl-1H-pyrazol-5-yl)amino)pyrimidin-4-yl)-4-oxo-6,7-dihydro thieno[3,2-c]pyridin-5(4H)-yl)propanoic acid14c

Compound **14b** (18 mg, 36 µmol) was dissolved in 1.5 mL of dichloromethane, followed by the addition of trifluoroacetic acid (0.5 mL). The reaction solution was stirred for 2 hours, and then concentrated under reduced pressure to obtain the crude title compound **14c** (15 mg). The product was directly used in the next step without purification.

MS m/z (ESI): 433.1 [M+1].

### Step 3

### (R)-2-(2-(5-Chloro-2-((1-methyl-1H-pyrazol-5-yl)amino)pyrimidin-4-yl)-4-oxo-6,7-dih ydrothieno[3,2-c]pyridin-5(4H)-yl)-N-((S)-1-(3-fluoro-5-methoxyphenyl)-2-hydroxyeth yl)propionamide 14

The crude compound **14c** (120 mg, 277 µmol), compound **9a** (66 mg, 356 µmol, Shanghai Bide Pharmatech Ltd.), 2-(7-azabenzotriazol-1-yl)-N,N,N',N'-tetramethyluronium hexafluorophosphate (158 mg, 415 µmol), and *N,N*-diisopropyl ethylamine (214 mg, 1.6 mmol) were dissolved in 2 mL of *N,N*-dimethyl formamide. The reaction solution was stirred for 2 hours, and then concentrated under reduced pressure. The residue was purified with preparative HPLC (instrument model: waters, chromatographic column: X-bridge Prep 30^{∗}150 mm, 5 µm, C18; mobile phase: A-water (10 mM ammonium acetate), B-acetonide; flow rate: 30 mL/min, column temperature: room temperature). At retention time 17.69 min, the title compound **14** (30 mg, 18.1%, compound in a single configuration with longer retention time) was obtained.

MS m/z (ESI): 600.2[M+1].

¹H NMR (400 MHz, CD₃OD): δ 8.45 (d, 2H), 7.47 (s, 1H), 6.75 (s, 1H), 6.70 (d, 1H), 6.59 (d, 1H), 6.39 (s, 1H), 5.35-5.32(m, 1H), 4.97-4.94 (m, 1H), 3.78-3.73(m, 10H), 3.25-3.23 (m, 1H), 3.14-3.11 (m, 1H), 1.44 (d, 3H).

### Example 15

### (R)-N-((S)-1-(3-Fluoro-5-methoxyphenyl)-2-hydroxyethyl)-2-(2-(2-((1-methyl-1H-pyra zol-5-yl)amino)pyrimidin-4-yl)-4-oxo-6,7-dihydrothieno[3,2-c]pyridin-5(4H)-yl)propio namide 15

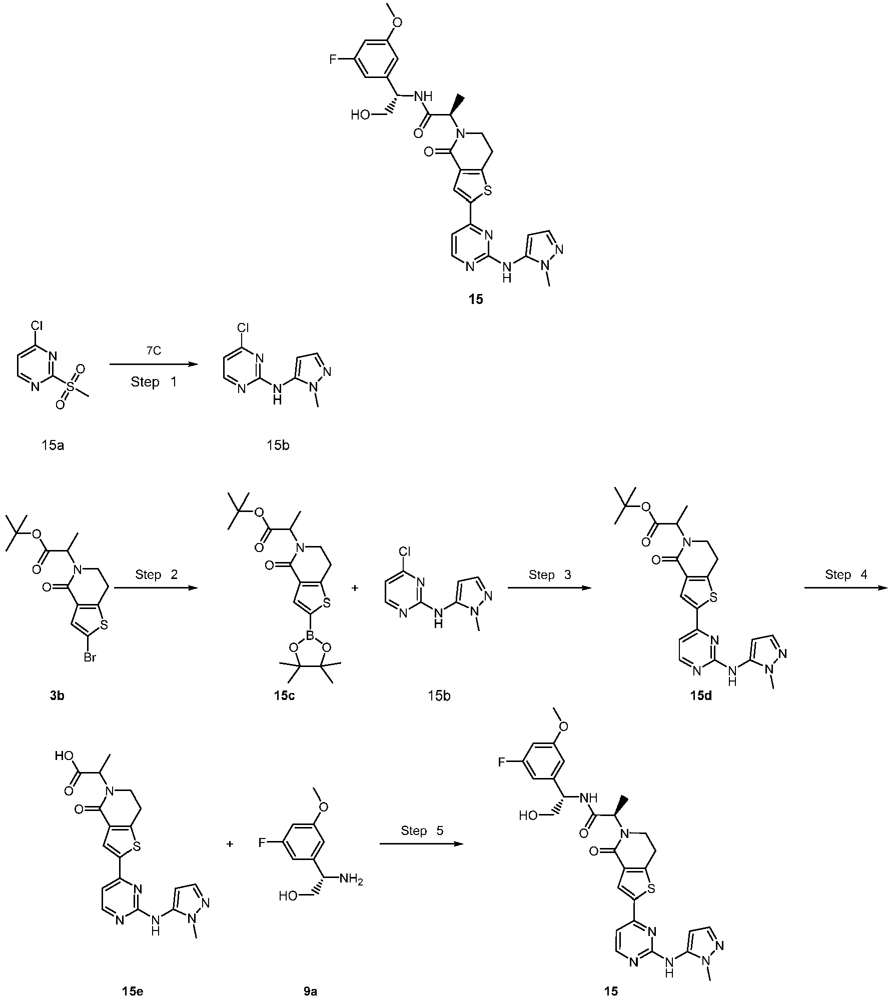

### Step 1

### 4-Chloro-N-(1-methyl-1H-pyrazol-5-yl)pyrimidin-2-amine 15b

*N*-(1-Methyl-1*H*-pyrazol-5-yl)formamide **7c** (324.82 mg, 2.60 mmol, prepared according to the method disclosed in patent application "WO2017/80979") was dissolved in 15 mL of *N,N*-dimethyl formamide, followed by the addition of sodium hydride (60%, 311.47 mg, 7.79 mmol) at 0°C. The reaction solution was stirred for 0.5 hours, followed by the addition of compound 4-chloro-2-(methylsulfonyl)pyrimidine **15a** (500 mg, 2.60 mmol). The reaction solution was further reacted for 2 hours. 20 mL of water was added, and the reaction solution was extracted with ethyl acetate (20 mL × 3). The organic phases were combined and concentrated under reduced pressure. The residue was purified by thin layer chromatography with developing solvent system C to obtain the title compound **15b** (270 mg), yield: 49.6%.

MS m/z (ESI): 210.3 [M+1].

### Step 2

### tert-Butyl

### 2-(4-oxo-2-(4,4,5,5-tetramethyl-1,3,2-dioxaborolan-2-yl)-6,7-dihydrothieno[3,2-c]pyrid in-5(4H)-yl)propanoate 15c

Under argon atmosphere, cmpound **3b** (2.0 g, 5.5 mmol) was dissolved in 70 mL tetrahydrofuran , followed by the addition of 4,4,4',4',5,5,5',5'-octamethyl-2,2'-di(1,3,2-dioxaborolane) (3.9 g, 15.5 mmol), cuprous iodide (1.1 g, 5.5 mmol), tri-n-butyl phosphine (1.1 g, 5.5 mmol) and potassium *tert-*butoxide (934 mg, 8.3 mmol) successively. The reaction solution was stirred at 60°C for 1 hour, and then cooled and filtered through diatomaceous earth. The filtrate was concentrated, and the residue was purified by column chromatography with eluent system C to obtain the title compound **15c** (1.5 g), yield: 66.3%.

MS m/z (ESI): 352.2 [M-55].

### Step 3

### tert-Butyl

### 2-2-(2-((1-methyl-1H-pyrazol-5-yl)amino)pyrimidin-4-yl)-4-oxo-6,7-dihydrothieno[3,2 -c]pyridin-5(4H)-yl)propanoate 15d

Under argon atmosphere, compound **15c** (520 mg, 1.28 mmol), pre-prepared compound **15b** (90 mg, 0.42 mmol), [1,1'-bis(diphenylphosphino)ferrocene]palladium (II) dichloride dichloromethane complex (72 mg, 0.085 mmol), and sodium carbonate (91 mg, 0.86 mmol) were suspended in 10 mL of 1,4-dioxane and 2 mL of water. The reaction solution was heated to 60°C and stirred for 1 hour. The reaction solution was cooled and filtered through diatomaceous earth. The filtrate was collected and extracted with ethyl acetate (20 mL × 3). The organic phases were combined and concentrated under reduced pressure. The residue was purified by column chromatography with eluent system A to obtain the title compound **1g** (100 mg), yield: 51.2%.

MS m/z (ESI): 455.1 [M+1].

### Step 4

### 2-2-(2-((1-Methyl-1H-pyrazol-5-yl)amino)pyrimidin-4-yl)-4-oxo-6,7-dihydrothieno[3,2 -c]pyridin-5(4H)-yl)propanoic acid 15e

Compound **15d** (120 mg, 264 µmol) was dissolved in 2 mL of dichloromethane, followed by the addition of trifluoroacetic acid (0.7 mL). The reaction solution was stirred for 2 hours, and then concentrated under reduced pressure to obtain the crude title compound **15e** (100 mg). The product was directly used in the next step without purification.

MS m/z (ESI): 399.0 [M+1].

### Step 5

### (R)-N-(S)-1-(3-Fluoro-5-methoxyphenyl)-2-hydroxyethyl)-2-(2-(2-((1-methyl-1H-pyra zol-5-yl)amino)pyrimidin-4-yl)-4-oxo-6,7-dihydrothieno[3,2-c]pyridin-5(4H)-yl)propio namide 15

The crude compound **15e** (100 mg, 250 µmol), compound **9a** (55 mg, 296 µmol, Shanghai Bide Pharmatech Ltd.), 2-(7-azabenzotriazol-1-yl)-N,N,N',N'-tetramethyluronium hexafluorophosphate (114 mg, 300 µmol), and *N,N*-diisopropyl ethylamine (194 mg, 1.5 mmol) were dissolved in 3 mL of *N,N*-dimethyl formamide. The reaction solution was stirred for 2 hours, and then concentrated under reduced pressure. The residue was purified with preparative HPLC (instrument model: waters, chromatographic column: Sharpsil-T, Prep 30^{∗}150 mm, 5 µm, C18; mobile phase: A-water (0.1%TFA), B-acetonide; flow rate: 30 mL/min, column temperature: room temperature), washed with saturated sodium bicarbonate solution and concentrated to dryness by rotary evaporation. At retention time 14.29 min, compound **15** (20 mg, 14.1%, compound in a single configuration with longer retention time) was obtained.

MS m/z (ESI): 566.2[M+1].

¹H NMR (400 MHz, CD₃OD): δ 8.41 (d, 1H), 8.07 (s, 1H), 7.56 (s, 1H), 7.33 (d, 1H), 6.75 (s, 1H), 6.70 (d, 1H), 6.59 (d, 1H), 6.49 (s, 1H), 5.34-5.32(m, 1H), 4.96-4.94 (m, 1H), 3.80-3.73(m, 6H), 3.71-3.68 (m, 4H), 3.25-3.22 (m, 1H), 3.12-3.09 (m, 1H), 1.44 (d, 3H).

### Example 16

### (R)-N-((S)-1-(3-Fluoro-5-methoxyphenyl)-2-hydroxyethyl)-2-(2-(5-methyl-2-((tetrahydr o-2H-pyran-4-yl)amino)pyrimidin-4-yl)-4-oxo-6,7-dihydrothieno[3,2-c]pyridin-5-(4H)-yl)propionamide16

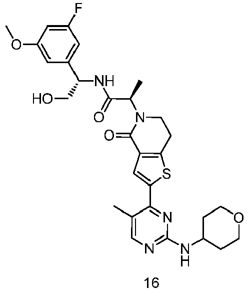

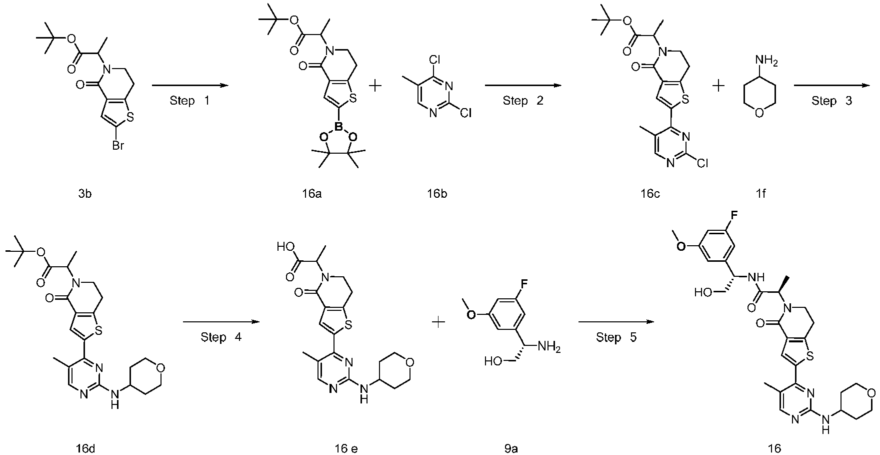

### Step 1

### tert-Butyl

### 2-(4-oxo-2-(4,4,5,5-tetramethyl-1,3,2-dioxaborolan-2-yl)-6,7-dihydrothieno[3,2-c]pyrid in-5-yl)propanoate 16a

Under argon atmosphere, compound **3b** (2 g, 5.56 mmol) was dissolved in 50 mL of THF, followed by the addition of 4,4,4',4',5,5,5',5'-octamethyl-2,2'-di(1,3,2-dioxaborolane) (3.9 g, 15.4 mmol), cuprous iodide (1 g, 5.3 mmol), tributylphosphine (1.1 g, 5.4 mmol) and [1,1'-bis(diphenylphosphino)ferrocene]palladium (II) dichloride (109 g, 0.15 mmol)successively. The reaction solution was stirred at 60°C for 40 minutes, and then cooled and filtered through diatomaceous earth. The filtrate was concentrated, and the residue was purified by column chromatography with eluent system C to obtain the title compound **16a** (2 g), yield: 88.4%.

MS m/z (ESI): 408.1 [M+1].

### Step 2

### tert-Butyl

### 2-(2-(2-chloro-5-methyl-pyrimidin-4-yl)-4-oxo-6,7-dihydrothieno[3,2-c]pyridin-5(4H)-yl)propanoate 16c

Under argon atmosphere, the mixture of compound **16a** (2 g, 4.9 mmol), 2,4-dichloro-5-methylpyrimidine **16b** (1.6 g, 9.8 mmol), tetrakis(triphenylphosphine)palladium (681 mg, 0.6 mmol) and sodium carbonate (1 g, 9.4 mmol) were suspended in 30 mL of 1,4-dioxane and 4 mL of water , and then the reaction solution was heated to 60°C and stirred for 3 hours. The reaction solution was cooled and filtered through diatomaceous earth. The filtrate was collected and extracted with ethyl acetate (20 mL × 3). The organic phases were combined and concentrated under reduced pressure. The residue was purified by column chromatography with eluent system A to obtain the title compound **16c** (1.5 g), yield: 74%.

MS m/z (ESI): 408.3 [M+1].

### Step 3

### tert-Butyl

### 2-(2-(5-methyl-2-((tetrahydro-2H-pyran-4-yl)amino)pyrimidin-4-yl)-4-oxo-6,7-dihydrot hieno[3,2-c]pyridin-5(4H)-yl)propanoate 16d

Compound **16c** (531 mg, 1.4 mmol) was dissolved in 5 mL of tetrahydrofuran, followed by the addition of compound **1f** (143 mg, 1.4 mmol) and *N,N*-diisopropyl ethylamine (546 mg, 4.2 mmol). The reaction solution was reacted in microwave reactor at 100°C for 4 hours, and then concentrated under reduced pressure. The residue was purified by thin layer chromatography with developing solvent system A to obtain the title compound **16d** (400 mg), yield: 64.3%.

MS m/z (ESI): 473.2 [M+1].

### Step 4

### 2-(2-(5-Methyl-2-((tetrahydro-2H-pyran-4-yl)amino)pyrimidin-4-yl)-4-oxo-6,7-dihydro thieno[3,2-c]pyridin-5(4H)-yl)propanoic acid 16e

Compound **16d** (200 mg, 0.4 mmol) was dissloved in 2 mL of dichloromethane, followed by the addition of trifluoroacetic acid (0.7 mL). The reaction solution was stirred for 2 hours, and then concentrated under reduced pressure to obtain the crude title compound **16e** (175 mg). The product was directly used in the next step without purification.

MS m/z (ESI): 417.2 [M+1].

### Step 5

### (R)-N-((S)-1-(3-Fluoro-5-methoxyphenyl)-2-hydroxyethyl)-2-(2-(5-methyl-2-((tetrahydr o-2H-pyran-4-yl)amino)pyrimidin-4-yl)-4-oxo-6,7-dihydrothieno[3,2-c]pyridin-5-(4H)-yl)propionamide 16

The crude compound **16e** (200 mg, 0.5 mmol), compound **9a** (90 mg, 0.5 mmol, Shanghai Bide Pharmatech Ltd.), 2-(7-azabenzotriazol-1-yl)-N,N,N',N'-tetramethyluronium hexafluorophosphate (113 mg, 0.5 mmol), and *N,N*-diisopropyl ethylamine (186 mg, 1.4 mmol) were dissolved in 2 mL of *N,N*-dimethyl formamide. The reaction solution wasstirred for 2 hours, and then concentrated under reduced pressure. The residue was purified with preparative HPLC (instrument model: waters, chromatographic column: X-bridge Prep 30^{∗}150 µm, 5 µm, C18; mobile phase: A-water (10mM ammonium acetate), B-acetonide; flow rate: 30 mL/min, column temperature: room temperature). At retention time 16.60 min, the title compound **16** (30 mg, 39.5%, compound in a single configuration with longer retention time) was obtained.

MS m/z (ESI): 584.2 [M+1].

¹H NMR (400 MHz, CD₃OD): δ 8.15 (s, 2H), 6.76-6.59 (m, 3H), 5.35-5.33 (m, 1H), 4.97-4.93(m, 1H), 4.04-4.01 (m, 3H), 3.82-3.73(m, 7H), 3.58-3.56 (m 2H), 3.34-3.21 (m, 2H), 2.50 (s, 3H), 2.06-2.02 (m, 2H), 1.71-1.70 (m, 2H), 1.48 (d, 3H).

### Example 17

(*R*)-*N*-((*S*)-1-(3-Fluoro-5-methoxyphenyl)-2-hydroxyethyl)-2-(2-(2-(((*S*)-1-hydroxyprop an-2-yl)amino)-5-methylpyrimidin-4-yl)-4-oxo-6,7-dihydrothieno[3,2-*c*]pyridin-5(4*H*)-yl)propionamide **17**

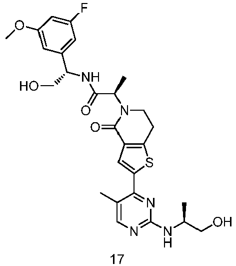

In accordance with the method of Example 16, compound **1f** was replaced with **12a**. The final product was purified by preparative HPLC (instrument model: Gilson, chromatographic column: Sharpsil-T, Prep 30^{∗}150 mm, 5 µm, C18; mobile phase: A-water (10 mM ammonium acetate), B-acetonide; flow rate: 30 mL/min; column temperature: room temperature). At retention time 14.10 min, the title compound **17** (30 mg, compound in a single configuration with longer retention time) was obtained.

MS m/z (ESI): 558.2[M+1].

¹H NMR (400 MHz, CD₃OD): δ 8.13 (s, 1H), 7.91 (s, 1H), 6.76 (s, 1H), 6.71 (d, 1H), 6.62 (d, 1H), 5.37-5.32 (m, 1H), 4.97-4.94 (m, 1H), 4.13-4.10 (m, 1H), 3.81(s, 3H), 3.78-3.73 (m, 4H), 3.65-3.58 (m, 2H), 3.26-3.15 (m, 2H), 2.41 (s, 3H), 1.47 (d, 3H), 1.28 (d, 3H).

### Biological Evaluation

### Test example 1: ERK1 enzyme activity test

### 1. Test Purpose

The purpose of this experiment is to detect the inhibitory ability of the compounds on the ERK1 enzyme activity and evaluate the activity of the compounds *in vitro* according to IC₅₀. The ADP-Glo^{™} Kinase Assay Kit was used in this experiment. Under the action of enzyme, the substrate was phosphorylated and ADP was produced at the same time. The ADP-Glo Reagent was added to remove unreacted ATP in the reaction system. The kinase detection reagent was used to detect the ADP produced by the reaction. In the presence of the compound, the inhibition rate of the compound was calculated by measuring the signal value.

### 2. Experimental Method

Enzyme and substrate preparation: ERK1 (1879-KS-010, R&D) and substrate (AS-61777, anaspec) were prepared to be 0.75 ng/µl and 100 µM respectively in the buffer. The enzyme solution and substrate solution were then prepared into a mixed solution at a volume ratio of 2:1 for later use. ATP was diluted to 300 µM with the buffer. The compounds were dissolved in DMSO (dimethyl sulfoxide, Shanghai Titan Scientific Co., Ltd.) to prepare a stock solution with an initial concentration of 20 mM, and then Bravo was used to prepare solutions of the compounds. Finally, a 3 µL of a mixed solution of enzyme and substrate, and 1 µL of different concentrations of the compound (the initial concentration is 50 µM, 4-fold dilution) were added to each well of the 384-well plate, and the plate was incubated at 30°C for 10 minutes, and finally 1 µL of 300 µM ATP solution was added to each well, and the plate was incubated at 30°C for 2 hours. Then 5 µL of ADP-Glo was added, and the plate was incubated at 30°C for 40 minutes. Then 10 µL of Kinase detection buffer was added, and the plate was incubated at 30°C for 40 minutes. The 384-well plate was taken out and placed in a microplate reader (BMG labtech, PHERAstar FS), and the chemiluminescence was measured by the microplate reader.

### 3. Data Analysis

The data were processed and analyzed with Microsoft Excel, Graphpad Prism 5. The IC₅₀ values of the compounds were obtained, and the results are shown in the table below.

| Example No. | IC₅₀ (nM) |
|---|---|
| 9-P2 | 19 |
| 10-P2 | 32 |
| 11 | 5 |
| 12 | 24 |
| 14 | 27 |
| 15 | 4 |
| 16 | 3 |
| 17 | 12 |

Conclusion: The compounds of the present disclosure had a significant inhibitory effect on ERK1 enzyme activity.

### Test example 2: ERK2 enzyme activity test

### 1. Test Purpose

The purpose of this experiment is to detect the inhibitory ability of the compounds on the ERK2 enzyme activity and evaluate the activity of the compounds *in vitro* according to IC₅₀. The ADP-Glo^{™} Kinase Assay Kit was used in this experiment. Under the action of enzyme, the substrate was phosphorylated and ADP was produced at the same time. The ADP-Glo Reagent was added to remove unreacted ATP in the reaction system. The kinase detection reagent was used to detect the ADP produced by the reaction. In the presence of the compound, the inhibition rate of the compound was calculated by measuring the signal value.

### 2. Experimental Method

Enzyme and substrate preparation: ERK2 (1230-KS-010, R&D) and substrate (Custom peptides, Gill Biochemical) were prepared to be 0.75 ng/µl and 1500 µM in the buffer (40 mM Tris, 20 mM MgCl₂, 0.1 mg/mL BSA, 50 µM DTT). The enzyme solution and substrate solution were then prepared into a mixed solution at a volume ratio of 2:1 for later use. ATP was diluted to 500 µM with the buffer. The compounds were dissolved in DMSO (dimethyl sulfoxide, Shanghai Titan Scientific Co., Ltd.) to prepare a stock solution with an initial concentration of 20 mM, and then Bravo was used to prepare solutions of the compounds. Finally, a 3 µL of a mixed solution of enzyme and substrate, and 1 µL of different concentrations of the compound (the initial concentration is 50 µM, 4-fold dilution) were added to each well of the 384-well plate, and the plate was incubated at 30°C for 10 minutes, and finally 1 µL of 500 µM ATP solution was added to each well, and the plate was incubated at 30°C for 2 hours. Then 5 µL of ADP-Glo was added, and the plate was incubated at 30°C for 40 minutes. Then 10 µL of Kinase detection buffer was added, and the plate was incubated at 30°C for 40 minutes. The 384-well plate was taken out and placed in a microplate reader (BMG labtech, PHERAstar FS), and the chemiluminescence was measured by the microplate reader.

### 3. Data Analysis

The data were processed and analyzed with Microsoft Excel, Graphpad Prism 5. The IC₅₀ values of the compounds were obtained, and the results are shown in the table below.

| Example No. | IC₅₀ (nM) |
|---|---|
| 1 | 1 |
| 2 | 3 |
| 3-P1 | 203 |
| 3-P2 | 4 |
| 4-P1 | 434 |
| 4-P2 | 6 |
| 5 | 22 |
| 6 | 29 |
| 7 | 3 |
| 9-P1 | 410 |
| 9-P2 | 3 |
| 10-P2 | 5 |
| 11 | 2 |
| 12 | 2 |
| 13 | 9 |
| 14 | 6 |
| 15 | 3 |
| 16 | 1 |
| 17 | 2 |

Conclusion: The compounds of the present disclosure had a significant inhibitory effect on ERK2 enzyme activity.

### Test Example 3: In Vitro proliferation inhibition test of compounds on Colo205 tumor cells

### 1. Test Purpose

The purpose of this experiment is to test the inhibitory activity of the compounds on the proliferation of Colo205 cells (CCL-222, ATCC) *in vitro.* The cells were treated with different concentrations of compounds *in vitro.* After 3 days of culture, the cell proliferation was detected using CTG (CellTiter-Glo^{®} Luminescent Cell Viability Assay, Promega, catalog number: G7573) reagent. The *in vitro* activity of the compounds was evaluated according to the IC₅₀ value.

### 2. Experimental Method

In the following, taking the in vitro proliferation inhibition test method of Colo205 cells as an example, the method in the present disclosure for testing the *in vitro* proliferation inhibitory activity of the compounds of the present disclosure is described. This method is also applicable to, but not limited to, the *in vitro* proliferation inhibitory activity test on other tumor cells.

Colo205 cells were digested, centrifuged and then resuspended. The single cell suspension was mixed well, and the density of viable cells was adjusted to 5.0×10⁴ cells/ml with cell culture medium (RPMI1640+2% FBS), and 95 µl/well was added to a 96-well cell culture plate. Only 100 µl medium was added to the peripheral wells of the 96-well plate. The culture plate was incubated in an incubator for 24 hours (37°C, 5% CO₂).

The compound was dissolved in DMSO (dimethyl sulfoxide, Shanghai Titan Scientific Co., Ltd.) and prepared into a stock solution with an initial concentration of 20 mM. The initial concentration of the small molecule compound is 2 mM, and then 4-fold diluted into to 9 points, and the tenth point is DMSO. Another 96-well plate was taken, 90 µl of cell culture medium (RPMI1640+2% FBS) was added to each well, then 10 µl of different concentrations of the test sample was added to each well, the mixture was mixed well, and then 5µl of different concentrations of the test sample was added to the cell culture plate, and each sample has two duplicate holes. The culture plate was incubated in the incubator for 3 days (37°C, 5% CO₂). The 96-well cell culture plate was taken out, 50 µl CTG solution was added to each well, and the plate was incubated for 10 minutes at room temperature. In a microplate reader (BMG labtech, PHERAstar FS), chemiluminescence was measured with the microplate reader.

### 3. Data Analysis

The data were processed and analyzed with Microsoft Excel, Graphpad Prism 5. The results are shown in the table below:

| Example No. | IC₅₀ (nM) |
|---|---|
| 1 | 8 |
| 3-P2 | 26 |
| 4-P2 | 29 |
| 7 | 38 |
| 9-P2 | 18 |
| 10-P2 | 50 |
| 11 | 16 |
| 12 | 5 |
| 13 | 26 |
| 14 | 14 |
| 15 | 49 |
| 17 | 17 |

Conclusion: The compounds of the present disclosure had a significant inhibitory effect on the proliferation of Colo205 tumor cells.

### Pharmacokinetic Evaluation

### Test Example 4. Pharmacokinetics assay of the compounds of the present disclosure in mice

### 1. Abstract

Mice were used as the test animal. The drug concentration in plasma at different time points was determined by LC/MS/MS method after administration of compound 3-P2, compound 4-P2, compound 9-P2, compound 10-P2, compound 12 and compound 15. The pharmacokinetic behavior of the compounds of the present disclosure in mice was studied, and the pharmacokinetic characteristics were evaluated.

### 2. Test Protocol

### 2.1. Test Samples

Compound 3-P2, Compound 4-P2, Compound 9-P2, Compound 10-P2, Compound 12 and Compound 15.

### 2.2. Test Animals

54 of C57 mice (female) were equally divided into 6 groups, which were purchased from Shanghai Jiesijie Laboratory Animal Co., Ltd., with the animal production license number: SCXK (Shanghai) 2013-0006.

### 2.3 Preparation of the test compound

A certain amount of compound was weighted, followed by the addition of 5% volume of DMSO and 5% Tween 80 to dissolve it, then followed by the addition of 90% normal saline to prepare a colorless and clear solution of 0.1 mg/mL.

### 2.4 Administration

After an overnight fast, C57 mice were intragastrically administered the test compound at an administration dose of 2 mg/kg and an administration volume of 0.2ml/10g.

### 3. Process

The mice were intragastrically administered the test compounds. 0.1mL of blood was taken before the administration and at 0.25, 0.5, 1.0, 2.0, 4.0, 6.0, 8.0, 11.0, 24.0 hours after the administration. The blood was placed in a heparinized test tube, and centrifuged at 3500 rpm for 10 minutes. The plasma was separated and stored at -20°C.

The content of the test compound in the plasma of mice after injection administration of the test compound at different concentrations was determined: 25 µl of mouse plasma at each time point after the administration was taken, followed by the addition of 50 µl of the internal standard camptothecin solution (100 ng/mL) and 200 µl of acetonitrile. The resulting solution was vortex-mixed for 5 minutes, and centrifuged for 10 minutes (4000 rpm). 4 µl of the supernatant was taken from the plasma samples for LC/MS/MS analysis.

### 4. Results of pharmacokinetic parameters

The pharmacokinetic parameters of the compounds of the present disclosure are shown below:

| N o . | Pharmacokinetics assay in mice | | | | | |
|---|---|---|---|---|---|---|
| | Plasma Concentration | Area under curve | Half Life | Residence Time | Clearance | Apparent distribution volume |
| | Cmax (ng /mL) | AUC (ng /mL^{∗}h) | T1/2 (h) | MRT (h) | CLz/F (mL/min/kg) | Vz/F (mL/kg) |
| 3-P2 | 708 | 914 | 0.945 | 1.16 | 36.5 | 2985 |
| 4-P2 | 376 | 364 | 1.02 | 1.14 | 91.5 | 8092 |
| 9-P2 | 1477 | 1816 | 4.49 | 1.85 | 18.4 | 7136 |
| 10-P2 | 1003 | 1829 | 1.26 | 1.64 | 18.2 | 1993 |
| 12 | 1476 | 1602 | 2.65 | 1.05 | 20.8 | 4766 |
| 15 | 1070 | 1832 | 1.11 | 1.85 | 18.2 | 1755 |

Conclusion: The compounds of the present disclosure were well absorbed, and had a significant pharmacokinetic advantage.

## Claims

1. A compound of formula (I), 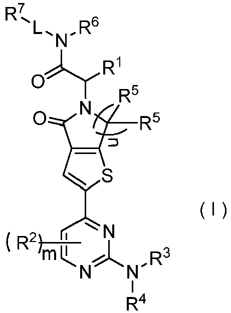 or a stereoisomer, tautomer, mesomer, racemate, enantiomer, diastereomer thereof, or mixture thereof, or a pharmaceutically acceptable salt thereof,
wherein:
R¹ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R² is identical or different, and each is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R³ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁴ is selected from the group consisting of hydrogen atom, halogen, alkyl, alkoxy, haloalkyl, haloalkoxy, hydroxy, hydroxyalkyl, cyano, amino, nitro, cycloalkyl and heterocyclyl;
R⁵ is identical or different, and each is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁶ is selected from the group consisting of hydrogen atom, halogen, alkyl, alkoxy, haloalkyl, haloalkoxy, hydroxy and hydroxyalkyl;
L is a bond or alkylene, wherein the alkylene is optionally further substituted by one or more substituents selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
R⁷ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, haloalkyl, haloalkoxy and aminoalkyl;
n is 1, 2 or 3; and
m is 0, 1 or 2.

2. The compound of formula (I), or the stereoisomer, tautomer, mesomer, racemate, enantiomer, diastereomer thereof, or mixture thereof, or the pharmaceutically acceptable salt thereof according to claim 1, being a compound of formula (I-P2): 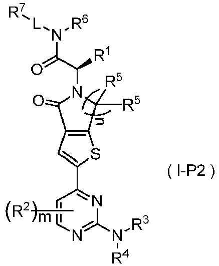 or a stereoisomer, tautomer, mesomer, racemate, enantiomer, diastereomer thereof, or mixture thereof, or a pharmaceutically acceptable salt thereof, wherein:
L, m, n and R¹ to R⁷ are as defined in claim 1.

3. The compound of formula (I), or the stereoisomer, tautomer, mesomer, racemate, enantiomer, diastereomer thereof, or mixture thereof, or the pharmaceutically acceptable salt thereof according to claims 1 or 2, wherein n is 1 or 2.

4. The compound of formula (I) according to claim 1, being a compound of formula (II): 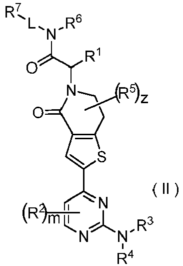 or a stereoisomer, tautomer, mesomer, racemate, enantiomer, diastereomer thereof, or mixture thereof, or a pharmaceutically acceptable salt thereof, wherein:
z is 0, 1, 2, 3 or 4; and
L, m and R¹ to R⁷ are as defined in claim 1.

5. The compound of formula (I), or the stereoisomer, tautomer, mesomer, racemate, enantiomer, diastereomer thereof, or mixture thereof, or the pharmaceutically acceptable salt thereof according to any one of claims 1 to 4, wherein the L is an alkylene, wherein the alkylene is optionally further substituted by one or more substituents selected from the group consisting of alkyl, alkoxy, oxo, halogen, amino, cyano, nitro, hydroxy, hydroxyalkyl and aminoalkyl; preferably further substituted by hydroxyalkyl; and more preferably, L is - CH(R⁸)-, wherein R⁸ is C₁₋₆ hydroxyalkyl.

6. The compound of formula (I) according to any one of claims 1, 3 and 4, being a compound of formula (III): 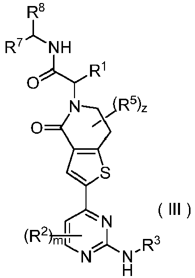 or a stereoisomer, tautomer, mesomer, racemate, enantiomer, diastereomer thereof, or mixture thereof, or a pharmaceutically acceptable salt thereof, wherein:
R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl; and
R¹ to R³, R⁵, R⁷, m and z are as defined in claim 1.

7. The compound of formula (III), or the stereoisomer, tautomer, mesomer, racemate, enantiomer, diastereomer thereof, or mixture thereof, or the pharmaceutically acceptable salt thereof according to claim 6, being a compound of formula (III-P2): 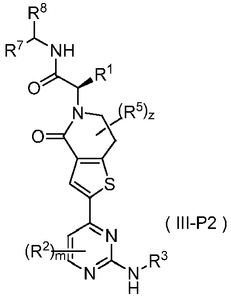 or a stereoisomer, tautomer, mesomer, racemate, enantiomer, diastereomer thereof, or mixture thereof, or a pharmaceutically acceptable salt thereof, wherein:
R¹ to R³, R⁵, R⁷, R⁸, m and z are as defined in claim 6.

8. The compound of formula (I), or the stereoisomer, tautomer, mesomer, racemate, enantiomer, diastereomer thereof, or mixture thereof, or the pharmaceutically acceptable salt thereof according to any one of claims 1 to 7, wherein R³ is selected from the group consisting of C₁₋₆ hydroxyalkyl, 3 to 6 membered heterocyclyl and 5 or 6 membered heteroaryl, wherein the C₁₋₆ hydroxyalkyl group, 3 to 6 membered heterocyclyl and 5 or 6 membered heteroaryl are each optionally further substituted by one or more substituents selected from the group consisting of C₁₋₆ alkyl, C₁₋₆ alkoxy, halogen, amino, cyano, hydroxy and C₁₋₆ hydroxyalkyl.

9. The compound of formula (I), or the stereoisomer, tautomer, mesomer, racemate, enantiomer, diastereomer thereof, or mixture thereof, or the pharmaceutically acceptable salt thereof according to any one of claims 1 to 7, wherein R³ is a heterocyclyl, wherein the heterocyclyl is optionally further substituted by one or more substituents selected from the group consisting of alkyl, alkoxy, oxo, halogen, amino, cyano, nitro, hydroxy and hydroxyalkyl.

10. The compound of formula (I), or the stereoisomer, tautomer, mesomer, racemate, enantiomer, diastereomer thereof, or mixture thereof, or the pharmaceutically acceptable salt thereof according to any one of claims 1 to 7, wherein R³ is a heteroaryl, wherein the heteroaryl is optionally further substituted by one or more substituents selected from the group consisting of alkyl, alkoxy, oxo, halogen, amino, cyano, nitro, hydroxy and hydroxyalkyl.

11. The compound of formula (I), or the stereoisomer, tautomer, mesomer, racemate, enantiomer, diastereomer thereof, or mixture thereof, or the pharmaceutically acceptable salt thereof according to any one of claims 1 to 10, wherein R⁷ is an aryl, wherein the aryl is optionally further substituted by one or more substituents selected from the group consisting of alkyl, alkoxy, oxo, halogen, amino, cyano, nitro, hydroxy and hydroxyalkyl; preferably, R⁷ is a phenyl, wherein the phenyl is optionally further substituted by one or more substituents selected from the group consisting of C₁₋₆ alkyl, C₁₋₆ alkoxy, halogen, amino, cyano, hydroxy and C₁₋₆ hydroxyalkyl.

12. The compound of formula (I), or the stereoisomer, tautomer, mesomer, racemate, enantiomer, diastereomer thereof, or mixture thereof, or the pharmaceutically acceptable salt thereof according to any one of claims 6 to 11, wherein R⁸ is selected from the group consisting of alkyl, alkoxy, oxo, halogen, amino, cyano, nitro, hydroxy, hydroxyalkyl and aminoalkyl, preferably hydroxyalkyl or aminoalkyl; and more preferably C₁₋₆ hydroxyalkyl.

13. The compound of formula (I), or the stereoisomer, tautomer, mesomer, racemate, enantiomer, diastereomer thereof, or mixture thereof, or the pharmaceutically acceptable salt thereof according to any one of claims 1 to 12, wherein R¹ is selected from the group consisting of hydrogen atom, halogen, alkyl, alkoxy, haloalkyl, haloalkoxy, hydroxy and hydroxyalkyl, preferably hydrogen atom, halogen or alkyl, more preferably hydrogen atom or alkyl; and most preferably hydrogen atom or C₁₋₆ alkyl.

14. The compound of formula (I), or the stereoisomer, tautomer, mesomer, racemate, enantiomer, diastereomer thereof, or mixture thereof, or the pharmaceutically acceptable salt thereof according to any one of claims 1 to 5 and 8 to 13, wherein R¹ is an alkyl, and R⁴ is a hydrogen atom; preferably, R¹ is a C₁₋₆ alkyl, and R⁴ is a hydrogen atom.

15. The compound of formula (I), or the stereoisomer, tautomer, mesomer, racemate, enantiomer, diastereomer thereof, or mixture thereof, or the pharmaceutically acceptable salt thereof according to any one of claims 1 to 14, wherein R² is selected from the group consisting of hydrogen atom, halogen, alkyl, alkoxy, haloalkyl, haloalkoxy, hydroxy and hydroxyalkyl; preferably hydrogen atom, halogen or alkyl; more preferably hydrogen atom, halogen or C₁₋₆ alkyl; and most preferably halogen.

16. The compound of formula (I), or the stereoisomer, tautomer, mesomer, racemate, enantiomer, diastereomer thereof, or mixture thereof, or the pharmaceutically acceptable salt thereof according to any one of claims 1 to 15, wherein R⁵ is identical or different, and each is selected from the group consisting of hydrogen atom, halogen, alkyl, alkoxy, haloalkyl, haloalkoxy, hydroxy and hydroxyalkyl; preferably hydrogen atom or alkyl; and more preferably hydrogen atom or C₁₋₆ alkyl.

17. The compound of formula (I), or the stereoisomer, tautomer, mesomer, racemate, enantiomer, diastereomer thereof, or mixture thereof, or the pharmaceutically acceptable salt thereof according to any one of claims 1 to 16, selected from the group consisting of: 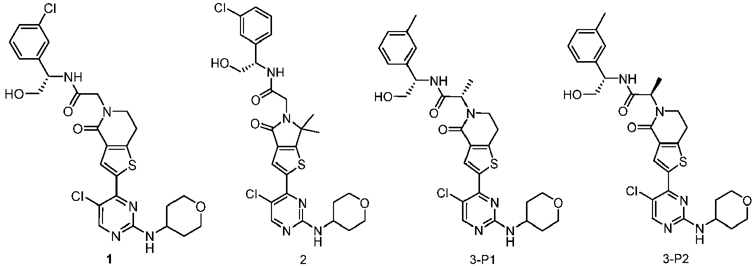 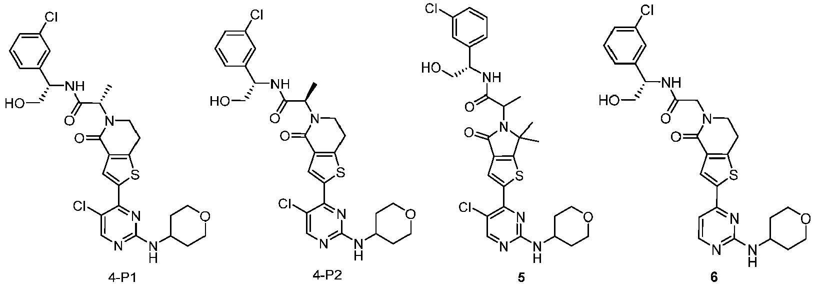 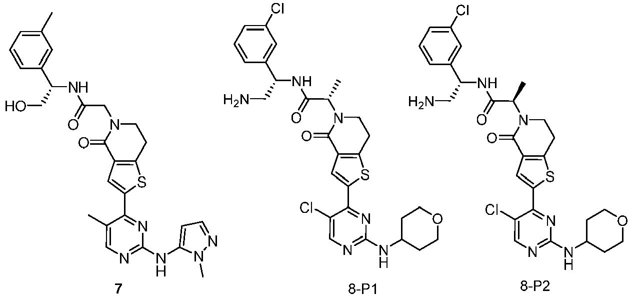 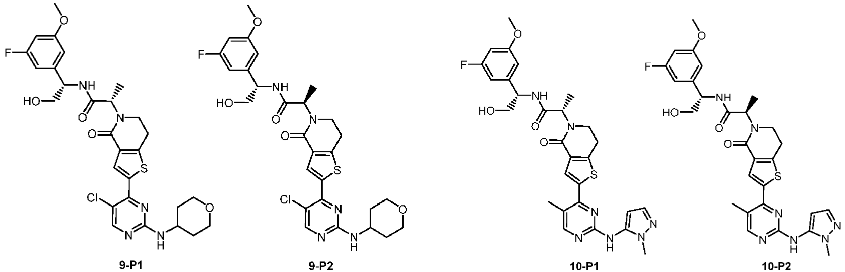 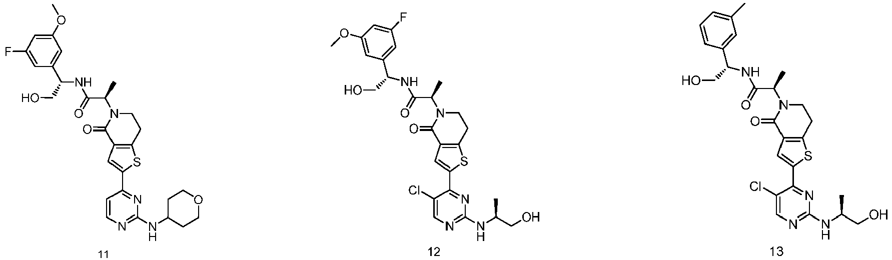 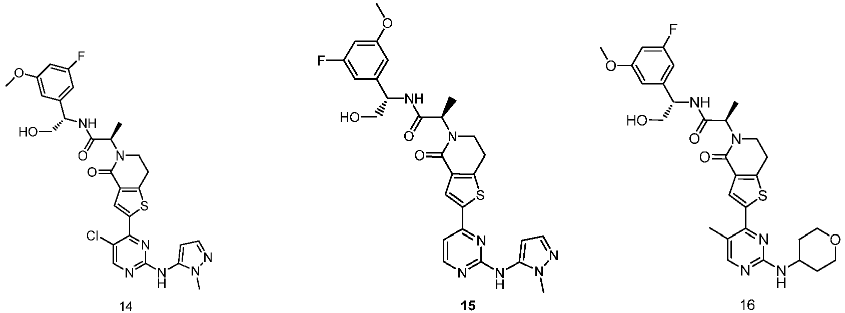 and

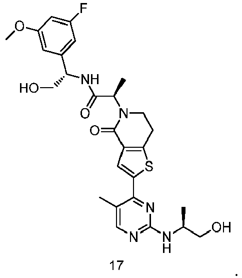

18. A compound of formula (IA): 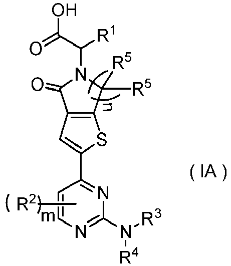
or a stereoisomer, tautomer, mesomer, racemate, enantiomer, diastereomer thereof, or mixture thereof, or a pharmaceutically acceptable salt thereof,
wherein: R¹ to R⁵, m and n are as defined in claim 1.

19. The compound of formula (IA) according to claim 18, selected from the group consisting of:

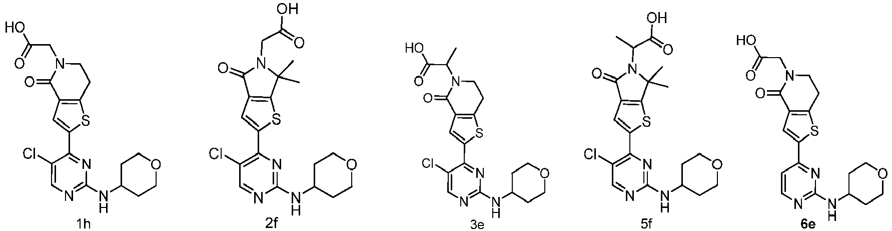

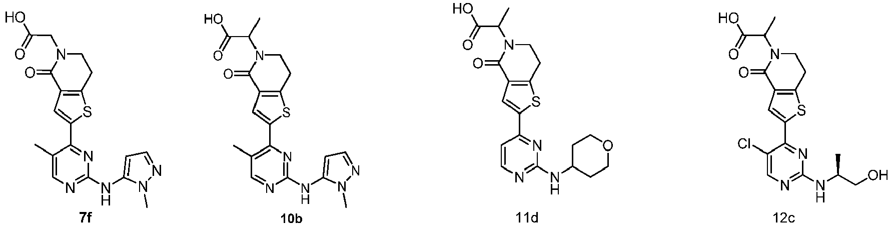

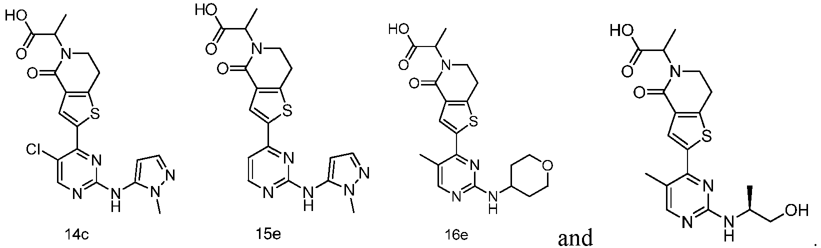

20. A method for preparing the compound of formula (I) according to claim 1, comprising a step of: 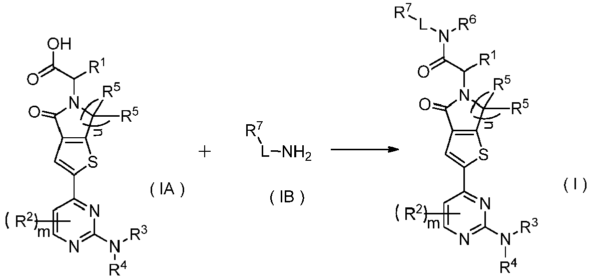
subjecting a compound of formula (IA) and a compound of formula (IB) to a condensation reaction in the presence of an alkaline reagent to obtain the compound of formula (I),
wherein: the alkaline reagent is preferably *N,N*-diisopropyl ethylamine; and
R¹ to R⁷, L, m and n are as defined in claim 1.

21. A pharmaceutical composition comprising a therapeutically effective amount of the compound of formula (I), or the stereoisomer, tautomer, mesomer, racemate, enantiomer, diastereomer thereof, or mixture thereof, or the pharmaceutically acceptable salt thereof according to any one of claims 1 to 17, and one or more pharmaceutically acceptable carriers, diluents or excipients.

22. Use of the compound of formula (I), or the stereoisomer, tautomer, mesomer, racemate, enantiomer, diastereomer thereof, or mixture thereof, or the pharmaceutically acceptable salt thereof according to any one of claims 1 to 17, or the pharmaceutical composition according to claim 21, in the preparation of a medicament for inhibiting ERK

23. Use of the compound of formula (I), or the stereoisomer, tautomer, mesomer, racemate, enantiomer, diastereomer thereof, or mixture thereof, or the pharmaceutically acceptable salt thereof according to any one of claims 1 to 17, or the pharmaceutical composition according to claim 21, in the preparation of a medicament for treating or preventing cancer, inflammation, or other proliferative diseases, preferably cancer, and more preferably wherein the cancer is selected from the group consisting of melanoma, liver cancer, kidney cancer, lung cancer, nasopharyngeal carcinoma, colorectal cancer, colon cancer, rectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma, astrocytoma, and glioma.
